(19) Europäisches Patentamt
European Patent Office
Office européen des brevets

(11) Numéro de publication: **0 178 980**
**B1**

(12) # FASCICULE DE BREVET EUROPÉEN

(45) Date de publication du fascicule du brevet: **29.03.89**

(51) Int. Cl.⁴: **C 07 D 501/34**, C 07 D 501/36, A 61 K 31/545

(21) Numéro de dépôt: **85401837.1**

(22) Date de dépôt: **23.09.85**

(54) **Dérivés de céphalosporines, procédé d'obtention et leur application à titre d'antibiotiques.**

(30) Priorité: **27.09.84 FR 8414878**

(43) Date de publication de la demande: **23.04.86 Bulletin 86/17**

(45) Mention de la délivrance du brevet: **29.03.89 Bulletin 89/13**

(84) Etats contractants désignés: **AT BE CH DE FR GB IT LI LU NL SE**

(56) Documents cité:
**EP-A-0 060 745**

**Spec. Publ. R-Soc. chim. 1981 (Publ. 1980) 38 Recent Adv. Chem of B lactam. Antibiot. W. Dürckheimer et al. p. 46-56**

**Le dossier contient des informations techniques présentées postérieurement au dépôt de la demande et ne figurant pas dans le présent fascicule.**

(73) Titulaire: **SANOFI, société anonyme, 40, Avenue George V, F-75008 Paris (FR)**

(72) Inventeur: **Olliero, Dominique Bât. Lorraine, Rés. des Facultés Avenue de la Justice, F-34100 Montpellier (FR)**
Inventeur: **Salhi, Ali, 639, rue de Valène, F-34980 Saint- Gely- du- Fesc (FR)**

(74) Mandataire: **Gillard, Marie- Louise, Cabinet Beau de Loménie 55, Rue d'Amsterdam, F-75008 Paris (FR)**

**EP 0 178 980 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

## Description

La présente invention concerne des dérivés de la famille des céphalosporines, leur procédé de préparation et leur application en thérapeutique.

D'autres dérivés de la famille des céphalosporines composés S-oxydes - ont été décrits dans la demande EP 60745. Ils présentent des spectres d'activité, notamment sur les staphylocoques, différents de ceux des composés selon l'invention.

Les composés, selon l'invention, répondent à la formule:

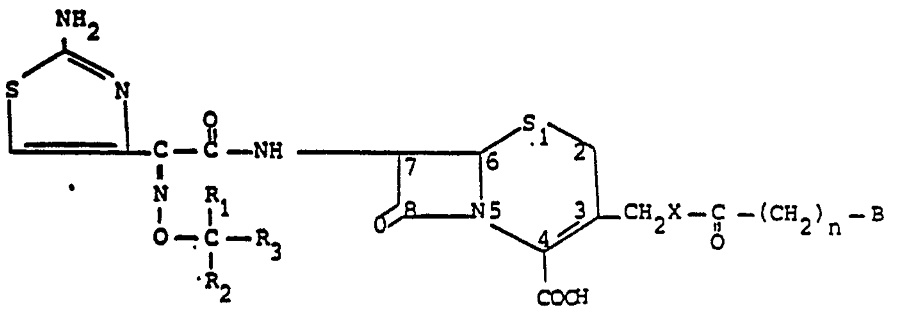

(I)

dans laquelle:

- X désigne un atome d'oxygène ou un atome de soufre.
- n est zéro, 1 ou 2.
- $R_1$, $R_2$ et $R_3$ désignent chacun un atome d'hydrogène, ou encore R et R désignent un atome d'hydrogène ou un groupe méthyle et R désigne un groupe carboxyle ou cyclopropyle, ou encore
- $R_1$ et $R_2$ pris ensemble avec l'atome de carbone auquel ils sont liés forment un noyau cyclobutyle ou cyclopentyle et $R_3$ désigne un groupe carboxyle.
- B est le reste d'une amine primaire ou secondaire choisie parmi les groupements suivants:

- $Z$-$NH_2$ où Z est un groupe alkylène à chaîne droite ou ramifiée ayant de 2 à 7 atomes de carbone, éventuellement interrompue par un atome de soufre, ou encore Z représente un groupe 1,3-cyclohexylène ou 1,4-cyclohexylène.

- Z'-Alk-NH-R où Z' représente un groupe 1,2-phénylène ou 1,3-phénylène ou 1,4-phénylène éventuellement substitué par 1, 2 ou 3 groupes méthyle ou bien Z' représente un groupe 1,2-cyclohexylène, 1,3-cyclohexylène ou 1,4-cyclohexylène.

Alk représente un groupe alkylène droit ou ramifié ayant de 1 à 3 atomes de carbone, éventuellement interrompu par un atome de soufre.

R représente un atome d'hydrogène ou un groupe alkyle ayant de 1 à 3 atomes de carbone.

- Z"-N-CO-Y-NH-R où Z" représente un groupe 1,3-phénylène
R'

ou 1,4-phénylène, Y désigne un groupe alkylène $(CH_2)_m$ dans lequel m = 1, 2 ou 3, R' est hydrogène ou méthyle et R est tel que désigné ci-dessus.

- Z"-CO-NH-Y-$NH_2$ ou Z" et Y sont tels que définis ci-dessus.

- Z"-Y'-NH-CO-Y-$NH_2$ où Z" et Y sont tels que définis ci-dessus et Y' représente un groupe alkylène droit ou ramifié ayant 1 ou 2 atomes de carbone

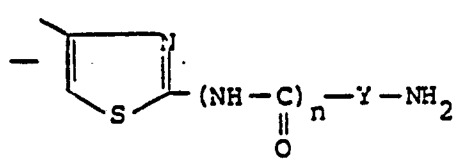

où n = 0 ou 1 et Y est tel que défini précédemment. un groupe 2-pipéridyl 3-pipéridyl ou 4-pypéridyl éventuellement substitué sur l'atome d'azote par un groupe -CO-Y-$NH_2$ ou Y est tel que défini précédemment.

— un groupe 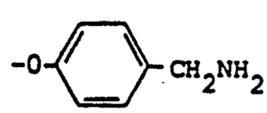

- le groupe bicyclique 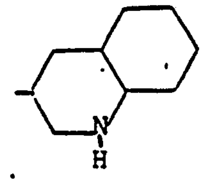

Par suite de la présence dans la formule d'un groupement oxime, les composés (1) existent sous 2 formes isomères syn et anti. Les isomères syn, dont l'activité thérapeutique est supérieure, sont les composés préférés.

Il est entendu que les composés (I) indiqués ci-dessus peuvent exister:

- soit sous la forme indiquée dans la formule (I)
- soit sous la forme tautomère (I'):

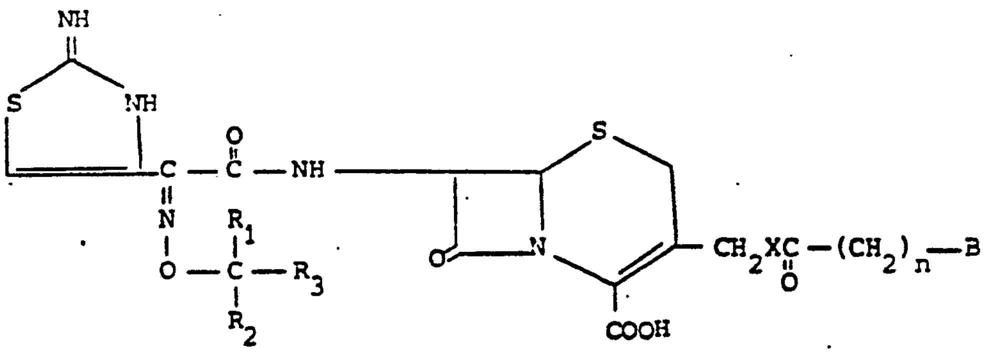

(I')

dans laquelle B, X, $R_1$, $R_2$, $R_3$ et n ont les significations indiquées précédemment.

Les sels des composés de formule I (ou I') font partie intégrante de l'invention.

Il s'agit aussi bien des sels avec les acides pharmaceutiquement acceptables qui peuvent se former avec la fonction aminée de la molécule que des sels alcalins, alcalino-terreux ou des sels d'aminoacides ou d'amines telles que la triéthylamine ou les éthanolamines qui sont susceptibles de se former avec le groupe carboxyle en position 4 du composé (I) ou lorsqu'il existe avec le groupe carboxyle présent dans le substituant de l'oxime ou encore avec les 2 groupes carboxyliques.

Il en est de même pour les esters facilement hydrolysables ou métaboliquement labiles dérivant de l'un ou de l'autre ou des 2 groupes carboxyliques éventuellement présents dans la molécule.

L'invention concerne également un procédé de préparation des composés de formule (I).

Les composés de formule (I) peuvent être préparés selon le schéma réactionnel suivant:

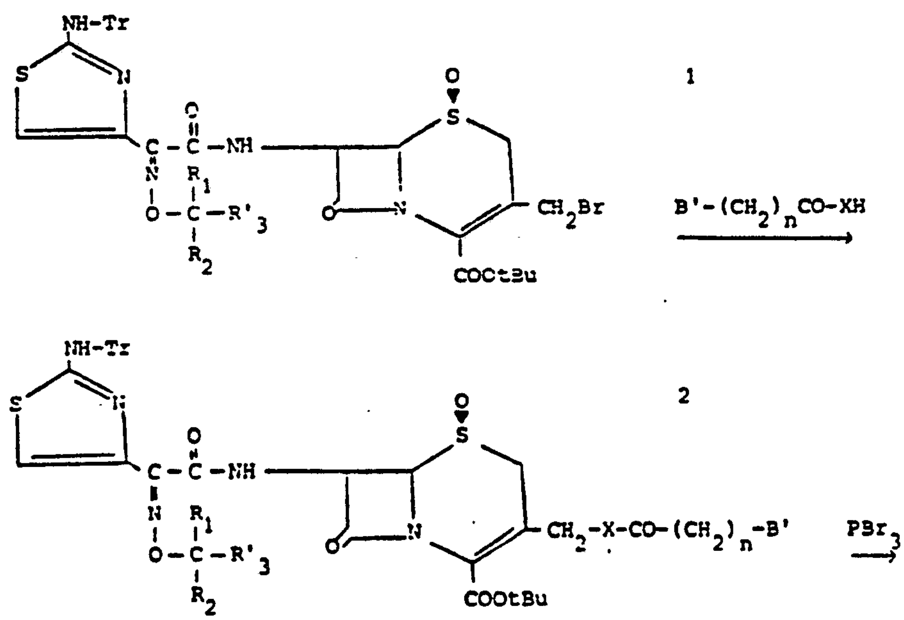

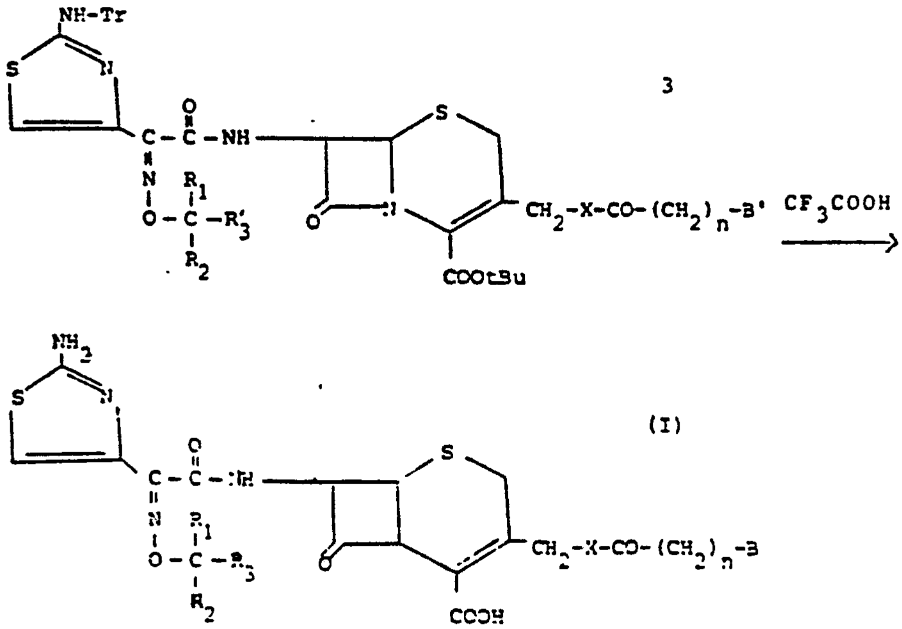


3

(I)

Dans ces formules, Tr représente un groupe protecteur de la fonction amine, de préférence le groupe trityle, tBu représente le groupe tertiobutyle, B' représente le groupe B dans lequel la fonction amine est protégée et $R'_3$ désigne l'hydrogène, un groupe cyclopropyle ou un groupe ester, facilement labile et de préférence un groupe -COOtBu. $R_1$, $R_2$, $R_3$, X, n et B ont les significations précédemment définies.

Sur le composé 1, on fait agir l'acide ou le thioacide $B-(CH_2)_n-COXH$ dont la fonction amine a été préalablement protégée, selon une méthode connue, par un groupement tel que le tertiobutoxycarbonyle ou le trichloroéthoxycarbonyle.

Le plus souvent, on utilise le sel de sodium de l'acide ou du thioacide et on opère alors au sein d'un solvant convenable, tel que le diméthylformamide ou le tétrahydrofuranne.

Dans le cas des thioacides, on peut utiliser le thioacide lui-même au lieu de son sel. On opère alors dans l'acétone anhydre en présence de bicarbonate de potassium et d'iodure de sodium.

A partir du composé 2 ainsi obtenu, l'action du tribromure de phosphore è basse température, au sein d'un solvant comme le dichlorométhane, permet d'éliminer le S-oxyde et conduit au composé 3.

Enfin, pour aboutir au composé (I), les groupes protecteurs sur les amines et le ou les esters sont éliminés par un procédé connu, en particulier par hydrolyse en milieu acide en utilisant, par exemple, l'acide trifluoracétique ou un mélange acide formique -acide chlorhydrique.

Le composé (I) est isolé directement sous forme de sel du groupement aminé B avec l'acide fort utilisé pour la déprotection, c'est-à-dire sous forme de trifluoracétate ou de chlorhydrate. On peut éventuellement transformer ce sel en un autre sel d'acide fort. Pour ce faire, on passe le trifluoracétate ou le chlorhydrate sur une résine échangeuse d'ions sous forme acide faible (formiate ou acétate par exemple), puis on traite la solution par l'acide fort dont on veut obtenir le sel (par exemple l'acide sulfurique ou l'acide phosphorique) et on isole le sel d'acide fort par lyophilisation.

A partir d'un tel sel, par action d'une base, on obtient le composé de formule (I) attendu.

Les produits de départ 1 peuvent être préparés selon les procédés connus et notamment par acylation de l'amino-7 bromométhyl-3 céphème-3 carboxylate de tertiobutyle-4 S-oxyde-1 par l'acide

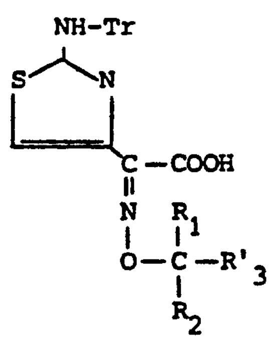

selon le procédé décrit dans le brevet européen N°60 745.

Dans le cas des thioacides, (X = S), un second procédé permet d'obtenir les composés I (X = S). Ce

procédé est résumé dans le schéma réactionnel suivant:

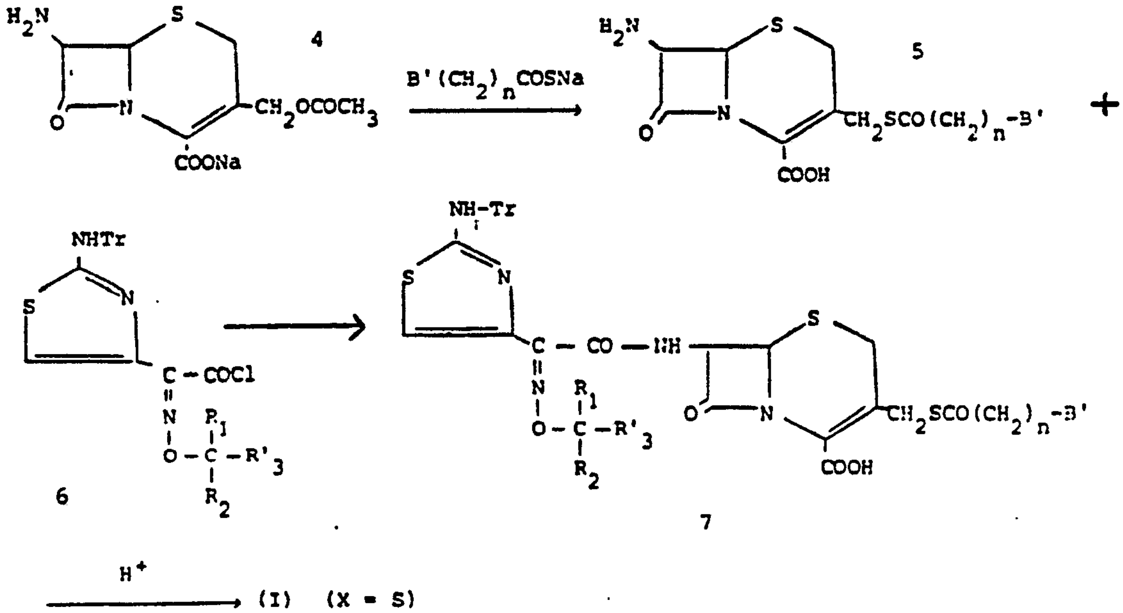


4 5 6 7

A partir du sel de sodium de l'acide amino-7 céphalosporanique 4, l'action du sel de sodium du thioacide protégé B'-$(CH_2)$-COSNa en milieu aqueux tamponné à pH : 6,3 - 6,4 permet d'obtenir le composé 5 convenablement substitué en position 3.

Ce dernier, après bloquage temporaire in situ de la fonction carboxylique par un groupe triméthylsilyl est acylé par le chlorure d'acide 6. On opère en solution dans le dichlorométhane en présence de triéthylamine et de diméthylaniline.

Le dérivé protégé 7 ainsi obtenu conduit au dérivé (1) (X = S) par traitement acide ainsi qu'il a été indiqué dans le premier procédé.

Les sels et esters des composés (I) de l'invention s'obtiennent à partir des composés (I) par des réactions connues en elles-mêmes.

Ainsi, les sels minéraux sont obtenus par action sur les composés (I) d'une base minérale telle que la soude ou la potasse ou le bicarbonate de sodium en quantité équimoléculaire; la réaction de salification est réalisée dans un solvant tel que l'eau ou l'éthanol et le sel obtenu est isolé par évaporation de la solution.

Les sels de bases organiques sont obtenus par action, sur une solution de l'acide I, dans un solvant ou un mélange de solvants convenables, d'une quantité équimoléculaire de la base organique. Le sel est isolé par précipitation avec l'éther.

Les esters sont obtenus par les procédés connus d'estérification; par exemple, on utilisera avantageusement l'action d'un dérivé halogéné sur un sel tel que le sel de sodium de l'acide; on réalisera de préférence la réaction dans un solvant capable de dissoudre le dérivé acide de départ, par exemple, dans le diméthylformamide.

Les isomères de forme syn et anti s'obtiennent par un choix convenable des réactifs.

Les exemples suivants permettent de mieux comprendre la portée de l'invention, sans toutefois la limiter.

Ainsi qu'il est habituel dans cette famille de composés, les produits selon l'invention ne présentent pas de point de fusion net, mais seulement des points de décomposition ne permettant pas de les caractériser.

Les produits seront donc caractérisés par leur spectre de résonance magnétique nucléaire enregistré à 250 MHz, l'étalon interne étant l'hexaméthyldisiloxane. Les spectres sont enregistrés dans le diméthylsulfoxyde deutérié : 10 mg dans 0,5 ml.

Les déplacements chimiques sont mesurés à ± 0,01 ppm et les constantes de couplage à ± 0,5 Hz.

Les abréviations suivantes seront utilisées:

- S : singulet
- D : doublet
- D de D : double de doublet
- S. e. : singulet élargi
- M : multiplet
- Q : quadruplet
- T : triplet
- AB : système AB
- J : représente la constante de couplage.

De plus, les microanalyses élémentaires ont été effectuées dans chaque cas et sont en accord avec les

formules indiquées.

Les spectres infra-rouge servent également à caractériser les produits obtenus. Ils sont enregistrés entre 4000 $cm^{-1}$ et 600 $cm^{-1}$ à partir d'une preparation constituée d'une pastille de bromure de potassium contenant le produit à une concentration d'environ 2 % ; lorsque le spectre est enregistré en solution à 1 % dans un solvant chloré, la nature de celui-ci est mentionnée. On opère la fréquence de vibration d'élongation des groupements carbonyles de la molécule (ν CO).

## EXEMPLE 1

Bis trifluoracétate de l'acide [(amino-2 thiazolyl-4)-2 (carboxy-2 propyl-2 oxyimino)-2 acétamido]-7 (pipéridyl-4 carbonyloxyméthyl)-3 céphème-3 carboxylique-4 isomère syn (SR 42800)

(I) $R_1 = R_2 = CH_3$ $R_3 = COOH$ $X = 0$ $n = 0$

B = 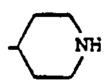

a) [(Tritylamino-2 thiazolyl-4)-2 (tertiobutoxycarbonyl-2 propyl-2 oxyimino)-2 acétamido]-7 [(tertiobutoxycarbonyl-1 pipéridyl-4) carbonyloxyméthyl]-3 céphème-3 carboxylate de tertiobutyle-4 S-oxyde 1 β isomère syn.

A une solution de 1,38 g de [(tritylamino-2 thiazolyl-4)-2 (tertiobutoxycarbonyl-2 propyl-2 oxyimino)-2 acétamido]-7 bromométhyl-3 céphème-3 carboxylate de tertiobutyle-4 S-oxyde-1β isomère syn, dans 20 ml de diméthylformamide anhydre, on ajoute 1 g d'acide tertiobutyloxycarbonyl-1 piperidyl-4 carboxylique et 1,5 g d'hydrogénocarbonate de potassium.

Après 17 heures d'agitation à température ambiante (20 - 25° C), le mélange réactionnel est versé sur 200 ml d'eau glacée. Après une agitation vigoureuse, les cristaux sont filtrés et lavés à l'eau. Ils sont ensuite repris par 70 ml de dichlorométhane. La phase organique est alors lavée par une solution saturée de chlorure de sodium, séchée sur sulfate de magnésium et évaporée. La laque obtenue est chromatographiée sur une colonne de 50 g de silice. On élue par un mélange dichlorométhane-acétate d'éthyle 90 - 10 (vol/vol). Après évaporation des fractions contenant le composé de trituration dans l'hexane, on obtient 1,3 g du composé attendu.

Spectre IR: ν CO

- 1805 $cm^{-1}$ : C = 0 en 8 du β lactame
- 1735 $cm^{-1}$ : C = 0 des esters tertiobutyliques et de l'ester en 3
- 1695 $cm^{-1}$ : C = 0 de l'amide en 7 et C = 0 du tertiobutoxycarbonyl protecteur de la piperidine.

Spectre de RMN à 250 MHz

1H à 8,75 ppm (S, NH Tr) - 1H à 8,10 ppm (D, J = 9 Hz, CONH) - 15H à 7,25 ppm (M, H aromatiques) - 1H à 6,73 ppm (S, H Thiazole) - 1H à 5,81 ppm (M, $H_7$) - 1H à 5,25 ppm (D, J = 13 Hz, $CH_2$ OCO) - 1H à 4,94 ppm (D, J = 4 Hz, $H_6$) - 1H à 4,55 ppm (D, J = 13 Hz, $CH_2OCO$) - 1H à 3,90 ppm (D, J = 17 Hz, $CH_2SO$) - 2H à 3,79 ppm (D, J = 12 Hz, HCN Boc équatoriaux) - 1H à 3,53 ppm (D, J = 17 Hz, $CH_2SO$) - 2H à 2,75 ppm (M, HCN Boc axiaux) - 1H à 2,50 ppm (M, $HCCO_2$) - 2H à 1,75 ppm (D, J = 12 Hz, $HCHCO_2$ équatoriaux) - 9H à 1,46 ppm (S, $CO_2$ tBu) - 2H à 1,40 ppm (M, $HCCHCO_2$ axiaux) - 6H à 1,39 ppm (S, $(CH_3)_2C$) - 9H à 1,36 ppm (S, $CO_2tBu$) - 9H à 1,29 ppm (S, Boc N).

b) [(Tritylamino-2 thiazolyl-4)-2 (tertiobutoxycarbonyl-2 propyl-2 oxyimino)-2 acétamido]-7 [(tertiobutoxycarbonyl-1 piperidyl-4) carbonyloxyméthyl]-3 céphème-3 carboxylate de tertiobutyle-4 isomère syn.

A la solution de 1 g du composé obtenu ci-dessus dans 20 ml de chlorure de méthylène refroidie à - 20° C, on ajoute en 5 minutes une solution de 0,15 ml de tribromure de phosphore dans 20 ml de chlorure de méthylène. En maintenant la température à - 20° C, on agite ensuite pendant 15 minutes, puis on ajoute 100 ml d'une solution à 5 % de bicarbonate de sodium dans l'eau. On agite le mélange pendant 3 heures à 0° C puis on décante la phase organique et on la sèche sur sulfate de magnésium.

On évapore le solvant sous vide et on chromatographie le résidu sur silice en éluant avec un mélange chlorure de méthylène-méthanol 100 - 0,5 vol/vol. Après évaporation des solvants, le produit brut est utilisé tel quel pour l'opération suivante.

c) SR 42800

Le produit provenant de l'opération ci-dessus est mis en solution dans 15 ml d'acide trifluoracétique. Après 45 minutes à 25° C, on évapore les cristaux et on les lave avec de l'éther.

Après séchage, on obtient le produit attendu.

Spectre de RMN

1H à 9,45 ppm (D, J = 9 Hz, -CO-NH) - 1H à 8,65 ppm et 1H à 8,40 ppm (S.e., $NH^+{}_2$ pipéridinium) - 2H à 7,30

ppm (S.e. $NH_2$ thiazole) - 1H à 6,70 ppm (S, H thiazole) - 1H à 5,80 ppm (D de D, $J_1$ = 9 Hz, $J_2$ = 4 Hz, $H_7$) - 1H à 5,10 ppm (D, J = 4 Hz, $H_6$) - 2H à 4,99 et 4,70 ppm (D, J = 13 Hz, $CH_2OCO$) - 2H à 3,52 ppm (AB, $J_{AB}$ = 17 Hz, $CH_2$-S) - 2H à 3,25 ppm (M, HαN pipéridine) - 2H à 2,90 ppm (M, HαN pipéridine) - 1H à 2,63 ppm (M, HC-COO) - 2H à 1,95 ppm (M, HβN pipéridine) - 2H à 1,70 ppm (HβN pipéridine) - 6H à 1,40 ppm (2S, $(CH_3)_2$-C-).

**EXEMPLES 2 A 63**

En opérant comme dans l'exemple 1, on prépare les composés selon l'invention, sous forme de trifluoroacétate, décrits dans le tableau I, ci-après.

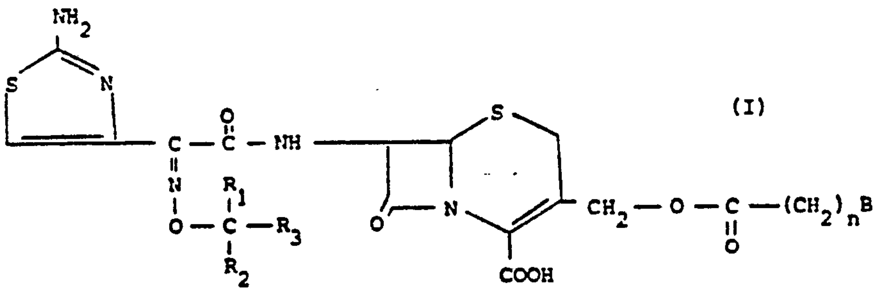

(I)

,TFA

Ces composés sont identifiés par un numéro de référence. On donne pour chacun d'entre eux les valeurs de $R_1$, $R_2$, $R_3$, B et n et le spectre de RMN.

Pour 3, dernier produit intermédiaire avant le déblocage des fonctions acides et amines de la molécule, on donne également les caractéristiques du spectre infrarouge. Les longueurs d'ondes indiquées en cm correspondent, dans l'ordre, aux fréquences de vibrations d'élongation du carbonyl en 8 du beta lactame, des esters tertiobutyliques et de l'ester en 3, de l'amide en 7 et du carbamate protecteur de l'amine. Lorsque 2 longueurs d'onde seulement sont indiquées, la seconde correspond à une bande large qui recouvre les fréquences de vibration d'élongation à la fois des esters, de l'amide et du carbamate protecteur de l'amine.

La liste des spectres de RMN des composés cités dans le tableau I est donnée à la suite de ce tableau.

**TABLEAU 1**

| N° SR | n | $R_1$ | $R_2$ | $R_3$ | B | IR Intermédiaire 3 ν CO $cm^{-1}$ | N° RMN |
|---|---|---|---|---|---|---|---|
| 42 796 | 0 | $CH_3$ | $CH_3$ | COOH | $(CH_2)_2-NH_2$ | 1795 - 1725<br>1690 | 1 |
| 42 797 | 0 | $CH_3$ | $CH_3$ | COOH | $(CH_2)_3-NH_2$ | 1795 - 1725<br>1690 | 2 |
| 42 798 | 0 | $CH_3$ | $CH_3$ | COOH | $(CH_2)_4-NH_2$ | 1790 - 1725<br>1690 | 3 |
| 42 799 | 0 | $CH_3$ | $CH_3$ | COOH | 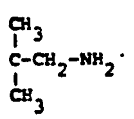 | 1795 - 1720 | 4 |
| 42 801 | 0 | $CH_3$ | $CH_3$ | COOH | 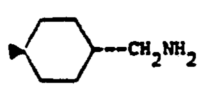 | 1790 - 1725 | 5 |

| | | | | | | |
|---|---|---|---|---|---|---|
| 42 803 | 0 | $CH_3$ | $CH_3$ COOH | 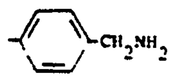 | 1790 - 1720 | 6 |
| 42 804 | 0 | $CH_3$ | $CH_3$ COOH | 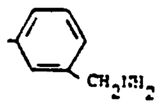 | 1790 - 1720 | 7 |
| 42 805 | 0 | $CH_3$ | $CH_3$ COOH | 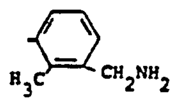 | 1790 - 1720 | 8 |
| 42 806 | 0 | $CH_3$ | $CH_3$ COOH | 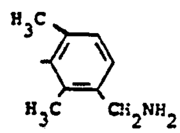 | 1795 - 1725 | 9 |
| 42 807 | 0 | $CH_3$ | $CH_3$ COOH | 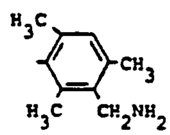 | 1795 - 1725 | 10 |
| 42 808 | 0 | $CH_3$ | $CH_3$ COOH | 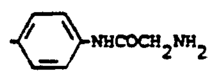 | 1790 - 1715 | 11 |
| 42 809 | 0 | $CH_3$ | $CH_3$ COOH | 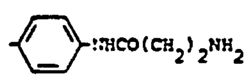 | 1795 - 1710 | 12 |
| 42 810 | 0 | $CH_3$ | $CH_3$ COOH | 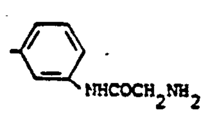 | 1790 - 1720 | 13 |
| 42 883 | 0 | H | H H | 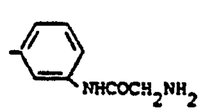 | 1790 - 1720<br>1690 | 14 |
| 42 885 | 0 | H | H H | 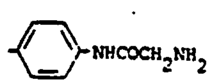 | 1790 - 1715 | 15 |

| | | | | | | | |
|---|---|---|---|---|---|---|---|
| 42 893 | 0 | H | H | H | 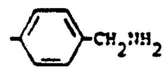 | 1790 - 1720 | 16 |
| 42 895 | 0 | H | H | H | 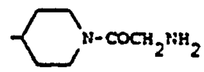 | 1790 - 1715<br>1655<br>($CH_2Cl_2$) | 17 |
| 42 897 | 1 | H | H | H | 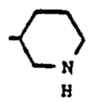 | 1790 - 1725<br>1680<br>($CH_2Cl_2$) | 18 |
| 42 898 | 0 | H | H | H | $CH_2SCH_2CH_2NH_2$ | 1790 - 1715<br>($CH_2Cl_2$) | 19 |
| 42 877 | 0 | $CH_3$ | $CH_3$ | COOH | 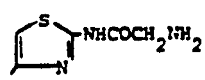 | 1790 - 1720 | 20 |
| 42 897 | 0 | $CH_3$ | $CH_3$ | COOH | $CH_2SCH_2CH_2NH_2$ | 1790 - 1715<br>($CH_2Cl_2$) | 21 |
| 42 880 | 1 | $CH_3$ | $CH_3$ | COOH | 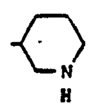 | 1790 - 1720<br>1680-($CH_2Cl_2$) | 22 |
| 42 881 | 0 | $CH_3$ | $CH_3$ | COOH | 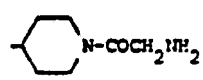 | 1790 - 1720<br>($CH_2Cl_2$) | 23 |
| 42 942 | 0 | $CH_3$ | $CH_3$ | COOH | 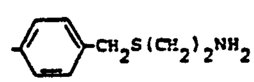 | 1790 - 1720 | 24 |
| 42 946 | 0 | H | H | H | 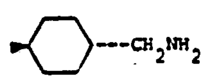 | 1790 - 1720 | 25 |

| | | | | | | | |
|---|---|---|---|---|---|---|---|
| 42 950 | 0 | H | H | H | 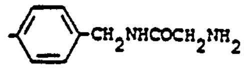 | - | 26 |
| 42 961 | 0 | $CH_3$ | $CH_3$ | COOH | 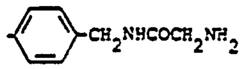 | 1790 - 1720<br>1680 | 27 |
| 42 963 | 0 | $CH_3$ | $CH_3$ | COOH | 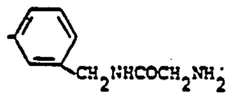 | 1790 - 1725 | 28 |
| 42 965 | 0 | H | H | H | 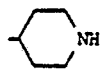 | 1790 - 1725<br>1685 | 29 |
| 42 971 | 0 | H | H | H | 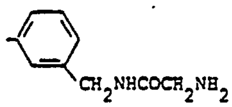 | - | 30 |
| 43 013 | 0 | $CH_3$ | $CH_3$ | COOH | 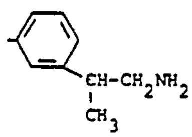 | 1790 - 1720 | 31 |
| 43 016 | 0 | 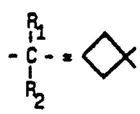 | | COOH | 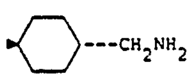 | 1795 - 1725 | 32 |
| 43 029 | 0 | H | H | H | 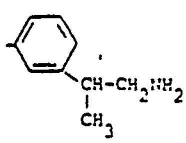 | 1785 - 1700 | 33 |
| 42 948 | 0 | H | H | H | 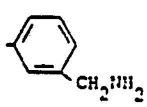 | 1790 - 1720 | 34 |
| 43 147 | 0 | H | H | H | 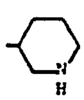 | 1790 - 1725 | 35 |

| | | | | | | | |
|---|---|---|---|---|---|---|---|
| 43 179 | 0 | H | H | H | 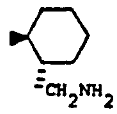 | 1790 - 1720 | 36 |
| 43 183 | 0 | H | H | H | 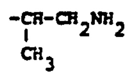 | 1790 - 1720 | 37 |
| 43 185 | 0 | H | H | H | 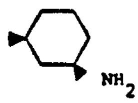 | 1790 - 1720 | 38 |
| 43 187 | 0 | H | H | H | 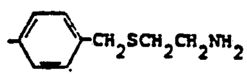 | 1790 - 1715 | 39 |
| 43 189 | 0 | H | H | H | 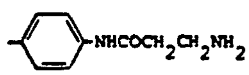 | 1790 - 1710 | 40 |
| 43 224 | 1 | H | H | H | 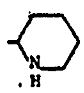 | 1790 - 1725<br>1680<br>($CH_2Cl_2$ | 41 |
| 43 226 | 1 | H | H | H | 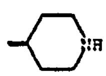 | 1790 - 1725<br>1680 ($CH_2Cl_2$) | 42 |
| 43 228 | 0 | H | H | H | 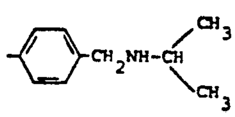 | 1790 - 1720<br>1685 | 43 |
| 43 230 | 0 | H | H | H | 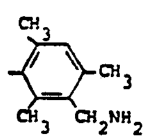 | 1785 - 1720 | 44 |
| 43 232 | 1 | H | H | H | 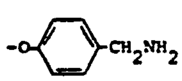 | 1785 - 1715<br>1685 | 45 |
| 43 323 | 0 | H | H | H | 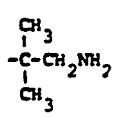 | 1790 - 1720 | 46 |

| | | | | | | | |
|---|---|---|---|---|---|---|---|
| 43 325 | 0 | H | H | H | 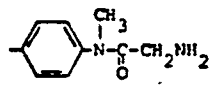 | 1790 - 1720<br>1675 | 47 |
| 43 329 | 0 | $CH_3$ | $CH_3$ | COOH | 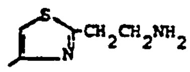 | 1790 - 1720 | 48 |
| 43 330 | 0 | H | H | H | 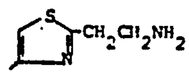 | 1785 - 1715 | 49 |
| 43 334 | 0 | $CH_3$ | $CH_3$ | COOH | 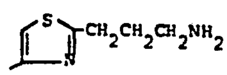 | 1790 - 1715 | 50 |
| 43 336 | 0 | H | H | H | 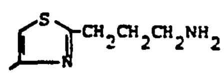 | 1785 - 1720 | 51 |
| 43 338 | 0 | H | H | H | 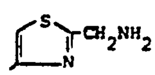 | 1785 - 1720 | 52 |
| 43 415 | 1 | H | H | 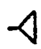 | 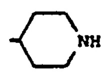 | 1780 - 1720<br>1680 ($CH_2Cl_2$) | 53 |
| 43 417 | 0 | H | H | H | $-(CH_2)_5NH_2$ | 1790 - 1720 | 54 |
| 43 419 | 0 | H | H | H | $-(CH_2)_7NH_2$ | 1790 - 1715<br>($CH_2Cl_2$) | 55 |
| 43 464 | 2 | H | H | H | 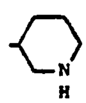 | 1785 - 1720<br>1665 ($CH_2Cl_2$) | 56 |
| 43 465 | 2 | $CH_3$ | $CH_3$ | COOH | 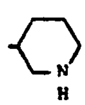 | 1785 - 1720<br>1675 ($CH_2Cl_2$) | 57 |
| 43 559 | 2 | H | H | H | 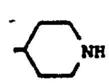 | 1790 - 1720<br>1680 ($CH_2Cl_2$) | 58 |

| | | | | | | | |
|---|---|---|---|---|---|---|---|
| 43 561 | 2 | $CH_3$ | $CH_3$ | COOH | 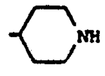 | 1790 - 1720<br>1675 ($CH_2Cl_2$) | 59 |
| 43 563 | 0 | $CH_3$ | $CH_3$ | COOH | 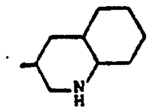 | 1785 - 1720<br>1685 ($CH_2Cl_2$) | 60 |
| 43 666 | 0 | H | H | H | 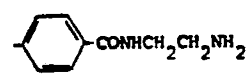 | 1785 - 1715<br>1685 ($CH_2Cl_2$) | 61 |
| 43 668 | 0 | $CH_3$ | $CH_3$ | COOH | 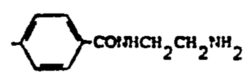 | 1785 - 1715<br>1685 ($CH_2Cl_2$) | 62 |

RMN n° 1

1H à 9,41 ppm (D, J = 9 Hz, CO-NH) - 3H à 7,86 ppm (S.e., $N^+H_3$) - 2H à 7,30 ppm (S.e., $NH_2$ thiazole) - 1H à 6,71 ppm (S, H thiazole) - 1H à 5,80 ppm (D de D, $J_1$ = 9 Hz, $J_2$ = 4 Hz, $H_7$) - 1H à 5,09 ppm (D, J = 4 Hz, $H_6$) - 1H à 5,05 ppm et 1H à 4,72 ppm (D, J = 13 Hz, $CH_2OCO$) - 2H à 3,51 ppm (AB, $J_{AB}$ = 17 Hz, $CH_2S$) - 2H à 3,00 ppm (M, $CH_2$-$N^+H_3$) - 2H à 2,62 ppm (T, J = 7 Hz, $CH_2$-$CH_2$-$N^+$) - 6H à 1,42 ppm (2S, $(CH_3)_2$-C).

RMN n° 2

1H à 9,45 ppm (D, J = 9 Hz, CONH) - 3H à 7,75 ppm (S.e., $CH_2N^+H_3$) - 2H à 7,45 ppm (S.e., $NH_2$ thiazole) - 1H à 6,70 ppm (S, H thiazole) - 1H à 5,80 ppm (D de D, $J_1$ = 9 Hz, $J_2$ = 4 Hz, $H_7$) - 1H à 5,14 ppm (D, J = 4 Hz, $H_6$) - 1H à 4,98 ppm et 1H à 4,69 ppm (D, J = 13 Hz, $CH_2OCO$) - 2H à 3,52 ppm (AB, $J_{AB}$ = 17 Hz, $CH_2S$) - 2H à 2,75 ppm (M, $CH_2N^+$) - 2H à 2,40 ppm (M, $OCOCH_2$) - 2H à 1,75 ppm (M, $CH_2$-$CH_2N^+$) - 6H à 1,40 ppm (2S, $(CH_3)_2$-C).

RMN n° 3

1H à 9,45 ppm (D, J = 9 Hz, CONH) - 3H à 7,70 ppm (S.e., $CH_2N^+H_3$) - 2H à 7,45 ppm (S.e., $NH_2$ thiazole) - 1H à 6,70 ppm (H thiazole) - 1H à 5,80 ppm (D de D, $J_1$ = 9 Hz, $J_2$ = 4 Hz, $H_7$) - 1H à 5,13 ppm (D, J = 4 Hz, $H_6$) - 1H à 4,98 ppm et 1H à 4,65 ppm (D, J = 13 Hz, $CH_2OCO$) - 2H à 3,52 ppm (AB, $J_{AB}$ = 17 Hz, $CH_2S$) - 2H à 2,75 ppm (M, $CH_2N^+$) - 2H à 2,35 ppm (M, $OCOCH_2$) - 4H à 1,50 ppm (M, $CH_2$-$CH_2$-$CH_2N^+$) - 6H à 1,40 ppm (2S, $(CH_3)_2$-C).

RMN n° 4

1H à 9,45 ppm (D, J = 9 Hz, CONH) - 3H à 7,95 ppm (S.e., $CH_2N^+H_3$) - 2H à 7,40 ppm (S.e., $NH_2$ thiazole) - 1H à 6,70 ppm (S, H thiazole) - 1H à 5,82 ppm (D de D, $J_1$ = 9 Hz, $J_2$ = 4 Hz, $H_7$) - 1H à 5,13 ppm (D, J = 4 Hz, $H_6$) - 1H à 5,05 ppm et 1H à 4,70 ppm (D, J = 13 Hz, $CH_2OCO$) - 2H à 3,55 ppm (AB, $J_{AB}$ = 17 Hz, $CH_2S$) - 2H à 2,95 ppm (M, $CH_2$+N) - 6H à 1,40 ppm (2S, $(CH_3)_2$C-ON) - 6H à 1,15 ppm (S, $(CH_3)_2$C-$CH_2$).

RMN n° 5

1H à 9,45 ppm (D, J = 9 Hz, CONH) - 3H à 7,70 ppm (S.e., $CH_2$-$N^+H_3$) - 2H à 7,45 ppm (S.e., $NH_2$ thiazole) - 1H à 6,70 ppm (S, H thiazole) - 1H à 5,82 ppm (D de D, $J_1$ = 9 Hz, $J_2$ = 4 Hz, $H_7$) - 1H à 5,10 ppm (D, J = 4 Hz, $H_6$) - 1H à 4,98 ppm et 1H à 4,65 ppm (D, J = 13 Hz, $CH_2OCO$) - 2H à 3,55 ppm (AB, $J_{AB}$ = 17 Hz, $CH_2S$) - 2H à 2,65 ppm (M, $CH_2N^+$) - 1H à 2,25 ppm (M, $OCOCH_2$) - 4H à 1,90 ppm (M, cyclohexane) - 4H à 1,25 ppm (M, cyclohexane) - 6H à 1,40 ppm (2S, $(CH_3)_2$C) - 2H à 1,00 ppm (cyclohexane).

RMN n° 6

1H à 9,42 ppm (D, J = 9 Hz, NHCO) - 3H à 8,30 ppm (S.e., $CH_2N^+H_3$) - 2H à 8,00 ppm (D, J = 8 Hz, H aromatiques ortho $CO_2$) - 2H à 7,55 ppm (D, J = 8 Hz, H aromatiques méta $CO_2$) - 2H à 7,30 ppm (S.e., $NH_2$ thiazole) - 1H à 6,70 ppm (S, H thiazole) - 1H à 5,84 ppm (D de D, $J_1$ = 9 Hz, $J_2$ = 4 Hz, $H_7$) - 1H à 5,16 ppm (D, J = 4 Hz, $H_6$) - 1H à 4,82 ppm (D, J = 13 Hz, $OCOCH_2$) - 1H à 5,25 ppm (D, J = 13 Hz, O CO $CH_2$) - 2H à 4,08 ppm (M, Ar-$CH_2$-$NH_2$) - 2H à 3,67 ppm (AB, $J_{AB}$ = 17 Hz, $CH_2S$) - 6H à 1,42 ppm (2S, $(CH_3)_2$ Cl).

RMN n° 7

1H à 9,45 ppm (D, J = 9 Hz, CONH) - 3H à 8,25 ppm (S.e., $CH_2N{+}H_3$) - 1H à 8,05 ppm (S, H aromatique ortho $CO_2$ et ortho $CH_2NH_2$) - 1H à 7,95 ppm (D, J = 8 Hz, H aromatique para $CH_2NH_2$) - 1H à 7,70 ppm (D, J = 8 Hz, H aromatique para $CO_2$) - 1H à 7,55 ppm (T, J = 8 Hz, H aromatique méta $CO_2$) - 2H à 7,40 ppm (S.e., $NH_2$ thiazole) - 1H à 6,70 ppm (S, H thiazole) - 1H à 5,85 ppm (D de D, $J_1$ = 9 Hz, $J_2$ = 4 Hz, $H_7$) - 1H à 5,25 ppm (D, J = 13 Hz, $CH_2OCO$) - 1H à 5,15 ppm (D, J = 4 Hz, $H_6$) - 1H à 4,95 ppm (D, J = 13 Hz, $CH_2OCO$) - 2H à 4,10 ppm (M, Ar $CH_2$ N) - 2H à 3,70 ppm (AB, $J_{AB}$ = 17 Hz, $CH_2S$) - 6H à 1,40 ppm (2S, $(CH_3)_2C$).

RMN n° 8

1H à 9,45 ppm (D, J = 9 Hz, COOH) - 3H à 8,25 ppm (S.e., $CH_2N{+}H_3$) - 1H à 7,70 ppm (D, J = 8 Hz, H aromatique ortho $CO_2$) - 1H à 7,55 ppm (D, J = 8 Hz, H aromatique para $CO_2$) - 1H à 7,35 ppm (T, J = 8 Hz, H aromatique méta $CO_2$) - 2H à 7,30 ppm (se, $NH_2$ thiazole) - 1H à 6,70 ppm (S, H thiazole) - 1H à 5,85 ppm (D de D, $J_1$ = 9 Hz, $J_2$ = 4 Hz, $H_7$) - 2H à 5,17 ppm (M, $H_6$ et $CH_2OCO$) - 1H à 4,90 ppm (D, J = 13 Hz, $CH_2OCO$) - 2H à 4,08 ppm (M, Ar $CH_2$ N) - 2H à 3,65 ppm (AB, $J_{AB}$ = 17 Hz, $CH_2S$) - 3H à 2,40 ppm (S, $CH_3$-Ar) - 6H à 1,40 ppm (2S, $(CH_3)_2C$).

RMN n° 9

1H à 9,45 ppm (D, J = 9 Hz, NHCO) - 3H à 8,15 ppm (S.e., $CH_2N{+}H_3$) - 2H à 7,40 ppm (S.e., $NH_2$ thiazole) - 1H à 7,30 ppm (D, J = 8 Hz, H aromatique para $CO_2$) - 1H à 7,15 ppm (D, J = 8 Hz, H aromatique méta $CO_2$) - 1H à 6,70 ppm (S, H thiazole) - 1H à 5,85 ppm (D de D, $J_1$ = 9 Hz, $J_2$ = 4 Hz, $H_7$) - 2H à 5,15 ppm (M, $H_6$ et $CH_2OCO$) - 1H à 4,98 ppm (D, J = 13 Hz, $CH_2OCO$) - 2H à 4,00 ppm (M, Ar $CH_2$ N) - 2H à 3,60 ppm (AB, $J_{AB}$ = 17 Hz, $CH_2S$) - 6H à 2,20 ppm (S, $2CH_3$ - Ar) - 6H à 1,40 ppm (2S, $(CH_3)_2C$).

RMN n° 10

1H à 9,43 ppm (D, J = 9 Hz, CONH) - 3H à 8,00 ppm (S.e., $CH_2$-N+$H_3$) - 2H à 7,40 ppm (S.e., $NH_2$ thiazole) - 1H à 7,00 ppm (S, H aromatique) - 1H à 6,70 ppm (S, H thiazole) - 1H à 5,85 ppm (D de D, $J_1$ = 9 Hz, $J_2$ = 4 Hz, $H_7$) - 2H à 5,15 ppm (M, $H_6$ et $CH_2OCO$) - 1H à 5,00 ppm (D, J = 13 Hz, $CH_2OCO$) - 2H à 4,00 ppm (M, $CH_2N$+) - 2H à 3,55 ppm (AB, $J_{AB}$ = 17 Hz, $CH_2S$) - 3H à 2,30 ppm (S, $CH_3$ Ar) - 3H à 2,25 ppm (S, $CH_3$ Ar) - 3H à 2,75 ppm (S, $CH_3$ Ar) - 6H à 1,40 ppm (2S $(CH_3)_2C$).

RMN n° 11

1H à 10,8 ppm (S, Ar NHCO) - 1H à 9,45 ppm (D, J = 9 Hz, NHCO) - 3H à 8,15 ppm (S.e., $CH_2N{+}H_3$) - 2H à 7,95 ppm (D, J = 8 Hz, H aromatiques ortho $CO_2$) - 2H à 7,67 ppm (D, J = 8 Hz, H aromatiques méta $CO_2$) - 2H à 7,45 ppm (S.e., $NH_2$ thiazole) - 1H à 6,70 ppm (S, H thiazole) - 1H à 5,81 ppm (D de D, $J_1$ = 9 Hz, $J_2$ = 4 Hz, $H_7$) - 2H à 5,20 ppm (M, $H_6$, et $CH_2OCO$) - 1H à 4,87 ppm (D, J = 13 Hz, $CH_2OCO$) - 2H à 3,80 ppm (M, $COCH_2N$) - 2H à 3,68 ppm (AB, $J_{AB}$ = 17 Hz, $CH_2S$) - 6H à 1,43 ppm (2S, $(CH_3)_2C$).

RMN n° 12

1H à 10,46 ppm (S, Ar NHCO) - 1H à 9,40 ppm (D, J = 9 Hz, CONH) - 2H à 7,92 ppm (D, J = 8 Hz, H aromatiques ortho $CO_2$) - 5H à 7,75 ppm (M, $CH_2N{+}H_3$ et H aromatiques méta $CO_2$) - 2H à 7,25 ppm (S.e., $NH_2$ thiazole) - 1H à 6,71 ppm (S, H thiazole) - 1H à 5,83 ppm (D de D, $J_1$ = 9 Hz, $J_2$ = 4 Hz, $H_7$) - 2H à 5,20 ppm (M, $H_6$ et $CH_2OCO$) - 1H à 4,88 ppm (D, J = 13 Hz, $CH_2OCO$) - 2H à 3,65 ppm (AB, $J_{AB}$ = 17 Hz, $CH_2S$) - 2H à 3,05 ppm (M, $CH_2CH_2N$) - 2H à 2,72 ppm (T, J = 7 Hz, $CH_2CH_2N$) - 6H à 1,40 ppm (2S, $(CH_3)_2C$).

RMN n° 13

1H à 10,70 ppm (S, Ar NHCO) - 1H à 9,45 ppm (D, J = 9 Hz, CONH) - 4H à 8,18 ppm (S.e., $CH_2N{+}H_3$ et H aromatique ortho $CO_2$ et ortho NH) - 1H à 7,85 ppm (D, J = 8 Hz, H aromatique para NH) - 1H à 7,67 ppm (D, J = 8 Hz, H aromatique para $CO_2$) - 1H à 7,50 ppm (T, J = 8 Hz, H aromatique méta $CO_2$') - 1H à 6,70 ppm (S, H thiazole) - 1H à 5,80 ppm (D de D, $J_1$ = 9 Hz, $J_2$ = 4 Hz, $H_7$) - 1H à 5,25 ppm (D, J = 13 Hz, $CH_2OCO$) - 1H à 5,15 ppm (D, J = 4 Hz, $H_6$) - 1H à 4,90 ppm (D, J = 13 Hz, $CH_2OCO$) - 2H à 3,75 ppm (M, CO-$CH_2$-N) - 2H à 3,68 ppm (AB, $J_{AB}$ = 17 Hz, $CH_2S$) - 6H à 1,43 ppm (2S, $(CH_3)_2C$).

RMN n° 14

1H à 10,70 ppm (S, Ar NHCO) - 1H à 9,55 ppm (D, J = 9 Hz, NH-CO) - 4H à 8,18 ppm (S.e., $CH_2N{+}H_3$, H aromatique ortho $CO_2$ et ortho NH) - 1H à 7,82 ppm (D, J = 8 Hz, H aromatique para NH) - 1H à 7,72 ppm (D, J = 8 Hz, H aromatique para $CO_2$) - 1H à 7,50 ppm (T, J = 8 Hz, H aromatique méta $CO_2$) - 2H à 7,20 ppm (S.e., $NH_2$ thiazole) - 1H à 6,69 ppm (S, H thiazole) - 1H à 5,80 ppm (D de D, $J_1$ = 9 Hz, $J_2$ = 4 Hz, $H_7$) - 1H à 5,25 ppm (D, J = 13 Hz, $CH_2OCO$) - 1H à 5,15 ppm (D, J = 4 Hz, $H_6$) - 1H à 4,93 ppm (D, J = 13 Hz, $CH_2OCO$) - 5H à 3,80 ppm (2S, $CH_3ON$ et $COCH_2N$) - 2H à 3,63 ppm (AB, $J_{AB}$ = 17 Hz, $CH_2S$).

RMN n° 15

1H à 10,80 ppm (S, Ar-NH-CO) - 1H à 9,55 ppm (D, J = 9 Hz, CONH) - 3H à 8,20 ppm (S.e., $CH_2N{+}H_3$) - 2H à 7,95 ppm (D, J = 8 Hz, H aromatique ortho $CO_2$) - 2H à 7,70 ppm (D, J = 8 Hz, H aromatique méta $CO_2$) - 2H à 7,20 ppm (S.e., $NH_2$ thiazole) - 1H à 6,70 ppm (S, H thiazole) - 1H à 5,75 ppm (D de D, $J_1$ = 9 Hz, $J_2$ = 4 Hz, $H_7$) - 1H à 5,20 ppm (D, J = 13 Hz, $CH_2OCO$) - 1H à 5,10 ppm (D, J = 4 Hz, $H_6$) - 1H à 4,92 ppm (D, J = 13 Hz,

$CH_2OCO$) - 5H à 3,85 ppm (S.e., N-$OCH_3$ et $COCH_2N$) - 2H à 3,60 ppm (AB, $J_{AB}$ = 17 Hz, $CH_2S$).

RMN n° 16

1H à 9,60 ppm (D, J = 9 Hz, NHCO) - 3H à 8,30 ppm (S.e., $CH_2N^+H_3$) - 2H à 7,97 ppm (D, J = 8 Hz, H aromatiques ortho $CO_2$) - 2H à 7,55 ppm (D, J = 8 Hz H aromatiques méta $CO_2$) - 2H à 7,20 ppm (S.e., $NH_2$ thiazole) - 1H à 6,70 ppm (S, H thiazole) - 1H à 5,75 ppm (D de D, $J_1$ = 9 Hz, $J_2$ = 4 Hz, $H_7$) - 1H à 5,25 ppm (D, J = 13 Hz, $CH_2OCO$) - 1H à 5,13 ppm (D, J = 4 Hz, $H_6$) - 1H à 4,93 ppm (D, J = 13 Hz, $CH_2OCO$) - 2H à 4,09 ppm (M, Ar $CH_2$ N) - 3H à 3,80 ppm (S, $CH_3ON$) - 2H à 3,66 ppm (AB, $J_{AB}$ = 17 Hz, $CH_2S$).

RMN n° 17

1H à 9,55 ppm (D, J = 9 Hz, CONH) - 3H à 8,00 ppm (S.e., $CH_2N^+H_3$) - 1H à 6,71 ppm (S, H thiazole) - 1H à 5,76 ppm (D de D, $J_1$ = 9 Hz, $J_2$ = 4 Hz, $H_7$) - 1H à 5,09 ppm (D, J = 4 Hz, $H_6$) - 1H à 4,97 ppm et 1H à 4,68 ppm (D, J = 13 Hz, $CH_2OCO$) - 1H à 4,12 ppm (M, H pipéridine α N équatorial) - 5H à 3,80 ppm (M, $CH_3ON$ et CO-$CH_2N$) - 3H à 3,50 ppm (M, $CH_2S$ et H pipéridine α N équatorial) - 1H à 3,05 ppm (M, H pipéridine α N axial) - 1H à 2,80 ppm (M, H pipéridine α N axial) - 1H à 2,66 ppm (M, H pipéridine γ N) - 2H à 1,85 ppm et 2H à 1,50 ppm (M, H, pipéridine β N).

RMN n° 18

1H à 9,55 ppm (D, J = 9 Hz, NHCO) - 1H à 8,50 ppm et 1H à 8,40 ppm (S.e., $N^+H_2$ pipéridinium) - 2H à 7,30 ppm (S.e., $NH_2$ thiazole) - 1H à 6,70 ppm (S, H thiazole) - 1H à 5,75 ppm (D de D, $J_1$ = 9 Hz, $J_2$ = 4 Hz, $H_7$) - 1H à 5,08 ppm (D, J = 4 Hz, $H_6$) - 1H à 4,95 ppm et 1H à 4,56 ppm (D, J = 13 Hz, $CH_2OCO$) - 3H à 3,80 ppm (S, $CH_3ON$) - 2H à 3,50 ppm (AB, $J_{AB}$ = 17 Hz, $CH_2S$) - 2H à 3,15 ppm (M, H pipéridine α N équatoriaux) - 2H à 2,65 ppm (M, H pipéridine α N axiaux) - 2H à 2,36 ppm (M, $OCOCH_2$) - 1H à 2,00 ppm (M, H pipéridine

$CH_2$-HC) - 4H entre 1,2 et 1,7 ppm (M, H pipéridine β et γ N).

RMN n° 19

1H à 9,58 ppm (D, J = 9 Hz, NHCO) - 3H à 7,80 ppm (S.e., $CH_2N^+H_3$) - 1H à 6,71 ppm (S, H thiazole) - 1H à 5,75 ppm (D de D, $J_1$ = 9 Hz, $J_2$ = 4 Hz, $H_7$) - 1H à 5,10 ppm (D, J = 4 Hz, $H_6$) - 1H à 5,03 ppm et 1H à 4,75 ppm (D, J = 13 Hz, $CH_2OCO$) - 3H à 3,84 ppm (S, $CH_3ON$) - 1H à 3,60 ppm (D, J = 17 Hz, $CH_2S$) - 3H à 3,48 ppm (M, $CH_2S$ et $OCOCH_2S$) - 2H à 3,00 ppm (M, $CH_2N^+H_3$) - 2H à 2,75 ppm (T, J = 7 Hz, $CH_2$-$CH_2$-$N^+H_3$).

RMN n° 20

1H à 12,80 ppm (S.e., thiazole-NHCO) - 1H à 9,45 ppm (D, J = 9 Hz, NHCO) - 3H à 8,40 ppm (S.e., $CH_2N^+H_3$) - 1H à 8,20 ppm (S, H thiazole ester) - 2H à 7,40 ppm (S.e., $NH_2$ thiazole) - 1H à 6,68 ppm (S, H thiazole aminé) - 1H à 5,88 ppm (D de D, $J_1$ = 9 Hz, $J_2$ = 4 Hz, $H_7$) - 2H à 5,20 ppm (M, $H_6$ et $CH_2OCO$) - 1H à 4,90 ppm (D, J = 13 Hz, $CH_2OCO$) - 2H à 3,84 ppm (S.e., $COCH_2N$) - 2H à 3,61 ppm (AB, $J_{AB}$ = 17 Hz, $CH_2S$) - 6H à 1,42 ppm (2S, $(CH_3)_2C$).

RMN n° 21

1H à 9,40 ppm (D, J = 9 Hz, CONH) - 3H à 7,80 ppm (S.e., $CH_2N^+H_3$) - 2H à 7,40 ppm (S.e., $NH_2$ thiazole) - 1H à 6,68 ppm (S, H thiazole) - 1H à 5,85 ppm (D de D, $J_1$ = 9 Hz, $J_2$ = 4 Hz, $H_7$) - 1H à 5,13 ppm (D, J = 4 Hz, $H_6$) - 1H à 5,04 ppm et 1H à 4,75 ppm (D, J = 13 Hz, $CH_2OCO$) - 1H à 3,60 ppm (D, J = 17 Hz, $CH_2S$) - 3H à 3,50 ppm (M, $CH_2S$ et $OCOCH_2S$-) - 2H à 2,98 ppm (M, $CH_2N$) - 2H à 2,75 ppm (M, $CH_2CH_2N$) - 6H à 1,42 ppm (2S, $(CH_3)_2C$).

RMN n° 22

1H à 9,40 ppm (D, J = 9 Hz, NHCO) - 1H à 8,60 ppm et 1H à 8,40 ppm (S.e., $N^+H_2$ pipéridinium) - 1H à 6,70 ppm (S, H thiazole) - 1H à 5,83 ppm (D de D, $J_1$ = 9 Hz, $J_2$ = 4 Hz, $H_7$) - 1H à 5,12 ppm (D, J = 4 Hz, $H_6$) - 1H à 4,95 ppm et 1H à 4,67 ppm (D, J = 13 Hz, $CH_2OCO$) - 2H à 3,50 ppm (AB, $J_{AB}$ = 17 Hz, $CH_2S$) - 2H à 3,20 ppm (M, H pipérdine α N équatoriaux) - 2H à 2,60 ppm (M, H pipéridine α N axiaux) - 2H à 2,30 ppm (M, $CH_2COO$) - 1H à 2,05 ppm (M, H pipéridine

-HC) - 4H entre 1,80 et 1,20 ppm (M, H pipéridine β et γ N) - 6H à 1,44 ppm (2S, $(CH_3)_2C$).

RMN n° 23

1H à 9,45 ppm (D, J = 9 Hz, NHCO) - 3H à 8,00 ppm (S.e., $CH_2N^+H_3$) - 1H à 6,73 ppm (S, H thiazole) - 1H à 5,84 ppm (D de D, $J_1$ = 9 Hz, $J_2$ = 4 Hz, $H_7$) - 1H à 5,16 ppm (D, J = 4 Hz, $H_6$) - 1H à 5,00 ppm et 1H à 4,66 ppm (D, J = 13 Hz, $CH_2OCO$) - 1H à 4,16 ppm (M, H pipéridine α N équatorial) - 2H à 3,80 ppm (M, $COCH_2N$) - 3H à 3,55 ppm (M, $CH_2S$ et H pipéridine α N équatorial) - 1H à 3,05 ppm (M, H pipéridine α N axial) - 1H à 2,80 ppm (M, H pipéridine α N axial) - 1H à 2,67 ppm (M, H pipéridine γ N) - 2H à 1,85 ppm et 2H à 1,50 ppm (M, H

pipéridine β N) - 6H à 1,44 ppm (2S, $(CH_3)_2C$).

RMN n° 24

1H à 9,40 ppm (D, J = 9 Hz, CONH) - 2H à 7,85 ppm (D, J = 8 Hz, H aromatiques ortho $CO_2$) - 3H à 7,70 ppm (S.e., $CH_2N^+H_3$) - 2H à 7,50 ppm (D, J = 8 Hz, H aromatiques méta $CO_2$) - 1H à 6,70 ppm (S, H thiazole) - 1H à 5,80 ppm (D de D, $J_1$ = 9 Hz, $J_2$ = 4 Hz, $H_7$) - 1H à 5,25 ppm (D, J = 13 Hz, $CH_2OCO$) - 1H à 5,20 ppm (D, J = 4 Hz, $H_6$) - 1H à 4,91 ppm (D, J = 13 Hz, $CH_2OCO$) - 2H à 3,84 ppm (S, $ArCH_2S$) - 2H à 3,68 ppm (AB, $J_{AB}$ = 17 Hz, $CH_2S$) - 2H à 2,95 ppm (M, $CH_2$-$N^+H_3$) - 2H à 2,51 ppm (M, $CH_2$-$CH_2N^+H_3$) - 6H à 1,38 ppm (2S, $(CH_3)_2C$).

RMN n° 25

1H à 9,55 ppm (D, J = 9 Hz, NHCO) - 3H à 7,80 ppm (S.e., $CH_2N^+H_3$) - 1H à 6,70 ppm (S, H thiazole) - 1H à 5,76 ppm (D de D, $J_1$ = 9 Hz, $J_2$ = 4 Hz, $H_7$) - 1H à 5,09 ppm (D, J = 4 Hz, $H_6$) - 1H à 4,95 ppm et 1H à 4,55 ppm (D, J = 13 Hz, $CH_2OCO$) - 3H à 3,80 ppm (S, $CH_3ON$) - 2H à 3,50 ppm (AB, $J_{AB}$ = 17 Hz, $CH_2S$) - 2H à 2,60 ppm (M, $CH_2N^+H_3$) - 1H à 2,25 ppm (M, H cyclohexane γ $CH_2N$) - 9H entre 0,8 et 2,0 ppm (M, H cyclohexane).

RMN n° 26

1H à 9,56 ppm (D, J = 9 Hz, CONH) - 1H à 8,88 ppm (T, J = 7 Hz, Ar $CH_2$ NH CO) - 3H à 8,00 ppm (S.e., $N^+H_3$) - 2H à 7,90 ppm (D, J = 8 Hz, H aromatiques ortho $CO_2$) - 2H à 7,37 ppm (D, J = 8 Hz, H aromatique méta $CO_2$ - 1H à 6,70 ppm (S, H thiazole) - 1H à 5,77 ppm (D de D, $J_1$ = 9 Hz, $J_2$ = 4 Hz, $H_7$) - 1H à 5,20 ppm (D, J = 13 Hz, $CH_2OCO$) - 1H à 5,13 ppm (D, J = 4 Hz, $H_6$) - 1H à 4,90 ppm (D, J = 13 Hz, $CH_2OCO$) - 2H à 3,41 ppm (D, J = 7 Hz, Ar $CH_2$ NH) - 3H à 3,80 ppm (S, $CH_3ON$) - 4H à 3,55 ppm (M, $CH_2N^+H_3$ et $CH_2S$).

RMN n° 27

1H à 9,40 ppm (D, J = 9 Hz, CONH) - 1H à 8,85 ppm (T, J = 7 Hz, Ar $CH_2$ NH CO) - 3H à 7,95 ppm (S.e., $N^+H_3$) - 2H à 7,91 ppm (D, J = 8 Hz, H aromatiques ortho $CO_2$) - 2H à 7,39 ppm (D, J = 8 Hz, H aromatiques méta $CO_2$) - 2H à 7,30 ppm (S.e., $NH_2$ thiazole) - 1H à 6,76 ppm (S, H thiazole) - 1H à 5,84 ppm (D de D, $J_1$ = 9 Hz, $J_2$ = 4 Hz, $H_7$) - 1H à 5,27 ppm (D, J = 13 Hz, $CH_2OCO$) - 1H à 5,16 ppm (D, J = 4 Hz, $H_6$) - 1H à 4,89 ppm (D, J = 13 Hz, $CH_2OCO$) - 2H à 4,40 ppm (D, J = 7 Hz, $ArCH_2NHCO$) - 4H à 3,60 ppm (M, $CH_2S$ et $CH_2N^+H_3$) - 6H à 1,41 ppm (2S, $(CH_3)_2C$).

RMN n° 28

1H à 9,40 ppm (D, J = 9 Hz, CONH) - 1H à 8,84 ppm (T, J = 7 Hz, Ar $CH_2$ NH) - 3H à 7,95 ppm (S.e., $NH^+{}_3$) - 2H à 7,85 ppm (M, H aromatiques ortho $CO_2$) - 2H à 7,51 ppm (M, H aromatiques méta et para $CO_2$) - 1H à 6,67 ppm (S, H thiazole) - 1H à 5,84 ppm (D de D, $J_1$ = 9 Hz, $J_2$ = 4 Hz, $H_7$) - 1H à 5,25 ppm (D, J = 13 Hz, $CH_2OCO$) - 1H à 5,17 ppm (D, J = 4 Hz, $H_6$) - 1H à 4,93 ppm (D, J = 13 Hz, $CH_2OCO$) - 2H à 4,42 ppm (T, J = 7 Hz, Ar $CH_2$ NH) - 4H à 3,60 ppm (M, $CH_2S$ et $CH_2$ $N^+H_3$) - 6H à 1,41 ppm (2S, $(CH_3)_2C$).

RMN n° 29

1H à 9,56 ppm (D, J = 9 Hz, CONH) - 1H à 8,70 ppm et 1H à 8,42 ppm (S.e., $NH_2^+$ pipéridine) - 2H à 7,30 ppm (S.e., $NH_2$ thiazole) - 1H à 6,65 ppm (S, H thiazole) - 1H à 5,76 ppm (D de D, $J_1$ = 9 Hz, $J_2$ = 4 Hz, $H_7$) - 1H à 5,08 ppm (D, J = 4 Hz, $H_6$) - 1H à 4,95 ppm et 1H à 4,71 ppm (D, J = 13 Hz, $CH_2OCO$) - 3H à 3,79 ppm (S, $CH_3ON$) - 2H à 3,55 ppm (AB, $J_{AB}$ = 17 Hz, $CH_2S$) - 2H à 3,20 ppm (M, H équatoriaux pipéridine α NH) - 2H à 2,90 ppm (M, H axiaux pipéridine α NH) - 1H à 2,66 ppm

(M, OCO CH) - 2H à 1,95 ppm (H écuatoriaux pipéridine βNH) - 2H à 1,66 ppm (H axiaux pipéridine βNH).

RMN n° 30

1H à 9,58 ppm (D, J = 9 Hz, CONH) - 1H à 8,85 ppm (T, J = 7 Hz, Ar $CH_2$ NH CO) - 3H à 8,02 ppm (S.e., $N^+H_3$) - 2H à 7,84 ppm (M, H aromatiques ortho $CO_2$) - 2H à 7,50 ppm (M, H aromatiques méta et para $CO_2$) - 1H à 6,71 ppm (S, H thiazole) - 1H à 5,80 ppm (D de D, $J_1$ = 9 Hz, $J_2$ = 4 Hz, $H_7$) - 1H à 5,24 ppm (D, J = 13 Hz, $CH_2OCO$) - 1H à 5,13 ppm (D, J = 4 Hz, $H_6$) - 1H à 4,92 ppm (D, J = 13 Hz, $CH_2OCO$) - 2H à 4,37 ppm (D, J = 7 Hz, Ar $CH_2$ NH) - 3H à 3,82 ppm (S, $CH_3ON$) - 4H à 3,60 ppm (M, $CH_2S$ et $CH_2N^+H_3$).

RMN n° 31

1H à 9,46 ppm (D, J = 9 Hz, CONH) - 2H à 7,86 ppm (M, H aromatiques ortho $CO_2$) - 3H à 7,80 ppm (S.e., $N^+H_3$) - 1H à 7,55 ppm (D, J = 8 Hz, H aromatiques para $CO_2$) - 1H à 7,50 ppm (T, J = 8 Hz, H aromatique méta $CO_2$) - 2H à 7,30 ppm (S.e., $NH_2$ thiazole) - 1H à 6,70 ppm (S, H thiazole) - 1H à 5,84 ppm (D de D, $J_1$ = 9 Hz, $J_2$ = 4 Hz, $H_7$) - 1H à 5,16 ppm (D, J = 4 Hz, $H_6$) - 1H à 5,25 ppm et 1H à 4,90 ppm (D, J = 13 Hz, $CH_2OCO$) - 2H à 3,62 ppm (AB, $J_{AB}$ = 17 Hz, $CH_2S$) - 3H à 3,02 ppm

(M, Ar-CH-$CH_2$) - 6H à 1,40 ppm (2S, $(CH_3)_2$-C) - 3H à 1,20 ppm (D, J = 7 Hz, $CH_3$-CH-Ar).

RMN n° 32

1H à 9,51 ppm (D, J = 9 Hz, CONH) - 1H à 7,80 ppm (S.e., $N^+H_3$) - 2H à 7,25 ppm (S.e., $NH_2$ thiazole) - 1H à 6,70 ppm (S, H thiazole) - 1H à 5,84 ppm (D de D, $J_1$ = 9 Hz, $J_2$ = 4 Hz, $H_7$) - 1H à 5,17 ppm (D, J = 4 Hz, $H_6$) - 1H à 4,97 ppm et 1H à 4,60 ppm (D, J = 13 Hz, $CH_2OCO$) - 1H à 3,56 ppm et 1H à 3,44 ppm (D, J = 17 Hz, $CH_2S$) - 2H à 2,56 ppm (M, $CH_2N^+H_3$) - 5H à 2,40 ppm

(M, 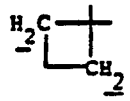 et OCO-CH)

- 6H à 1,90 ppm, 1H à 1,45 ppm, 2H à 1,25 ppm et 2H à 0,95 ppm

(M, 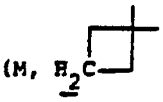

et $CH_2$ et CH cyclohexane).

RMN n° 33

1H à 9,60 ppm (D, J = 9 Hz, CONH) - 2H à 7,90 ppm (M, H aromatiques ortho $CO_2$) - 3H à 7,80 ppm (S.e., $N^+H_3$) - 1H à 7,55 ppm (D, J = 8 Hz, H aromatioue para $CO_2$) - 1H à 7,50 ppm (T, J = 8 Hz, H aromatique méta $CO_2$) - 2H à 7,36 ppm (S.e., $NH_2$ thiazole) - 1H à 6,71 ppm (S, H thiazole) - 1H à 5,79 ppm (D de D, $J_1$ = 9 Hz, $J_2$ = 4 Hz, $H_7$) - 1H à 5,11 ppm (D, J = 4 Hz, $H_6$) - 1H à 5,25 ppm et 1H à 4,95 ppm (D, J = 13 Hz, $CH_2OCO$) - 3H à 3,80 ppm (S, $H_3CON$) - 2H à 3,61 ppm (AB, $J_{AB}$ = 17 Hz, $CH_2S$) - 3H à 3,02 ppm

(M, ArCH-$CH_2$) - 3H à 1,21 ppm (D, J = 7 Hz, $CH_3$- CH-Ar).
      |                                                  |

RMN n° 34

1H à 9,60 ppm (D, J = 9 Hz, NHCO) - 3H à 8,25 ppm (S.e., $N^+H_3$) - 1H à 8,05 ppm (S, H aromatique ortho $CO_2$ et ortho $CH_2$) - 1H à 7,95 ppm (D, J = 8 Hz, H aromatique ortho $CO_2$ et para $CH_2$) - 1H à 7,70 ppm (D, J = 8 Hz, H aromatique para $CO_2$) - 1H à 7,55 ppm (T, J = 6 Hz, H aromatique méta $CO_2$) - 1H à 6,70 ppm (S, H thiazole) - 1H à 5,75 ppm (D de D, $J_1$ = 9 Hz, $J_2$ = 4 Hz, $H_7$) - 1H à 5,20 ppm (D, J = 13 Hz, $CH_2OCO$) - 1H à 5,10 ppm (D, J = 4 Hz, $H_6$) - 1H à 4,95 ppm (D, J = 13 Hz, $CH_2OCO$) - 2H à 4,10 ppm (M, $CH_2$ Ar) - 3H à 3,80 ppm (S, $CH_3ON$) - 2H à 3,70 ppm (AB, $J_{AB}$ = 17 Hz, $CH_2S$).

RMN n° 35

1H à 9,58 ppm (D, J = 9 Hz, NHCO) - 2H à 8,60 ppm (2 S.e., $NH_2^+$ piperidinium) - 2H à 7,40 ppm (S.e., $NH_2$ thiazole) - 1H à 7,11 ppm (S, H thiazole) - 1H à 5,75 ppm (D de D, $J_1$ = 9 Hz, $J_2$ = 4 Hz, $H_7$) - 1H à 5,10 ppm (D, J = 4 Hz, $H_6$) - 1H à 5,00 ppm (2 D, J = 13 Hz, $CH_2OCO$) - 1H à 4,75 ppm (2 D, J = 13 Hz, $CH_2OCO$) - 3H à 3,80 ppm (S, $NOCH_3$) - 2H à 3,50 ppm (AB, $J_{AB}$ = 17 Hz, $CH_2S$) - 5H entre 3,45 et 2,6 ppm (M, $CH_2N$ et

      |
$CO_2H$-CH) - 4H entre 2,00 et 1,45 ppm (M, $(CH_2)_2$ - $CH_2N$).
      |

RMN n° 36

1H à 9,56 ppm (2 D, J = 9 Hz, NHCO) - 3H à 7,80 ppm (S.e., $CH_2N^+H_3^+$) - 2H à 7,40 ppm (S.e., $NH_2$ thiazole) - 1H à 6,72 ppm (S, H thiazole) - 1H à 5,75 ppm (D de D, $J_1$ = 9 Hz, $J_2$ = 4 Hz, $H_7$) - 1H à 5,09 ppm (D, J = 4 Hz, $H_6$) - 1H à 5,00 ppm (2 D, J = 13 Hz, $CH_2OCO$) - 1H à 4,65 ppm (2 D, J = 13 Hz, $CH_2OCO$) - 3H à 3,83 ppm (S, $NOCH_3$) - 1H à 3,54 ppm (2 D, J = 17 Hz, $CH_2S$) - 1H à 3,45 ppm (2 D, J = 17 Hz, $CH_2S$) - 3H à 2,83 ppm (M, $CH_2NH_3^+$ et

|
CH-COOH) - 9H entre 2,0 et 1,1 ppm (M, $CH_2$ cyclohexane et -CH-$CH_2NH_3^+$).
|

RMN n° 37

1H à 9,61 ppm (D, J = 9 Hz, NHCO) - 3H à 7,85 ppm (S.e., $CH_2NH_3^+$) - 2H à 7,40 ppm (S.e., $NH_2$ thiazole) - 1H à 6,71 ppm (S, H thiazole) - 1H à 5,79 ppm (D de D, $J_1$ = 9 Hz, $J_2$ = 4 Hz, $H_7$) - 1H à 5,09 ppm (D, J = 4 Hz, $H_6$) - 1H à 5,02 ppm (2 D, J = 13 Hz, $CH_2OCO$) - 1H à 4,65 ppm (2 D, J = 13 Hz, $CH_2OCO$) - 3H à 3,83 ppm (S, $NOCH_3$) - 3H à 3,82 ppm (AB, $J_{AB}$ = 17 Hz, $CH_2S$) - 1H à 3,00 ppm et 1H à 2,87 ppm (2M, $CH_2NH_3^+$) - 1H à 2,77 ppm

(M, -CH-$CH_3$) - 3H à 1,12 ppm (D, J = 7 Hz, - CH-$CH_3$).
      |                                                  |

RMN n° 38

1H à 9,62 ppm (D, J = 9 Hz, CONH) - 3H à 7,85 ppm (S.e.,

|

$CH-N\underline{H}_3^+$) - 2H à 7,40 ppm (S.e., $N\underline{H}_2$ thiazole) - 1H à 6,70 ppm (S, H thiazole) - 1H à 5,76 ppm (D de D, $J_1$ = 9 Hz, $J_2$ = 4 Hz, $H_7$) - 1H à 5,08 ppm (D, J = 4 Hz, $H_6$) - 1H à 4,95 ppm (D, J = 13 Hz, $C\underline{H}_2OCO$) - 1H à 4,69 ppm (D, J = 13 Hz, $C\underline{H}_2OCO$) - 3H à 3,50 ppm (AB, $J_{AB}$ = 17 Hz, $-C\underline{H}_2S$) - 1H à 3,00 ppm

(M, $C\underline{H}-NH_3^+$) - 1H à 2,50 ppm (M, $C\underline{H}-COOH$) - 1H à 2,10 ppm, 3H à 1,85 ppm et 4H à 1,30 ppm (3M, $C\underline{H}_2$ cyclohexane).

RMN n° 39

1H à 9,40 ppm (D, J = 9 Hz, $N\underline{H}CO$) - 2H à 7,91 ppm (D, J = 8 Hz, H aromatiques ortho O-CO) - 3H à 7,90 ppm (S.e., $CH_2N\underline{H}_3^+$) - 2H à 7,45 ppm (D, J = 8 Hz, H aromatiques méta O-CO) - 2H à 7,40 ppm (S.e., $N\underline{H}_2$ thiazole) - 1H à 6,70 ppm (S, H thiazole) - 1H à 5,81 ppm (D de D, $J_1$ = 9 Hz, $J_2$ = 4 Hz, $H_7$) - 1H à 5,23 ppm (D, J = 13 Hz, $C\underline{H}_2OCO$) - 2H à 3,85 ppm (S, $ArC\underline{H}_2S$) - 3H à 3,81 ppm (S, $NOC\underline{H}_3$) - 2H à 3,70 ppm (AB, J = 17 Hz, $C\underline{H}_2S$) - 2H à 2,95 ppm (M. $C\underline{H}_2NH_3^+$) - 2H à 2,55 ppm (T, J = 7 Hz, $S-C\underline{H}_2CH_2NH_2$).

RMN n° 40

1H à 10,55 ppm (S, $ArN\underline{H}CO$) - 1H à 9,61 ppm (D, J = 9 Hz, $CON\underline{H}$) - 2H à 7,82 ppm (D, J = 8 Hz, H aromatiques ortho-OCO-) - 3H à 7,77 ppm (S.e., $CH_2N\underline{H}_3^+$) - 2H à 7,60 ppm (D, J = 8 Hz, H aromatiques méta-OCO-) - 2H à 7,35 ppm (S.e., $N\underline{H}_2$ thiazole) - 1H à 6,71 ppm (S, H thiazole) - 1H à 5,78 ppm (D de D, $J_1$ = 9 Hz, $J_2$ = 4 Hz, $H_7$) - 2H à 5,15 ppm (M, $H_6$ et $C\underline{H}_2OCO$) - 1H à 4,96 ppm (D, J = 13 Hz, $C\underline{H}_2OCO$) - 3H à 3,80 ppm (S, $NOC\underline{H}_3$) - 2H à 3,60 ppm (AB, $J_{AB}$ = 17 Hz, $C\underline{H}_2S$) - 2H à 3,06 ppm (M, $C\underline{H}_2NH_3^+$) - 2H à 2,70 ppm (T, J = 7 Hz, $C\underline{H}_2-CH_2NH_3^+$).

RMN n° 41

1H à 9,60 ppm (D, J = 9 Hz, $CON\underline{H}$-) - 2H à 8,60 ppm (S.e., $N\underline{H}_2^+$ pipéridinium) - 2H à 7,40 ppm (S.e., $N\underline{H}_2$ thiazole) - 1H à 6,76 ppm (S, H thiazole) - 1H à 5,82 ppm (D de D, $J_1$ = 9 Hz, $J_2$ = 4 Hz, $H_7$) - 1H à 5,15 ppm (D, J = 4 Hz, $H_6$) - 1H à 5,10 ppm (2D, J = 13 Hz, $C\underline{H}_2OCO$) - 1H à 4,76 ppm (2D, J = 13 Hz, $C\underline{H}_2OCO$) - 3H à 3,88 ppm ($N-OC\underline{H}_3$) - 2H à 3,57 ppm (AB, $J_{AB}$ = 17 Hz, $C\underline{H}_2S$) - 1H à 3,45 ppm (M, H $\alpha$ $NH_2^+$) - 1H à 3,26 ppm et 1H à 2,95 ppm (2M, $C\underline{H}_2$, $\alpha NH_2^+$) - 2H à 2,70 ppm (M, $OCO-C\underline{H}_2$) - 6H entre 1,3 et 2 ppm (M, $C\underline{H}_2$ $\beta$ et $\gamma$ $NH_2^+$).

RMN n° 42

1H à 9,70 ppm (D, J = 9 Hz, $CON\underline{H}$) - 1H à 8,60 ppm et 1H à 8,25 ppm (2M, $NH_2^+$ pipéridinium) - 2H à 7,40 ppm (S.e., $N\underline{H}_2$ thiazole) - 1H à 6,76 ppm (S, H thiazole) - 1H à 5,82 ppm (D de D, $J_1$ = 9 Hz, $J_2$ = 4 Hz, $H_7$) - 1H à 5,15 ppm (D, J = 4 Hz, $H_6$) - 1H à 5,02 ppm (D, J = 13 Hz, $C\underline{H}_2OCO$) - 1H à 4,74 ppm (D, J = 13 Hz, $C\underline{H}_2OCO$)- 3H à 3,87 ppm (S, $NOC\underline{H}_3$) - 2H à 3,57 ppm (AB, $J_{AB}$ = 17 Hz, $C\underline{H}_2S$) - 2H à 3,25 ppm (M, H équatorial pipéridium $\alpha$ $NH_2^+$) - 2H à 2,90 ppm (M, H axial pipéridinium $\alpha$ $NH_2^+$) - 2H à 2,35 ppm (D, J = 7 Hz, $OCOC\underline{H}_2$-) - 1H à 2,00 ppm

(M, $OCOCH_2\underline{C}H$) - 2H à 1,80 ppm et 2H à 1,40 ppm (2M, $C\underline{H}_2$ pipéridium $\beta$ $NH_2^+$).

RMN n° 43

1H à 9,70 ppm (D, J = 9 Hz, $CON\underline{H}$) - 2H à 9,00 ppm (S.e., $N\underline{H}_2^+$ -CH($CH_3$)$_2$) - 2H à 8,05 ppm (D, J = 8 Hz, H aromatiques ortho OCO) - 2H à 7,65 ppm (D, J = 8 Hz, H aromatiques méta OCO) - 2H à 7,40 ppm (S.e., $N\underline{H}_2$ thiazole) - 1H à 6,80 ppm (S, H thiazole) - 1H à 5,85 ppm (D de D, $J_1$ = 9 Hz, $J_2$ = 4 Hz, $H_7$) - 1H à 5,30 ppm (D, J = 13 Hz, $C\underline{H}_2OCO$) - 1H à 5,20 ppm (D, J = 4 Hz, $H_6$) - 1H à 5,00 ppm (D, J = 13 Hz, $C\underline{H}_2OCO$) - 2H à 4,27 ppm (M, $ArC\underline{H}_2NH_2^+$ -) - 3H à 3,90 ppm (S, $NOC\underline{H}_3$) - 2H à 3,75 ppm (AB, $J_{AB}$ = 17 Hz, $C\underline{H}_2S$) - 1H à 3,40 ppm (M, $-N^+H_2-C\underline{H}(CH_3)_2$) - 6H à 1,30 ppm (D, J = 7 Hz, $N^+H_2-CH(C\underline{H}_3)_2$).

RMN n° 44

1H à 9,20 ppm (D, J = 9 Hz, CONH) - 3H à 8,05 ppm (S.e., $ArCH_2N{+}H_3$) - 2H à 7,40 ppm (S.e., $NH_2$ thiazole) - 1H à 7,10 ppm (S, H aromatique) - 1H à 6,76 ppm (S, H thiazole) - 1H à 5,84 ppm (D de D, $J_1$ = 9 Hz, $J_2$ = 4 Hz, $H_7$) - 2H à 5,20 ppm (M, $H_6$ et $CH_2OCO$) - 1H à 5,04 ppm (D, J = 13 Hz, $CH_2OCO$) - 2H à 4,10 ppm (M, $ArCH_2N{+}H_3$) - 3H à 3,80 ppm (S, $NOCH_3$) - 2H à 3,60 ppm (AB, $J_{AB}$ = 17 Hz, $CH_2S$) - 3H à 1,40 ppm, 3H à 1,30 ppm et 3H à 1,20 ppm (3S, $CH_3Ar$).

RMN n° 45

1H à 9,70 ppm (D, J = 9 Hz, CONH) - 3H à 8,15 ppm (S.e., $NH_3{+}$) - 2H à 7,40 ppm (S.e., $NH_2$ thiazole) - 2H à 7,40 ppm et 2H à 7,00 ppm (2D, J = 8 Hz, H aromatiques) - 1H à 6,80 ppm (S, H thiazole) - 1H à 5,82 ppm (D de D, $J_1$ = 9 Hz, $J_2$ = 4 Hz, $H_7$) - 1H à 5,15 ppm (D, J = 4 Hz, $H_6$) - 1H à 5,12 ppm (D, J = 13 Hz, $CH_2OCO$) - 2H à 4,86 ppm (S, $CH_2OAr$) - 1H à 4,82 ppm (D, J = 13 Hz, $CH_2OCO$) - 2H à 4,00 ppm (M, $ArCH_2N{+}H_3$) - 3H à 3,85 ppm (S, $NOCH_3$) - 2H à 3,55 ppm (AB, $J_{AB}$ = 17 Hz, $CH_2S$).

RMN n° 46

1H à 9,58 ppm (D, J = 9 Hz, CONH) - 3H à 7,90 ppm (S.e.,

```
 |
-C-N+H3) - 2H à 7,40 ppm (S.e., NH2 thiazole) - 1H à 6,70 ppm (S, H thiazole) - 1H à 5,82 ppm (D de D, J1 =
 |
```

9 Hz, $J_2$ = 4 Hz, $H_7$) - 1H à 5,10 ppm (D, J = 4 Hz, $H_6$) - 1H à 5,05 ppm (D, J = 13 Hz, $CH_2OCO$) - 1H à 4,70 ppm (D, J = 13 Hz, $CH_2OCO$) - 3H à 3,80 ppm (S, N-$OCH_3$) - 2H à 3,55 ppm (AB, $J_{AB}$ = 17 Hz, $CH_2S$) - 2H à 2,95 ppm (M, $CH_2N{+}H_3$) - 6H à 1,16 ppm

```
    CH3
    |
(S, -C-N+-).
    |
    CH3
```

RMN n° 47

1H à 9,00 ppm (D, J = 9 Hz, CONH) - 3H à 8,05 ppm (S.e., $CH_2N{+}H_3$) - 2H à 8,00 ppm (D, J = 8 Hz, H aromatiques ortho OCO) - 2H à 7,50 ppm (D, J = 8 Hz, H aromatiques méta OCO) - 2H à 7,30 ppm (S.e., $NH_2$ thiazole) - 1H à 6,71 ppm (S, H thiazole) - 1H à 5,80 ppm (D de D, $J_1$ = 9 Hz, $J_2$ = 4 Hz, $H_7$) - 1H à 5,25 ppm (D, J = 13 Hz, $CH_2OCO$) - 1H à 5,10 ppm (D, J = 4 Hz, $H_6$) - 1H à 4,95 ppm (D, J = 13 Hz, $CH_2OCO$) - 3H à 3,82 ppm (S, $NOCH_3$) - 2H à 3,66 ppm (AB, $J_{AB}$ = 17 Hz, $CH_2S$) - 2H à 3,55 ppm (S.e., $CH_2N{+}H_3$) - 3H à 3,23 ppm

```
(S, -N-CO).
    |
    CH3
```

RMN n° 48

1H à 9,46 ppm (D, J = 9 Hz, CONH) - 1H à 8,51 ppm (S, H thiazole ester) - 3H à 7,90 ppm (S.e., $CH_2N{+}H_3$) - 2H à 7,45 ppm (S.e., $NH_2$ thiazole) - 1H à 6,69 ppm (S, H thiazole) - 1H à 5,82 ppm (D de D, $J_1$ = 9 Hz, $J_2$ = 4 Hz, $H_7$) - 2H à 5,20 ppm (M, $H_6$ et $CH_2OCO$) - 1H à 4,96 ppm (D, J = 13 Hz, $CH_2OCO$) - 2H à 3,60 ppm (AB, $J_{AB}$ = 17 Hz, $CH_2S$) - 4H à 3,25 ppm (M, $CH_2$-$CH_2NH_3{+}$ - 6H à 1,42 ppm

```
    CH3
    |
(2S, -C-).
    |
    CH3
```

RMN n° 49

1H à 9,60 ppm (D, J = 9 Hz, CONH) - 1H à 8,50 ppm (S, H thiazole ester) - 3H à 7,85 ppm (S.e., $CH_2N{+}H_3$) - 2H à 7,30 ppm (S.e., $NH_2$ thiazole) - 1H à 6,71 ppm (S, H thiazole) - 1H à 5,78 ppm (D de D, $J_1$ = 9 Hz, $J_2$ = 4 Hz, $H_7$) - 1H à 5,19 ppm (D, J = 13 Hz, $CH_2OCO$) - 1H à 5,14 ppm (D, J = 4 Hz, $H_6$) - 1H à 4,92 ppm (D, J = 13 Hz, $CH_2OCO$) - 3H à 3,81 ppm (S, $NOCH_3$) - 2H à 3,61 ppm (AB, $J_{AB}$ = 17 Hz, $CH_2S$) - 4H à 3,26 ppm (M, $CH_2CH_2N{+}H_3$).

RMN n° 50

1H à 9,46 ppm (D, J = 9 Hz, CONH) - 1H à 8,50 ppm (S, H thiazole ester) - 3H à 7,80 ppm (S.e., $CH_2N{+}H_3$) - 2H à 7,40 ppm (S.e., $NH_2$ thiazole) - 1H à 6,68 ppm (S, H thiazole) - 1H à 5,80 ppm (D de D, $J_1$ = 9 Hz, $J_2$ = 4 Hz, $H_7$) - 2H à 5,20 ppm (M, $H_6$ et $CH_2OCO$) - 1H à 4,86 ppm (D, J = 13 Hz, $CH_2OCO$) - 2H à 3,66 ppm (AB, $J_{AB}$ = 17 Hz,

$C\underline{H}_2S$) - 2H à 3,08 ppm (T, J = 7 Hz, $C\underline{H}_2CH_2CH_2N^+H_3$) - 2H à 2,84 ppm (M, $-C\underline{H}_2N^+H_3$) - 2H à 1,96 ppm (M, $CH_2C\underline{H}_2CH_2N^+H_3$) - 6H à 1,36 ppm

$$(2S, \; -\overset{\displaystyle C\underline{H}_3}{\overset{|}{\underset{|}{\underset{\displaystyle C\underline{H}_3}{C}}}}-).$$

RMN n° 51

1H à 9,46 ppm (D, J = 9 Hz, $CON\underline{H}$) - 1H à 8,50 ppm (S, H thiazole ester) - 3H à 7,80 ppm (S.e., $CH_2N^+\underline{H}_3$) - 2H à 7,40 ppm (S.e., $N\underline{H}_2$ thiazole) - 1H à 6,68 ppm (S, H thiazole) - 1H à 5,80 ppm (D de D, $J_1$ = 9 Hz, $J_2$ = 4 Hz, $H_7$) - 2H à 5,20 ppm (M, $H_6$ et $C\underline{H}_2OCO$) - 1H à 4,96 ppm (D, J = 13 Hz, $C\underline{H}_2OCO$) - 3H à 3,81 ppm (S, $NOC\underline{H}_3$) - 2H à 3,66 ppm (AB, $J_{AB}$ = 17 Hz, $C\underline{H}_2S$) - 2H à 3,08 ppm (T, J = 7 Hz, $-C\underline{H}_2CH_2CH_2N^+H_3$) - 2H à 2,84 ppm (M, $C\underline{H}_2N^+H_3$) - 2H à 1,96 ppm (M, $CH_2C\underline{H}_2CH_2N^+H_3$).

RMN n° 52

1H à 9,60 ppm (D, J = 9 Hz, $CON\underline{H}$) - 1H à 8,60 ppm (S, H thiazole ester) - 3H à 8,50 ppm (S.e., $CH_2N^+\underline{H}_3$) - 2H à 7,40 ppm (S.e., $N\underline{H}_2$ thiazole) - 1H à 6,70 ppm (S, E thiazole) - 1H à 5,80 ppm (D de D, $J_1$ = 9 Hz, $J_2$ = 4 Hz, $H_7$) - 2H à 5,15 ppm (M, $H_6$ et $C\underline{H}_2OCO$) - 1H à 4,96 ppm (D, J = 13 Hz, $C\underline{H}_2OCO$) - 2H à 4,45 ppm (S.e., $C\underline{H}_2N^+H_3$) - 3H à 3,78 ppm (S, $NOC\underline{H}_3$) - 2H à 3,57 ppm (AB, $J_{AB}$ = 17 Hz, $C\underline{H}_2S$).

RMN n° 53

1H à 9,70 ppm (D, J = 9 Hz, $CON\underline{H}$) - 1H à 8,70 ppm et 1H à 8,40 ppm (2M, $N^+\underline{H}_2$ pipéridinium) - 2H à 7,80 ppm (S.e., $N\underline{H}_2$ thiazole) - 1H à 6,80 ppm (S, H thiazole) - 1H à 5,85 ppm (D de D, $J_1$ = 9 Hz, $J_2$ = 4 Hz, $H_7$) - 1H à 5,20 ppm (D, J = 4 Hz, $H_6$) - 1H à 5,00 ppm (D, J = 13 Hz, $C\underline{H}_2OCO$) - 1H à 4,72 ppm (D, J = 13 Hz, $C\underline{H}_2OCO$) - 2H à 3,90 ppm (D, J = 7 Hz, $NOC\underline{H}_2$-) - 2H à 3,55 ppm (AB, $J_{AB}$ = 17 Hz, $C\underline{H}_2S$) - 2H à 3,25 ppm (M, H équatoriaux α $N^+H_2$ pipérdinium) - 2H à 2,90 ppm (M, H axiaux $N^+H_2$ pipéridinium) - 2H à 2,35 ppm (D, J = 7 Hz, $OCOC\underline{H}_2$) - 3H à 2,00 ppm et 3H à 1,20 ppm (M, $CH_2$ β $N^+H_2$,

$$OCOCH_2\text{-}\underset{|}{\overset{|}{C}}H \text{ et } C\underline{H} \text{ cyclopropyl}$$

) - 2H à 0,50 ppm et 2H à 0,30 ppm (2M, $C\underline{H}_2$ cyclopropane).

RMN n° 54

1H à 9,55 ppm (D, J = 9 Hz, $CON\underline{H}$) - 3H à 7,70 ppm (S.e., $CH_2N^+\underline{H}_3$) - 2H à 7,25 ppm (S.e., $N\underline{H}_2$ thiazole) - 1H à 6,72 ppm (S, H thiazole) - 1H à 5,75 ppm (D de D, $J_1$ = 9 Hz, $J_2$ = 4 Hz, $H_7$) - 1H à 5,08 ppm (D, J = 4 Hz, $H_6$) - 1H à 4,95 ppm (D, J = 13 Hz, $C\underline{H}_2OCO$) - 1H à 4,66 ppm (D, J = 13 Hz, $C\underline{H}_2OCO$) - 3H à 3,81 ppm (S, $NOC\underline{H}_3$) - 2H à 3,49 ppm (AB, $J_{AB}$ = 17 Hz, $C\underline{H}_2S$) - 2H à 2,70 ppm (M, $C\underline{H}_2N^+\underline{H}_3$) - 2H à 2,30 ppm (T, J = 7 Hz, $C\underline{H}_2$-$(CH_2)_4$ $NH_2$) - 4H à 1,50 ppm (M, $-CH_2\text{-}C\underline{H}_2CH_2\text{-}C\underline{H}_2\text{-}CH_2N^+H_3$) - 2H à 1,20 ppm (M, $-(CH_2)_2\text{-}C\underline{H}_2\text{-}(CH_2)_2N^+H_3$).

RMN n° 55

1H à 9,62 ppm (D, J = 9 Hz, $CON\underline{H}$) - 3H à 7,80 ppm (S.e., $CH_2N^+\underline{H}_3$) - 2H à 7,55 ppm (S.e., $N\underline{H}_2$ thiazole) - 1H à 6,76 ppm (S, H thiazole) - 1H à 5,80 ppm (D de D, $J_1$ = 9 Hz, $J_2$ = 4 Hz, $H_7$) - 1H à 5,15 ppm (D, J = 4 Hz, $H_6$) - 1H à 5,10 ppm (D, J = 13 Hz, $C\underline{H}_2OCO$) - 1H à 4,70 ppm (D, J = 13 Hz, $C\underline{H}_2OCO$) - 3H à 3,85 ppm (S, $N\text{-}OC\underline{H}_3$) - 2H à 3,52 ppm (AB, $J_{AB}$ = 17 Hz, $C\underline{H}_2S$) - 2H à 2,80 ppm (M, $C\underline{H}_2N^+H_3$) - 2H à 2,35 ppm (T, J = 7 Hz, $OCOC\underline{H}_2$) - 4H à 1,51 ppm et 6H à 1,26 ppm (2M, $OCOCH_2(C\underline{H}_2)_5\text{-}CH_2N^+H_3$).

RMN n° 56

1H à 9,56 ppm (D, J = 9 Hz, $CON\underline{H}$) - 2H à 8,30 et 8,55 ppm (2M, $N^+\underline{H}_2$ pipéridinium) - 2H à 7,40 ppm (S.e., $N\underline{H}_2$ thiazole) - 1H à 6,69 ppm (S, H thiazole) - 1H à 5,74 ppm (D de D, $J_1$ = 9 Hz, $J_2$ = 4 Hz, $H_7$) - 1H à 5,10 ppm (D, J = 4 Hz, $H_6$) - 1H à 4,96 ppm (2D, J = 13 Hz, $C\underline{H}_2OCO$) - 1H à 4,66 ppm (D, J = 13 Hz, $C\underline{H}_2OCO$) - 3H à 3,80 ppm (S, $NOC\underline{H}_3$) - 2H à 3,49 ppm (AB, $J_{AB}$ = 17 Hz, $C\underline{H}_2S$) - 2H à 3,15 ppm (M, H équatoriaux à α $N^+H_2$ pipéridinium) - 1H à 2,75 ppm et 1H à 2,55 ppm (2M, H axiaux α $N^+H_2$ pipéridinium) - 2H à 2,36 ppm (T, J = 7 Hz, $OCOC\underline{H}_2$) - 7H entre 1 et 2 ppm (M, $OCOCH_2C\underline{H}_2$- et 5H pipéridinium).

RMN n° 57

1H à 9,45 ppm (D, J = 9 Hz, $CON\underline{H}$) - 1H à 8,55 ppm et 1H à 8,40 ppm (2M, $N^+\underline{H}_2$ pipéridinium) - 2H à 7,40 ppm (S.e., $N\underline{H}_2$ thiazole) - 1H à 6,69 ppm (S, H thiazole) - 1H à 5,84 ppm (D de D, $J_1$ = 9 Hz, $J_2$ = 4 Hz, $H_7$) - 1H à 5,13 ppm (D, J = 4 Hz, $H_6$) - 1H à 4,97 ppm (2D, J = 13 Hz, $C\underline{H}_2OCO$) - 1H à 4,66 ppm (D, J = 13 Hz, $C\underline{H}_2OCO$) - 2H à 3,51 ppm (AB, $J_{AB}$ = 17 Hz, $C\underline{H}_2S$) - 2H à 3,16 ppm (M, H équatoriaux α $N^+H_2$ pipéridinium) - 1H à 2,75 ppm et 1H à 2,55 ppm (2M, H axiaux α $N^+H_2$ pipéridinium) - 2H à 2,36 ppm (T, J = 7 Hz, $OCOC\underline{H}_2$-) - 6H à 1,42 ppm

$CH_3$
|
(2S, -C-) - 7H entre 1 et 2 ppm (M, $OCOCH_2CH_2$, 5H pipéridinium).
|
$CH_3$

RMN n° 58

1H à 9,60 ppm (D, J = 9 Hz, CONH) - 1H à 8,55 ppm et 1H à 8,26 ppm (2 M.e., $N^+H_2$ pipéridinium) - 2H à 7,50 ppm (S.e., $NH_2$ thiazole) - 1H à 6,70 ppm (S, H thiazole) - 1H à 5,75 ppm (D de D, $J_1$ = 9 Hz, $J_2$ = 4 Hz, $H_7$) - 1H à 5,10 ppm (D, J = 4 Hz, $H_6$) - 1H à 4,96 ppm (D, J = 13 Hz, $CH_2OCO$) - 1H à 4,66 ppm (D, J = 13 Hz, $CH_2OCO$) - 3H à 3,82 ppm (S, $NOCH_3$) - 2H à 3,50 ppm (AB, $J_{AB}$ = 17 Hz, $CH_2S$) - 2H à 3,20 ppm (M, H équatoriaux $\alpha$ $N^+H_2$ pipéridinium) - 2H à 2,80 ppm (M, H axiaux $\alpha$ $N^+H_2$ pipéridinium) - 2H à 2,38 ppm (T, J = 7 Hz, $OCOCH_2$) - 2H à 1,70, 3H à 1,48 et 2H à 1,20 ppm (3M, $CH_2$ $\beta$ $N^+H_2$ pipéridinium,

|
-CH- pipéridinium et $OCOCH_2CH_2$).
|

RMN n° 59

1H à 9,45 ppm (D, J = 9 Hz, CONH) - 1H à 8,50 ppm et 1H à 8,20 ppm (2 M.e., $N^+H_2$ pipérdinium) - 2H à 7,50 ppm (S. e., $NH_2$ thiazole) - 1H à 6,71 ppm (S, H thiazole) - 1H à 5,84 ppm (D de D, $J_1$ = 9 Hz, $J_2$ = 4 Hz, $H_7$) - 1H à 5,11 ppm (D, J = 4 Hz, $H_6$) - 1H à 4,96 ppm (D, J = 13 Hz, $CH_2OCO$) - 1H à 4,67 ppm (D, J = 13 Hz, $CH_2OCO$) - 2H à 3,52 ppm (AB, $J_{AB}$ = 17 Hz, $CH_2S$) - 2H à 3,24 ppm (M, H équatoriaux $\alpha$ $N^+H_2$ pipéridinium) - 2H à 2,77 ppm (M, H axiaux $\alpha$ $N^+H_2$ pipéridinium) - 2H à 2,42 ppm (T, J = 7 Hz, $OCOCH_2$-) - 2H à 1,70, 3H à 1,40 et 2H à 1,20 ppm (3M, $CH_2$ $\beta$ $N^+H_2$ pipéridinium,

$CH_3$
| |
-CH pipéridinium et $OCOCH_2CH_2$) - 6H à 1,42 ppm (2S, - C-).
| |
$CH_3$

RMN n° 60

1H à 9,47 ppm (D, J = 9 Hz, CONH) - 2H à 8,80 ppm (M, $N^+H_2$ décahydroquinoléinium) - 2H à 7,60 ppm (S.e., $NH_2$ thiazole) - 1H à 6,71 ppm (S, H thiazole) - 1H à 5,84 ppm (D de D, $J_1$ = 9 Hz, $J_2$ = 4 Hz, $H_7$) - 1H à 5,12 ppm (D, J = 4 Hz, $H_6$) - 1H à 5,06 ppm (2D, J = 13 Hz, $CH_2OCO$) - 1H à 4,72 ppm (D, J = 13 Hz, $CH_2OCO$) - 2H à 3,55 ppm (AB, $J_{AB}$ = 17 Hz, $CH_2S$) - 1H à 3,40 ppm (M, H $\alpha$ $N^+H_2$ jonction de cycle) - 1H à 3,20 ppm (M, H équatorial $\alpha$ $N^+H_2$ décahydroquinoléinium) - 1H à 3,00 ppm (M, H axial $\alpha$ $N^+H_2$ décahydroquinoléinium) - 1H à 2,80 ppm

$CH_3$
| |
(M, OCO-CH) - 6H à 1,42 ppm (2S, - C-) - 11H entre 1 et 2,05 ppm (M, H décahydroquinolénium).
| |
$CH_3$

RMN n° 61

1H à 9,60 ppm (D J = 9 Hz, CONH) - 1H à 8,80 ppm (T, J = 7 Hz, ArCONH) - 2H à 8,00 ppm (D, J = 8 Hz, H aromatiques) - 2H à 7,90 ppm (D, J = 8 Hz, H aromatiques) - 3H à 7,75 ppm (S.e., $CH_2N^+H_3$) - 2H à 7,30 ppm (S.e., $NH_2$ thiazole) - 1H à 6,71 ppm (S, H thiazole) - 1H à 5,79 ppm (D de D, $J_1$ = 9 Hz, $J_2$ = 4 Hz, $H_7$) - 1H à 5,24 ppm (D, J = 13 Hz, $CH_2OCO$) - 1H à 5,12 ppm (D, J = 4 Hz, $H_6$) - 1H à 4,94 ppm (D, J = 13 Hz, $CH_2OCO$) - 3H à 3,80 ppm (S, $NOCH_3$) - 2H à 3,68 ppm (AB, $J_{AB}$ = 17 Hz, $CH_2S$) - 2H à 3,50 ppm (M, $CH_2N^+H_3$) - 2H à 2,95 ppm (M, $CH_2CH_2N^+H_3$).

RMN n° 62

1H à 9,45 ppm (D, J = 9 Hz, CONH) - 1H à 8,80 ppm (T, J = 7 Hz, ArCONH) - 2H à 8,00 ppm (D, J = 8 Hz, H aromatiques) - 2H à 7,90 ppm (D, J = 8 Hz, H aromatiques) - 3H à 7,80 ppm (S.e., $CH_2N^+H_3$) - 2H à 7,40 ppm (S.e., $NH_2$ thiazole) - 1H à 6,70 ppm (S, H thiazole) - 1H à 5,84 ppm (D de D, $J_1$ = 9 Hz, $J_2$ = 4 Hz, $H_7$) - 1H à 5,25 ppm (D, J = 13 Hz, $CH_2OCO$) - 1H à 5,15 ppm (D, J = 4 Hz, $H_6$) - 1H à 4,95 ppm (D, J = 13 Hz, $CH_2OCO$) - 2H à 3,69 ppm (AB, $J_{AB}$ = 17 Hz, $CH_2S$) - 2H à 3,50 ppm (M, $CH_2N^+H_3$) - 2H à 2,98 ppm (M, $CH_2$-$CH_2N^+H_3$) - 6H à 1,41 ppm

$CH_3$
|
(2S, -C-).
|
$CH_3$

**EXEMPLE 64**

Bis trifluoracétate de l'acide [(amino-2 thiazolyl-4)-2 (carboxy-2 propyl-2 oxyimino)-2 acétamido]-7 [(méthyl-2 aminométhyl-4 benzoyl) thiométhyl)-3 céphème-3 carboxylique-4 isomère syn (SR 42943)

(I) $R_1 = R_2 = CH_3$    $R_3 = COOH$    X = S    n = O    B = 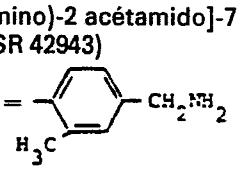

a) Acide méthyl-2 tertiobutoxycarbonylaminométhyl-4 thiobenzoïque

On dissout 2,65 g d'acide méthyl-2 tertiobutoxycarbonylaminométhyl-4 benzoïque et 1,4 ml de triéthylamine dans 60 ml de chlorure de méthylène. On refroidit la solution à 4°C puis on ajoute 1,3 ml de chloroformiate d'isobutyle et agite pendant 15 minutes. En maintenant la température à 4°C, cn fait barboter dans la solution un courant d'hydrogène sulfuré et on ajoute 1,5 ml de triéthylamine. Après 15 minutes, on arrête le passage d'hydrogène sulfuré et laisse sous agitation pendant 15 minutes, toujours à 4° C. On évapore le solvant sous vide et raprend le résidu dans le chlorure de méthylène et le tampon sulfate pH2. On sépare la phase organique et réexrait la phase aqueuse avec du chlorure de méthylène. On réunit les phases organiques, sèche sur sulfate de magnésium et évapore à siccité. Le résidu est repris dans 40 ml d'acétone et la solution utilisée telle quelle pour l'étape suivante.

b) [(Tritylamino-2 thiazolyl-4)-2(tertiobutoxycarbonyl-2 propyl-2 oxyimino)-2 acétamido]-7 (méthyl-2 tertiobutoxycarbonylaminométhyl-4 benzoyl) thiométhyl]-3 céphème-3 carboxylate de tertiobutyle-4 S-oxyde-1β isomère syn

On agite pendant 3 heures à température ambiante, le mélange de 2 g de [(tritylamino-2 thiazolyl-4)-2 (tertiobutoxycarbonyl-2 propyl-2 oxyimino)-2 acétamido]-7 bromométhyl-3 céphème-3 carboxylate de tertiobonate de potassium dans 21 ml de la solution acétonique de thioacide préparée ci-dessus.

On évapore le solvant à siccité et reprend le résidu dans l'eau et le chlorure de méthylène. On sépare la phase organique, sèche sur sulfate de magnésium et évapore à siccité.

On chromatographie sur colonne de silice H. En éluant avec le mélange chlorure de méthylène-méthanol: 100 - 0,7 (vol/vol), on obtient le produit attendu.

c) [(Tritylamino-2 thiazolyl-4)-2(tertiobutoxycarbonyl-2 propyl-2 oxyimino)-2 acétamido]-7 [(méthyl-2 tertiobutoxycarbonylaminométhyl-4 benzoyl) thiométhyl)-3 céphème-3 carboxylate de tertiobutyle-4 isomère syn

On opère la réduction du S-oxyde par le tribromure de phosphore comme indiqué dans l'exemple 1b.

On purifie le produit obtenu par chromatographie sur silice H en éluant avec le mélange chlorure de méthylène-acétate d'éthyle 95 - 5 (vol/vol).

d) SR 42943

A partir du produit précédent, on effectue la déprotection comme indiqué dans l'exemple 1c.

De la même façon, on isole le produit attendu.

Spectre de RMN

1H à 9,40 ppm (D, J = 9 Hz, CO NH) - 3H à 8,20 ppm (S.e., Ar $CH_2N^+H_3$) - 1H à 7,80 ppm (D, J = 8 Hz, H aromatique ortho OCS) - 2H à 7,40 ppm (M, H aromatiques méta OCS) - 1H à 6,70 ppm (S, H thiazole) - 1H à 5,80 ppm (D de D, $J_1$ = 9 Hz, $J_2$ = 4 Hz, $H_7$) - 1H à 5,10 ppm (D, J = 4 Hz, $H_6$) - 1H à 4,20 ppm (D, J = 13 Hz, $CH_2SCO$) - 2H à 4,00 ppm (M, Ar $CH_2$ $N^+H_3$) - 1H à 3,95 ppm (D, J = 13 Hz, $CH_2SCO$) - 1H à 3,75 ppm et 1H à 3,40 ppm (D, J = 17 Hz, $CH_2S$) - 1H à 2,37 ppm (S, Ar $CH_3$) - 6H à 1,42 ppm (2S, $(CH_3)_2$-C).

**EXEMPLES 65 A 84**

Suivant le procédé de l'exemple 64, on prépare les composés selon l'invention, isolés sous forme de trifluoracétate, décrits dans le tableau II, ci-après.

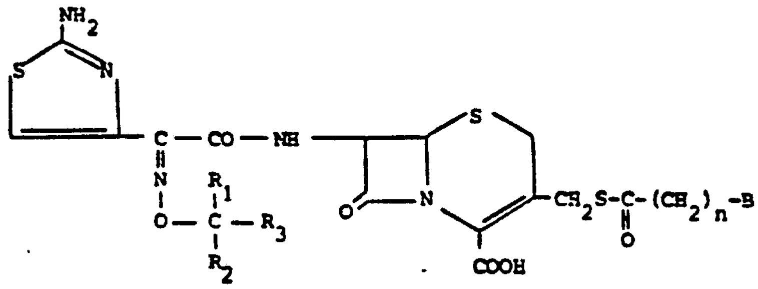

**TABLEAU II**

| N° SR | n | $R_1$ | $R_2$ | $R_3$ | B | Spectre IR Intermédiaire 3 $\gamma$ CO $cm^{-1}$ | N° Spectre RMN |
|---|---|---|---|---|---|---|---|
| 42 887 | 0 | H | H | H | 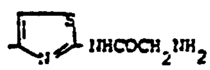 | 1790 - 1710<br>1685<br>($CH_2Cl_2$) | 63 |
| 42 936 | 0 | H | H | H | 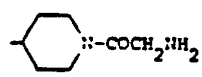 | 1785 - 1700<br>1650 | 64 |
| 42 878 | 0 | $CH_3$ | $CH_3$ | COOH | 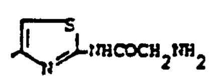 | 1790 - 1720<br>1685 | 65 |
| 42 935 | 0 | $CH_3$ | $CH_3$ | COOH | 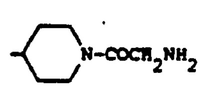 | 1790 - 1715<br>1655<br>($CH_2Cl_2$) | 66 |
| 42 944 | 0 | $CH_3$ | $CH_3$ | COOH | 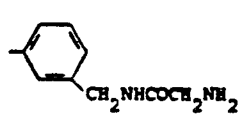 | 1790 - 1720 | 67 |

| | | | | | | | |
|---|---|---|---|---|---|---|---|
| 42 967 | 0 | H | H | H | 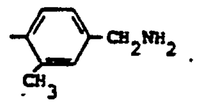 | 785 - 1710 | 68 |
| 42 969 | 0 | H | H | H | 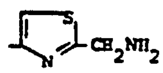 | 1785 - 1710<br>1675 | 69 |
| 42 973 | 0 | H | H | H | 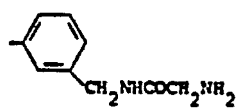 | 1785 - 1715<br>1670 | 70 |
| 42 975 | 0 | H | H | H | 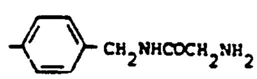 | 1785 - 1715<br>1670 | 71 |
| 43 014 | 0 | $CH_3$ | $CH_3$ | COOH | 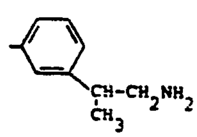 | 1790 - 1715 | 72 |
| 43 015 | 0 | $CH_3$ | $CH_3$ | COOH | 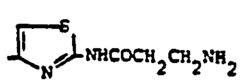 | 1790 - 1715<br>1685 | 73 |
| 43 025 | 0 | H | H | H | 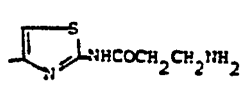 | 1790 - 1710<br>1675 | 74 |

| | | | | | | | |
|---|---|---|---|---|---|---|---|
| 43 031 | 0 | H | H | H | 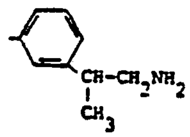 | 1785 - 1710 | 75 |
| 43 027 | 0 | H | H | H | 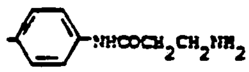 | 1790 - 1685 | 76 |
| 43 191 | 0 | H | H | H | 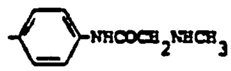 | 1785 - 1710<br>1675 | 77 |
| 43 192 | 0 | $CH_3$ | $CH_3$ | COOH | 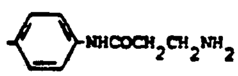 | 1790 - 1700 | 78 |
| 43 193 | 0 | $CH_3$ | $CH_3$ | COOH | 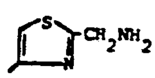 | 1790 - 1720 | 79 |
| 43 234 | 1 | H | H | H | 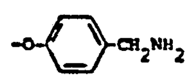 | - | 80 |
| 43 235 | 1 | $CH_3$ | $CH_3$ | COOH | 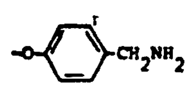 | - | 81 |
| 43 525 | 0 | H | H | 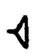 | 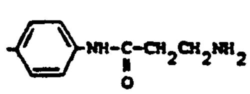 | 1785 - 1680 | 82 |

RMN n° 63

1H à 12,73 ppm (S, thiazole-NH CO) - 4H à 8,30 ppm (S.e., $CH_2N^+H_3$ et NH CO) - 1H à 8,10 ppm (S, H thiazole thioester) - 2H à 7,25 ppm (S.e., $NH_2$ thiazole) - 1H à 6,69 ppm (S, H thiazole aminé) - 1H à 5,70 ppm (D de D, $J_1$ = 9 Hz, $J_2$ = 4 Hz, $H_7$) - 1H à 5,07 ppm (D, J = 4 Hz, $H_6$) - 1H à 4,20 ppm (D, J = 13 Hz, $CH_2SCO$) - 3H à 3,90 ppm (M, $CH_2SCO$ et $NHCOCH_2N^+$) - 3H à 3,80 ppm (S, $CH_3ON$) - 1H à 3,67 ppm et 1H à 3,34 ppm (D, J = 17 Hz, $CH_2S$).

RMN n° 64

1H à 9,55 ppm (D, J = 9 Hz, NH CO) - 3H à 8,00 ppm (S.e., $N^+H_3$) - 2H à 7,30 ppm (S.e., $NH_2$ thiazole) - 1H à 6,71 ppm (S, H thiazole) - 1H à 5,73 ppm (D de D, $J_1$ = 9 Hz, $J_2$ = 4 Hz, $H_7$) - 1H à 5,10 ppm (D, J = 4 Hz, $H_6$) - 1H à 4,30 ppm (M, H pipéridine α N équatorial) - 4H à 3,90 ppm (M, $CH_2SCO$ et $COCH_2N^+H_3$) - 3H à 3,80 ppm (S, $CH_3ON$) - 2H à 3,60 ppm (M, H pipéridine α N équatorial et $CH_2S$) - 1H à 3,25 ppm (D, J = 17 Hz, $CH_2S$) - 1H à 3,05 ppm (M, H pipéridine α N axial) - 1H à 2,90 ppm

(M, S-CO-CH) - 1H à 2,75 ppm (M, H pipéridine α N axial) - 4H entre 1,3 et 2 ppm (M, H pipéridine β N).

RMN n° 65

1H à 12,69 ppm (S, thiazole-NH-CO) - 1H à 9,42 ppm (D, J = 9 Hz, CO NH) - 3H à 8,30 ppm (S.e., $N^+H_3$) - 1H à 8,10 ppm (S, H thiazole thioester) - 2H à 7,30 ppm (S.e., $NH_2$ thiazole) - 1H à 6,70 ppm (S, H thiazole aminé) - 1H à 5,76 ppm (D de D, $J_1$ = 9 Hz, $J_2$ = 4 Hz, $H_7$) - 1H à 5,11 ppm (D, J = 4 Hz, $H_6$) - 1H à 4,25 ppm (D, J = 13 Hz, $CH_2SCO$) - 2H à 3,87 ppm (M, $COCH_2N^+$) - 1H à 3,83 ppm (D, J = 13 Hz, $CH_2SCO$) - 1H à 3,67 ppm et 1H à 3,34 ppm (D, J = 17 Hz, $CH_2S$) - 6H à 1,40 ppm (2S, $(CH_3)_2C$).

RMN n° 66

1H à 9,42 ppm (D, J = 9 Hz NHCO) - 3H à 8,00 ppm (S.e., $N^+H_3$) - 2H à 7 30 ppm (S.e., $NH_2$ thiazole) - 1H à 6,66 ppm (S, H thiazole) - 1H à 5,75 ppm (D de D, $J_1$ = 9 Hz, $J_2$ = 4 Hz, $H_7$) - 1H à 5,07 ppm (D, J = 4 Hz, $H_6$) - 1H à 4,20 ppm (M, H pipéridine α N équatorial) - 1H à 4,00 ppm (D, J = 13 Hz, $CH_2SCO$) - 2H à 3,80 ppm (M, CO-$CH_2N^+$) - 1H à 3,70 ppm (D, J = 13 Hz, $CH_2SCO$) - 2H à 3,60 ppm (M, H pipéridine α N équatorial et $CH_2S$) - 2H à 3,27 ppm (D, J = 17 Hz, $CH_2S$) - 1H à 3,00 ppm (M, H pipéridine α N axial) - 1H à 2,90 ppm

(M, S-CO CH) - 1H à 2,75 ppm (M, H pipéridine α H axial) - 4H entre 1,5 et 2 ppm (M, H pipéridine β N) - 6H à

1,40 ppm (2S, $(CH_3)_2C$).

RMN n° 67

1H à 9,40 ppm (D, J = 9 Hz, NHCO) - 1H à 8,85 ppm (T, J=7 Hz, Ar $CH_2$ NH CO) - 3H à 8,00 ppm (S.e., $N^+H_3$) - 2H à 7,81 ppm (M, H aromatiques ortho COS) - 2H à 7,50 ppm (M, H aromatiques méta, para COS) - 1H à 6,66 ppm (S, H thiazole) - 1H à 5,80 ppm (D de D, $J_1$ = 13 Hz, $J_2$ = 4 Hz, $H_7$) - 1H à 5,10 ppm (D, J = 4 Hz, $H_6$) - 2H à 4,39 ppm (D, J = 7 Hz, Ar $CH_2$ NH CO) - 1H à 4,27 ppm et 1H à 3,94 ppm (D, J = 13 Hz, $CH_2SCO$) - 1H à 3,66 ppm (D, J = 17 Hz, $CH_2S$) - 2H à 3,55 ppm (M, $CH_2NHCO$) - 1H à 3,37 ppm (D, J = 17 Hz, $CH_2S$) - 6H à 1,40 ppm (2S, $(CH_3)_2C$).

RMN n° 68

1H à 9,54 ppm (D, J = 9 Hz, NHCO) - 3H à 8,24 ppm (S. e., Ar $CH_2$ $N^+H_3$) - 1H à 7,77 ppm (D, J = 8 Hz, H aromatique ortho COS) - 2H à 7,39 ppm (M, H aromatiques méta COS) - 2H à 7,27 ppm (S.e., $NH_2$ thiazole) - 1H à 6,71 ppm (S, H thiazole) - 1H à 5,75 ppm (D de D, $J_1$ = 9 Hz, $J_2$ = 4 Hz, $H_7$) - 1H à 5,09 ppm (D, J = 4 Hz, $H_6$) - 1H à 4,21 ppm (D, J = 13 Hz, $CH_2SCO$) - 2H à 4,03 ppm (M, Ar $CH_2N^+$) - 1H à 3,92 ppm (D, J = 13 Hz, $CH_2SCO$) - 3H à 3,79 ppm (S, $CH_3ON$) - 1H à 3,71 ppm et 1H à 3,37 ppm (D, J = 17 Hz, $CH_2S$) - 3H à 2,37 ppm (S, $CH_3$-Ar).

RMN n° 69

1H à 9,53 ppm (D, J = 9 Hz, NHCO) - 1H à 8,57 ppm (S, H thiazole ester) - 3H à 8,55 ppm (S. e., $CH_2N^+H_3$) - 2H à 7,30 ppm (S.e., $NH_2$ thiazole) - 1H à 6,71 ppm (S, H thiazole aminé) - 1H à 5,71 ppm (D de D, $J_1$ = 9 Hz, $J_2$ = 4 Hz, $H_7$) - 1H à 5,09 ppm (D, J = 4 Hz, $H_6$) - 2H à 4,45 ppm (S.e., thiazole-$CH_2N^+H_3$) - 1H à 4,21 ppm et 1H à 3,89 ppm (D, J = 13 Hz, $CH_2SCO$) - 3H à 3,79 ppm (S, $CH_3ON$) - 1H à 3,67 ppm et 1H à 3,39 ppm (D, J = 17 Hz, $CH_2S$).

RMN n° 70

1H à 9,52 ppm (D, J = 9 Hz, NHCO) - 1H à 8,90 ppm (T, J = 7 Hz, Ar $CH_2$ NH CO) - 3H à 8,00 ppm (S.e., $N^+H_3$) - 2H à 7,80 ppm (M, H aromatiques ortho COS) - 2H à 7,53 ppm (M, H aromatiques méta et para COS) - 2H à 7,30 ppm (S.e., $NH_2$ thiazole) - 1H à 6,68 ppm (S, H thiazole) - 1H à 5,69 ppm (D de D, $J_1$ = 9 Hz, $J_2$ = 4 Hz, $H_7$) - 1H à 5,08 ppm (D, J = 4 Hz, $H_6$) - 1H à 4,39 ppm (D, J = 7 Hz, Ar $CH_2$ NH) - 1H à 4,27 ppm et 1H à 3,95 ppm (D, J = 13 Hz, $CH_2SCO$) - 3H à 3,79 ppm (S, $CH_3ON$) - 1H à 3,66 ppm (D, J = 17 Hz, $CH_2S$) - 2H à 3,55 ppm (M, $NHCOCH_2N$) - 1H à 3,34 ppm (D, J = 17 Hz, $CH_2S$).

RMN n° 71

1H à 9,55 ppm (D, J = 9 Hz, NHCO) - 1H à 8,89 ppm (T, J = 7 Hz, Ar $CH_2$ NHCO) - 3H à 8,02 ppm (S.e., $N^+H_3$) - 2H à 7,83 ppm (D, J = 8 Hz, H aromatiques ortho COS) - 2H à 7,40 ppm (D, J = 8 Hz, H aromatiques méta

COS) - 2H à 7,28 ppm (S.e., $N\underline{H}_2$ thiazole) - 1H à 6,68 ppm (S, $\underline{H}$ thiazole) - 1H à 5,71 ppm (D de D, $J_1$ = 9 Hz, $J_2$ = 4 Hz, $\underline{H}_7$) - 1H à 5,11 ppm (D, J = 4 Hz, $\underline{H}_6$) - 2H à 4,39 ppm (D, J = 7 Hz, Ar $C\underline{H}_2$ NH) - 1H à 4,27 ppm et 1H à 3,95 ppm (D, J = 13 Hz, $C\underline{H}_2SCO$) - 3H à 3,79 ppm (S, $C\underline{H}_3ON$) - 1H à 3,78 ppm (D, J = 17 Hz, $C\underline{H}_2$ S) - 2H à 3,63 ppm (M, $COC\underline{H}_2N^+$) - 1H à 3,34 ppm (D, J = 17 Hz, $C\underline{H}_2S$).

RMN n° 72
1H à 9,40 ppm (D, J = 9 Hz, CO $N\underline{H}$) - 5H à 7,80 ppm (M, $CH_2N^+\underline{H}_3$ et $\underline{H}$ aromatiques ortho COS) - 1H à 7,56 ppm (D, J = 8 Hz, $\underline{H}$ aromatique para COS) - 1H à 7,50 ppm (T, J = 8 Hz, $\underline{H}$ aromatique méta COS) - 2H à 7,23 ppm (S.e., $N\underline{H}_2$ thiazole) - 1H à 6,68 ppm (S, H thiazole) - 1H à 5,75 ppm (D de D, $J_1$ = 9 Hz, $J_2$ = 4 Hz, $\underline{H}_7$) - 1H à 5,10 ppm (D, J = 4 Hz, $\underline{H}_6$) - 1H à 4,28 ppm et 1H à 3,94 ppm (D, J = 13 Hz, $C\underline{H}_2SCO$) - 1H à 3,69 ppm et 1H à 3,36 ppm (D, J = 17 Hz, $C\underline{H}_2S$) - 3H à 3,00 ppm

(M, Ar-$C\underline{H}$-$C\underline{H}_2N^+$) - 6H à 1,40 ppm (2S, $(C\underline{H}_3)_2C$) - 2H à 1,22 ppm (D, J = 7 Hz, Ar- CH-$C\underline{H}_3$).

RMN n° 73
1H à 12,70 ppm (S, thiazole $N\underline{H}CO$) - 1H à 9,45 ppm (D, J = 9 Hz, $N\underline{H}CO$) - 1H à 8,05 ppm (S, $\underline{H}$ thiazole ester) - 3H à 7,80 ppm (S.e., $N^+\underline{H}_3$) - 2H à 7,36 ppm (S.e., $N\underline{H}_2$ thiazole) - 1H à 6,69 ppm (S, $\underline{H}$ thiazole aminé) - 1H à 5,70 ppm (D de D, $J_1$ = 9 Hz, $J_2$ = 4 Hz, $\underline{H}_7$) - 1H à 5,07 ppm (D, J = 4 Hz, $\underline{H}_6$) - 1H à 4,24 ppm et 1H à 3,80 ppm (D, J = 13 Hz, $C\underline{H}_2SCO$) - 1H à 3,66 ppm et 1H à 3,40 ppm (D, J = 17 Hz, $C\underline{H}_2S$) - 2H à 3,05 ppm (M, $C\underline{H}_2N^+H_3$) - 2H à 2,80 ppm (M, $C\underline{H}_2CH_2N^+H_3$) - 6H à 1,40 ppm (2S, $(C\underline{H}_3)_2C$).

RMN n° 74
1H à 12,60 ppm (S, thiazole $N\underline{H}CO$) - 1H à 9,55 ppm (D, J = 9 Hz, $N\underline{H}CO$) - 1H à 8,05 ppm (S, $\underline{H}$ thiazole ester) - 3H à 7,83 ppm (S.e., $N\underline{H}_3^+$) - 2H à 7,32 ppm (S.e., $N\underline{H}_2$ thiazole) - 1H à 6,71 ppm (S, $\underline{H}$ thiazole aminé) - 1H à 5,70 ppm (D de D, $J_1$ = 9 Hz, $J_2$ = 4 Hz, $\underline{H}_7$) - 1H à 5,07 ppm (D, J = 4 Hz, $\underline{H}_6$) - 1H à 4,25 ppm et 1H à 3,84 ppm (D, J = 13 Hz, $C\underline{H}_2SCO$) - 3H à 3,82 ppm (S, $C\underline{H}_3ON$) - 1H à 3,65 ppm et 1H à 3,31 ppm (D, J = 17 Hz, $C\underline{H}_2S$) - 2H à 3,06 ppm (M, $C\underline{H}_2N^+H_3$) - 2H à 2,80 ppm (M, CO-$C\underline{H}_2CH_2N^+$).

RMN n° 75
1H à 9,53 ppm D, J = 9 Hz, $N\underline{H}CO$) - 5H à 7,85 ppm (M, $N^+\underline{H}_3$ et $\underline{H}$ aromatiques ortho COS) - 1H à 7,55 ppm (D, J = 8 Hz, $\underline{H}$ aromatique para COS) - 1H à 7,00 ppm (T, J = 8 Hz, $\underline{H}$ aromatique méta COS) - 2H à 7,30 ppm (S.e., $N\underline{H}_2$ thiazole) - 1H à 6,70 ppm (S, $\underline{H}$ thiazole) - 1H à 5,70 ppm (D de D, $J_1$ = 9 Hz, $J_2$ = 4 Hz, $\underline{H}_7$) - 1H à 5,05 ppm (D, J = 4 Hz, $\underline{H}_6$) - 1H à 4,25 ppm et 1H à 3,93 ppm (D, J = 13 Hz, $C\underline{H}_2SCO$) - 3H à 3,80 ppm (S, $C\underline{H}_3ON$) - 1H à 3,67 ppm et 1H à 3,32 ppm (D, J = 17 Hz, $C\underline{H}_2S$) - 3H à 3,01 ppm

(M, Ar$C\underline{H}$-$C\underline{H}_2$) - 3H à 1,21 ppm (D, J = 7 Hz, Ar-CH-$C\underline{H}_3$).

RMN n° 76
1H à 10,55 ppm (S, $ArN\underline{H}CO$) - 1H à 9,53 ppm (D, J = 9 Hz, $CON\underline{H}$) - 2H à 7,84 ppm (D, J = 8 Hz, $\underline{H}$ aromatiques ortho COS) - 3H à 7,80 ppm (S.e., $CH_2N^+\underline{H}_3$) - 2H à 7,71 ppm (D, J = 8 Hz, $\underline{H}$ aromatiques méta COS) - 2H à 7,30 ppm (S.e., $N\underline{H}_2$ thiazole) - 1H à 6,71 ppm (S, $\underline{H}$ thiazole) - 1H à 5,72 ppm (D de D, $J_1$ = 9 Hz, $J_2$ = 4 Hz, $\underline{H}_7$) - 1H à 5,08 ppm (D, J = 4 Hz, $\underline{H}_6$) - 1H à 4,26 ppm et 1H à 3,92 ppm (D, J = 13 Hz, $C\underline{H}_2SCO$) - 3H à 3,79 ppm (S, $C\underline{H}_3ON$) - 1H à 3,70 ppm et 1H à 3,34 ppm (D, J = 17 Hz, $C\underline{H}_2S$) - 2H à 3,05 ppm (M, $C\underline{H}_2N^+H_3$) - 2H à 2,70 ppm (M, $C\underline{H}_2CH_2N^+H_3$).

RMN n° 77
1H à 10,95 ppm ($ArN\underline{H}CO$) - 1H à 9,55 ppm (D, J = 9 Hz, $CON\underline{H}$) - 2H à 8,90 ppm (S.e., $N^+\underline{H}_2$-$CH_3$) - 2H à 7,95 ppm (D, J = 8 Hz, H aromatiques ortho SCO) - 2H à 7,75 ppm (D, J = 8 Hz, H aromatiques méta SCO) - 2H à 7,30 ppm (S.e., $N\underline{H}_2$ thiazol 1H à 6,70 ppm (S, H thiazole) - 1H à 5,70 ppm (D de D, $J_1$ = 9 Hz, $J_2$ = 4 Hz, $H_7$) - 1H à 5,08 ppm (D, J = 4 Hz, $H_6$) - 1H à 4,30 ppm (D, J = 13 Hz, $C\underline{H}_2SCO$) - 3H à 3,90 ppm (M, $C\underline{H}_2N^+$ et $C\underline{H}_2SCO$) - 3H à 3,80 ppm (S, $NOC\underline{H}_3$) - 1H à 3,65 ppm (D, J = 17 Hz, $C\underline{H}_2S$) - 1H à 3,40 ppm (D, J = 17 Hz, $C\underline{H}_2S$) - 3H à 2,60 ppm (S.e., -$N^+H_2$-$C\underline{H}_3$).

RMN n° 78
1H à 10,12 ppm (S, $ArN\underline{H}CO$) - 1H à 9,39 ppm (D, J = 9 Hz, $N\underline{H}CO$) - 1H à 7,90 ppm (D, J = 8 Hz, H aromatiques ortho SCO) - 3H à 7,80 ppm (S.e., $CH_2N^+\underline{H}_3$) - 2H à 7,75 ppm (D, J = 8 Hz, H aromatiques méta SCO) - 2H à 7,50 ppm (S.e., $N\underline{H}_2$ thiazole) - 1H à 6,71 ppm (S, H thiazole) - 1H à 5,76 ppm (D de D, $J_1$ = 9 Hz, $J_2$ = 4 Hz, $H_7$) - 1H à 5,11 ppm (D, J = 4 Hz, $H_6$) - 1H à 4,22 ppm (D, J = 13 Hz, $C\underline{H}_2SCO$) - 1H à 3,93 ppm (D, J = 13 Hz, $C\underline{H}_2SCO$) - 1H à 3,71 ppm (D, J = 17 Hz, $C\underline{H}_2S$) - 1H à 3,45 ppm (D, J = 17 Hz, $C\underline{H}_2S$) - 2H à 3,07 ppm (M, $C\underline{H}_2N^+H_3$) - 2H à 2,73 ppm (M, $C\underline{H}_2CH_2N^+H_3$) - 6H à 1,41 ppm

$CH_3$
|
(S, -C-).
|
$CH_3$

RMN n° 79
1H à 9,45 ppm (D, J = 9 Hz, CONH) - 1H à 8,58 ppm (S, H thiazole ester) - 3H à 8,53 ppm (S.e., $CH_2N^+H_3$) - 2H à 7,50 ppm (S.e., $NH_2$ thiazole) - 1H à 6,70 ppm (S, H thiazole) - 1H à 5,80 ppm (D de D, $J_1$ = 9 Hz, $J_2$ = 4 Hz, $H_7$) - 1H à 5,20 ppm (D, J = 4 Hz, $H_6$) - 2H à 4,50 ppm (S.e., $CH_2N^+H_3$) - 1H à 4,20 ppm (D, J = 13 Hz, $CH_2SCO$) - 1H à 3,90 ppm (D, J = 13 Hz, $CH_2SCO$) - 1H à 3,71 ppm (D, J = 17 Hz, $CH_2S$) - 1H à 3,38 ppm (D, J = 17 Hz, $CH_2S$) - 6H à 1,42 ppm

$CH_3$
|
(S, -C-).
|
$CH_3$

RMN n° 80
1H à 9,70 ppm (D, J = 9 Hz, CONH) - 3H à 8,15 ppm (S.e., $CH_2N^+H_3$) - 2H à 7,45 ppm (S.e., $NH_2$ thiazole) - 2H à 7,40 ppm (D, J = 8 Hz, H aromatiques méta O) - 2H à 7,05 ppm (D, J = 8 Hz, H aromatiques ortho O) - 1H à 6,76 ppm (S, H thiazole) - 1H à 5,77 ppm (D de D, $J_1$ = 9 Hz, $J_2$ = 4 Hz, $H_7$) - 1H à 5,12 ppm (D, J = 4 Hz, $H_6$) - 2H à 5,00 ppm (S, $CH_2O$-Ar) - 1H à 4,20 ppm (D, J = 13 Hz, $CH_2SCO$) - 2H à 4,00 ppm (M, $CH_2N^+H_3$) - 3H à 3,85 ppm (S, $NOCH_3$) - 1H à 3,76 ppm (D, J = 13 Hz, $CH_2SCO$) - 1H à 3,70 ppm (D, J = 17 Hz, $CH_2S$) - 1H à 3,30 ppm (D, J = 17 Hz, $CH_2S$).

RMN n° 81
1H à 9,42 ppm (D, J = 9 Hz, CONH) - 3H à 8,07 ppm (S.e., $CH_2N^+H_3$) - 2H à 7,41 ppm (D, J = 8 Hz, H aromatiques méta O) - 2H à 7,40 ppm (S.e., $NH_2$ thiazole) - 2H à 7,00 ppm (D, J = 8 Hz, H aromatiques ortho O) - 1H à 6,66 ppm (S, H thiazole) - 1H à 5,80 ppm (D de D, $J_1$ = 9 Hz, $J_2$ = 4 Hz, $H_7$) - 1H à 5,09 ppm (D, J = 4 Hz, $H_6$) - 2H à 4,96 ppm (S, $CH_2OAr$) - 1H à 4,10 ppm (D, J = 13 Hz, $CH_2SCO$) - 2H à 3,80 ppm (M, $ArCH_2N^+H_3$) - 1H à 3,75 ppm (D, J = 13 Hz, $CH_2SCO$) - 1H à 3,60 ppm (D, J = 17 Hz, $CH_2S$) - 1H à 3,30 ppm (D, J = 17 Hz, $CH_2S$) - 6H à 1,41 ppm

$CH_3$
|
(2S, -C-).
|
$CH_3$

RMN n° 82
1H à 10,56 ppm (S, ArNHCO) - 1H à 9,55 ppm (D, J = 9 Hz, CONH) - 2H à 7,90 ppm (D, J = 8 Hz, H aromatiques ortho SCO) - 3H à 7,75 ppm (S.e., $CH_2N^+H_3$) - 2H à 7,70 ppm (D, J = 8 Hz, H aromatiques méta SCO) - 2H à 7,45 ppm (S.e., $NH_2$ thiazole) - 1H à 6,66 ppm (S, H thiazole) - 1H à 5,74 ppm (D de D, $J_1$ = 9 Hz, $J_2$ = 4 Hz, $H_7$) - 1H à 5,11 ppm (D, J = 4 Hz, $H_6$) - 1H à 4,25 ppm (D, J = 13 Hz, $CH_2SCO$) - 1H à 3,92 ppm (D, J = 13 Hz, $CH_2SCO$) - 2H à 3,84 ppm (D, J = 7 Hz, N-$OCH_2$-) - 1H à 3,66 ppm (D, J = 17 Hz, $CH_2S$) - 1H à 3,34 ppm (D, J = 17 Hz, $CH_2S$) - 2H à 3,05 ppm (M, $CH_2N^+H_3$) - 2H à 2,68 ppm (T, J=7 Hz, $CH_2CH_2N^+H_3$) - 1H à 1,05 ppm

|
(M, N-$OCH_2CH$) - 2H à 0,46 ppm et 2H à 0,23 ppm (2M, $CH_2$ cyclopropane).
|

## EXEMPLE 85

Bis trifluoracétate de l'acide [(amino-2 thiazolyl-4)-2 (carboxy-2 propyl-2 oxyimino)-2 acétamido]-7 [(pipéridyl-4) carbonylthiométhyl]-3 céphème-3 carboxylique-4 isomère syn (SR 42318).

(I) $R_1 = R_2 = H$ $R_3$ = COOH X = S n = O B = 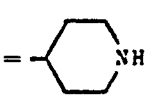

a) Acide tertiobutoxycarbonyl-1 pipéridyl-4 thiocarboxylique
On le prépare à partir de 9,16 g d'acide tertiobutoxycarbonyl-1 pipéridyl-4 carboxylique, selon la technique de l'exemple 64a. Le produit brut huileux est utilisé tel quel.

b) Acide amino-7 [(tertiobutoxycarbonyl-1 pipéridyl-4) carbonylthiométhyl]-3 céphème-3 carboxylique-4

On dissout 9 g d'acide amino-7 céphalosporanique et 2,8 g de bicarbonate de sodium dans 100 ml de tampon phosphate pH 6,65. On chauffe la solution à 50°C sous atmosphère d'azote, puis on ajoute la solution comprenant le thioacide préparé ci-dessus et 3,36 g de bicarbonate de sodium dans 70 ml de tampon phosphate pH 6,65.

Au cours de la réaction, il se forme un précipité et le pH tend à remonter. On le maintient à 6,35 par addition d'une solution à 10 % d'acide phosphorique. Après 4 heures le milieu réactionnel est refroidi à 0°C et on essore les cristaux. On les lave avec de l'eau froide, de l'acétone, de l'éther et enfin avec de l'hexane.

Poids : 7,5 g.

Spectre IR : ν CO β-lactame 1810 $cm^{-1}$.

c) Acide [(tritylamino-2 thiazolyl-4)-2 (tertiobutoxycarbonyl-2 propyl-2 oxyimino)-2 acétamido]-7 (tertiobutoxycarbonyl-1 pipéridyl-4) carbonylthiométhyl)-3 céphème-3 carboxylique-4 isomère syn.

On met en suspension 11,4 g de l'acide obtenu ci-dessus dans 250 ml de dichlorométhane anhydre et on refroidit à +5°C. On ajoute, goutte à goutte, 3,47 ml de triéthylamine et 9,5 ml de diméthylaniline en solution dans 20 ml de dichlorométhane. On ajoute ensuite, goutte à goutte, une solution de 7,94 ml de chlorotriméthylsilane dans 10 ml de dichlorométhane. On laisse remonter la température à 20°C environ et laisse pendant 2 heures à cette température.

On refroidit à nouveau à +5°C, puis on ajoute par petites portions 14 g de chlorure de l'acide (tritylamino-2 thiazolyl-4)-2 (tertiobutoxycarbonyl-2 propyl-2 oxyimino)-2 acétique isomère syn. On agite pendant 1 heure à température ambiante, puis on place le mélange réactionnel au réfrigérateur pendant 15 heures.

On ajoute alors 40 ml d'eau et évapore le dichlorométhane. On reprend le résidu dans 600 ml d'acétate d'éthyle et 300 ml de tampon sulfate pH 2. On décante la phase organique et on la lave à nouveau avec 150 ml de tampon sulfate pH 2, puis avec 100 ml d'eau.

Après séchage sur sulfate de magnésium, on concentre la solution organique à environ 150 ml. On verse la solution, goutte à goutte, dans 1200 ml d'hexane fortement agité. Après 30 minutes d'agitation, on essore le solide. On le lave avec de l'hexane, puis on le sèche sous vide.

On obtient 20,8 g du produit attendu.

Spectre de RMN

1H à 9,32 ppm (D, J = 9 Hz, CO NH) - 1H à 8,80 ppm (S, NH-trityle) - 15H à 7,25 ppm (M, H aromatiques trityle) - 1H à 6,60 ppm (S, H thiazole) - 1H à 5,66 ppm (D de D, $J_1$ = 9 Hz, $J_2$ = 4 Hz, $H_7$) - 1H à 5,06 ppm (D, J = 4 Hz, $H_6$) - 1H à 4,00 ppm et 1H à 3,75 ppm (D, J = 13 Hz, $CH_2SCO$) - 1H à 3,56 ppm et 1H à 3,32 ppm (D, J = 17 Hz, $CH_2S$) - 2H à 3,85 ppm, 3H à 2,75 ppm, 2H à 1,75 ppm et 2H à 1,38 ppm (M, CH et $CH_2$ pipéridine) - 2H à 1,34 ppm (2S, COOtBu, N-Boc, $-C(CH_3)_2$).

d) SR 42318

On déprotège le composé obtenu ci-dessus par l'acide trifluoracétique comme indiqué à l'exemple 1c. De la même façon, on obtient le produit attendu.

Spectre de RMN

1H à 9,40 ppm (D, J = 9 Hz, NH CO) - 1H à 8,60 ppm et 1H à 8,35 ppm (2 S.e., $NH_2^+$ pipéridine) - 2H à 7,30 ppm (S.e., $NH_2$ thiazole) - 1H à 6,66 ppm (S, H thiazole) - 1H à 5,75 ppm (D de D, $J_1$ = 9 Hz, $J_2$ = 4 Hz, $H_7$) - 1H à 5,08 ppm (D, J = 4 Hz, $H_6$) - 1H à 4,05 ppm et 1H à 3,75 ppm (D, J = 13 Hz, $CH_2SCO$) - 1H à 3,61 ppm (D, J = 17 Hz, $CH_2S$) - 3H à 3,25 ppm (M, $CH_2S$ et H pipéridine $\alpha NH^+{}_2$ équatoriaux) - 3H à 2,90 ppm (M, SCO-CH et H pipéridine $\alpha N^+H_2$ axiaux) - 2H à 1,95 ppm (M, H pipéridine $\beta NH_2^+$ équatoriaux) - 2H à 1,70 ppm (M, H, pipéridine $\beta NH_2^+$ axiaux) - 6H à 1,42 ppm (2S, $(CH_3)_2C$).

**EXEMPLES 86 A 100**

a) En opérant comme dans l'exemple 85b, mais en faisant varier le thioacide, on obtient les acides amino-7 céphème-3 carboxylique-4 diversement substitués en 3 figurant dans le tableau III.

**TABLEAU III**

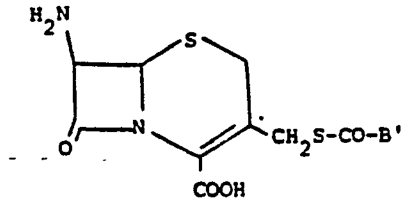

| B' | Spectre IR νCO lactame | ANALYSE ELEMENTAIRE Calculé | Trouvé |
|---|---|---|---|
| 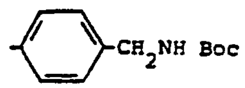 | 1810 $cm^{-1}$ | C : 52,59<br>H : 5,25<br>N : 8,76 | 52,27<br>5,22<br>8,40 |
| 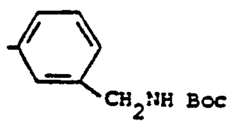 | 1810 $cm^{-1}$ | C : 52,59<br>H : 5,25<br>N : 8,76 | 52,14<br>5,13<br>8,72 |
| 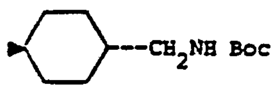 | 1805 $cm^{-1}$ | C : 51,94<br>H : 6,43<br>N : 8,65 | 51,75<br>6,39<br>8,38 |
| 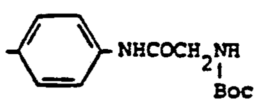 | 1815 $cm^{-1}$ | C : 50,56<br>H : 5,01<br>N : 10,72 | 50,32<br>5,13<br>10,59 |

b) A partir des différents produits ainsi préparés, en opérant selon le procédé décrit dans l'exemple 85c, et d et en faisant varier le chlorure d'acide utilisé, on obtient les différents produits (I) X = S figurant dans le tableau IV ci-après.

**TABLEAU IV**

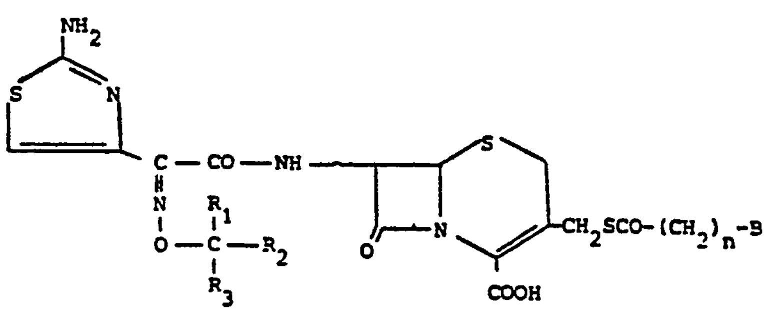

| N° SR | n | $R_1$ | $R_2$ | $R_3$ | B | Spectre IR Composé 7 νCO $cm^{-1}$ | N° RMN |
|---|---|---|---|---|---|---|---|
| 42 316 | 0 | H | H | H | 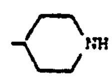 | 1790 - 1730<br>1690 | 83 |

| | | | | | | | |
|---|---|---|---|---|---|---|---|
| 42 317 | 0 | H | H | H | 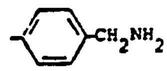 | 1790 - 1720 | 84 |
| 42 319 | 0 | $CH_3$ | $CH_3$ | COOH | 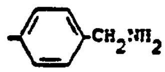 | 1795 - 1720 | 85 |
| 42 802 | 0 | $CH_3$ | $CH_3$ | COOH | 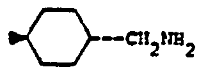 | 1790 - 1720<br>1690 | 86 |
| 42 889 | 0 | H | H | H | 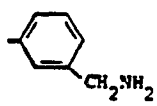 | 1790 - 1710 | 87 |
| 42 882 | 0 | $CH_3$ | $CH_3$ | COOH | 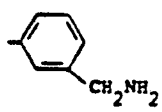 | 1790 - 1715 | 88 |
| 43 017 | 0 | $(CH_2)_3$ | | COOH | 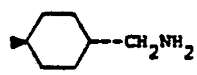 | 1790 - 1715<br>1690 | 89 |
| 43 019 | 0 | H | $CH_3$ | COOH | 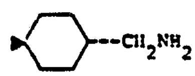 | 1790 - 1720<br>1690 | 90 |
| 43 020 | 0 | H | H | COOH | 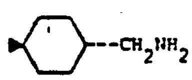 | 1790 - 1715<br>1690 | 91 |
| 43 021 | 0 | H | H | 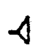 | 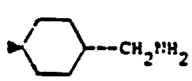 | 1790 - 1685 | 92 |

| | | | | | | | |
|---|---|---|---|---|---|---|---|
| 43 023 | 0 | H | H | H | 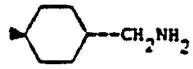 | 1790 - 1690 | 93 |
| 43 520 | 0 | H | H | H | 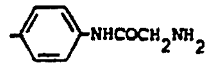 | 1785 - 1690 (Chlorure de méthylène) | 94 |
| 43 522 | 0 | H | H | 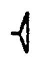 | 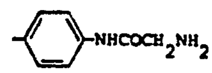 | 1785 - 1690 (Chlorure de) méthylène) | 95 |
| 43 524 | 0 | $CH_3$ | $CH_3$ | COOH | 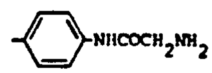 | 1785 - 1715 (Chlorure de méthylène) | 96 |
| 43 697 | 0 | H | H | COOH | 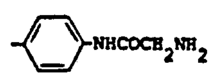 | 1785 - 1715 | 97 |

RMN n° 83
1H à 9,50 ppm (D, J = 9 Hz, CO NH) - 1H à 8,70 ppm et 1H à 8,45 ppm (2 S.e., $NH_2^+$, pipéridine) - 2H à 7,30 ppm (S.e., $NH_2$ thiazole) - 1H à 6,71 ppm (S, H thiazole) - 1H à 5,76 ppm (D de D, $J_1$ = 9 Hz, $J_2$ = 4 Hz, $H_7$) - 1H à 5,07 ppm (D, J = 4 Hz, $H_6$) - 1H à 4,05 ppm (D, J = 13 Hz, $CH_2SCO$) - 4H à 3,78 ppm (M, $CH_3ON$ et $CH_2SCO$) - 1H à 3,58 ppm (D, J = 17 Hz, $CH_2S$) - 3H à 3,27 ppm (M, $CH_2S$ et H équatoriaux pipéridine α $N^+H_2$) - 3H à 2,95 ppm (M, SCO-CH et H axiaux pipéridine α$N^+H_2$) - 2H à 2,00 ppm et 2H à 1,70 ppm (M, H pipéridine β$NH_2^+$).

RMN n° 84
1H à 9,50 ppm (D, J = 9 Hz, CO NH) - 3H à 8,40 ppm (S.e., $CH_2NH_3^+$) - 2H à 7,95 ppm (D, J = 7 Hz, H aromatiques ortho CO) - 2H à 7,55 ppm (D, J = 7 Hz, H aromatiques méta CO) - 2H à 7,30 ppm (S.e., $NH_2$ thiazole) - 1H à 6,73 ppm (S, H thiazole) - 1H à 5,71 ppm (D de D, $J_1$ = 9 Hz, $J_2$ = 4 Hz, $H_7$) - 1H à 5,08 ppm (D, J = 4 Hz, $H_6$) - 1H à 4,26 ppm (D, J = 13 Hz, $CH_2SCO$) - 2H à 4,12 ppm (M, $CH_2N^+H_3$) - 1H à 3,92 ppm (D, J = 13 Hz, $CH_2SCO$) - 3H à 3,78 ppm (S, $NOCH_3$) - 1H à 3,68 ppm (D, J = 17 Hz, $CH_2S$) - 1H à 3,40 ppm (D, J = 17 Hz, $CH_2S$).

RMN n° 85
1H à 9,45 ppm (D, J = 9 Hz, NHCO) - 3H à 8,25 ppm (S.e., $CH_2NH_3^+$) - 2H à 7,95 ppm (D, J = 7 Hz, H aromatiques ortho CO) - 2H à 7,55 ppm (D, J = 7 Hz, H aromatiques méta CO) - 2H à 7,20 ppm (S.e., $NH_2$ thiazole) - 1H à 6,68 ppm (S, H thiazole) - 1H à 5,79 ppm (D de D, $J_1$ = 9 Hz, $J_2$ = 4 Hz, $H_7$) - 1H à 5,10 ppm (D, J

= 4 Hz, $\underline{H}_6$) - 1H à 4,27 ppm (D, J = 13 Hz, $C\underline{H}_2SCO$) - 2H à 4,10 ppm (M, $C\underline{H}_2N^+H_3$) - 1H à 3,95 ppm (D, J = 13 Hz, $C\underline{H}_2SCO$) - 1H à 3,69 ppm (D, J = 17 Hz, $C\underline{H}_2S$) - 1H à 3,40 ppm (D, J = 17 Hz, $C\underline{H}_2S$) - 6H à 1,40 ppm (2S, $(C\underline{H}_3)_2C$).

RMN n° 86

1H à 9,45 ppm (D, J = 9 Hz, N$\underline{H}$ CO) - 3H à 7,75 ppm (S.e., $CH_2N\underline{H}_3^+$) - 2H à 7,45 ppm (S.e., $N\underline{H}_2$ thiazole) - 1H à 6,70 ppm (S, $\underline{H}$ thiazole) - 1H à 5,80 ppm (D de D, $J_1$ = 9 Hz, $J_2$ = 4 Hz, $\underline{H}_7$) - 1H à 5,20 ppm (D, J = 4 Hz, $\underline{H}_6$) - 1H à 4,00 ppm et 1H à 3,74 ppm (D, J = 13 Hz, $C\underline{H}_2$ SCO) - 1H à 3,60 ppm (D, J = 17 Hz, $C\underline{H}_2S$) - 1H à 3,25 ppm (D, J = 17 Hz, $C\underline{H}_2S$) - 2H à 2,60 ppm (M, $C\underline{H}_2N^+$) - 1H à 2,45 ppm (M, SCOC$\underline{H}$) - 4H à 1,85 ppm, 3H à 1,40 ppm et 2H à 1,00 ppm (M, H cyclohexane) - 6H à 1,40 ppm (2S, $(C\underline{H}_3)_2C$).

RMN n° 87

1H à 9,55 ppm (D, J = 9 Hz, N$\underline{H}$CO) - 3H à 8,35 ppm (S.e., $CH_2$ $N^+\underline{H}_3$) - 1H à 8,05 ppm (S, $\underline{H}$ aromatique ortho COS et ortho $CH_2$ $N^+H_3$) - 1H à 7,90 ppm (D, J = 8 Hz, $\underline{H}$ aromatique ortho COS et para $CH_2N^+H_3$) - 1H à 7,72 ppm (D, J = 8 Hz, $\underline{H}$ aromatique para COS) - 1H à 7,55 ppm (T, J = 8 Hz, $\underline{H}$ aromatique méta COS) - 1H à 6,72 ppm (S, $\underline{H}$ thiazole) - 1H à 5,74 ppm (D de D, $J_1$ = 9 Hz, $J_2$ = 4 Hz, $H_7$) - 1H à 5,08 ppm (D, J = 4 Hz, $\underline{H}_6$) - 1H à 4,30 ppm (D, J = 13 Hz, $C\underline{H}_2SCO$) - 2H à 4,10 ppm (M, Ar $C\underline{H}_2$ $N^+$) - 1H à 3,95 ppm (D, J = 13 Hz, $C\underline{H}_2SCO$) - 3H à 3,80 ppm (S, $C\underline{H}_3ON$) - 1H à 3,70 ppm et 1H à 3,40 ppm (D, J = 17 Hz, $C\underline{H}_2S$).

RMN n° 88

1H à 9,42 ppm (D, J = 9 Hz, CO N$\underline{H}$) - 3H à 8,25 ppm (S.e., $CH_2N^+\underline{H}_3$) - 1H à 8,00 ppm (S, $\underline{H}$ aromatique ortho COS et ortho $CH_2N^+$) - 1H à 7,88 ppm (D, J = 8 Hz, $\underline{H}$ aromatique ortho COS et para $CH_2N^+$) - 1H à 7,75 ppm (D, J = 8 Hz, $\underline{H}$ aromatique para COS) - 1H à 7,55 ppm (T, J = 8 Hz, $\underline{H}$ aromatique méta COS) - 2H à 7,40 ppm (S. e., $N\underline{H}_2$ thiazole) - 1H à 6,67 ppm (S, $\underline{H}$ thiazole) - 1H à 5,81 ppm (D de D, $J_1$ = 9 Hz, $J_2$ = 4 Hz, $\underline{H}_7$) - 1H à 5,12 ppm (D, J = 4 Hz, $\underline{H}_6$) - 1H à 4,30 ppm (D, J = 13 Hz, $C\underline{H}_2SCO$) - 2H à 4,08 ppm (M, Ar $C\underline{H}_2$ $N^+$) - 1H à 3,95 ppm (D, J = 13 Hz, $C\underline{H}_2SCO$) - 1H à 3,71 ppm et 1H à 3,40 ppm (D, J = 17 Hz, $C\underline{H}_2S$) - 6H à 1,43 ppm (2S, $(C\underline{H}_3)_2C$).

RMN n° 89

1H à 9,50 ppm (D, J = 9 Hz, CO N$\underline{H}$) - 3H à 7,75 ppm (S.e., $CH_2N\underline{H}_3^+$) - 2H à 7,30 ppm (S.e., $N\underline{H}_2$ thiazole) - 1H à 6,70 ppm (S, $\underline{H}$ thiazole) - 1H à 5,81 ppm (D de D, $J_1$ = 9 Hz, $J_2$ = 4 Hz, $\underline{H}_7$) - 1H à 5,14 ppm (D, J = 4 Hz, $\underline{H}_6$) - 1H à 4,02 ppm et 1H à 3,74 ppm (D, J = 13 Hz, $C\underline{H}_2SCO$) - 1H à 3,63 ppm et 1H à 3,27 ppm (D, J=17 Hz, $C\underline{H}_2S$) - 2H à 2,58 ppm (M, $C\underline{H}_2N^+$) - 1H à 2,50 ppm

(M, SCO-CH) - 4H à 2,30 ppm (M, 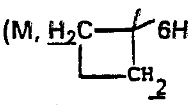 6H à 1,85 ppm, 1H à 1,45 ppm, 2H à 1,25 ppm et 2H à 0,95 ppm

(M, 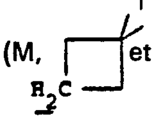 et CH et $CH_2$ cyclohexane).

RMN n° 90

1H à 9,45 ppm (2D, J = 9 Hz, CON$\underline{H}$)° - 3H à 7,75 ppm (S.e., $CH_2N^+\underline{H}_3$) - 2H à 7,25 ppm (S.e., $N\underline{H}_2$ thiazole) - 1H à 6,70 ppm (S, $\underline{H}$ thiazole) - 1H à 5,80 ppm (2Q, $J_1$ = 9 Hz, $J_2$ = 4 Hz, $H_7$)° - 1H à 5,10 ppm (D, J = 4 Hz, $\underline{H}_6$) - 1H à 4,56 ppm (Q, J = 7 Hz,

NOC$\underline{H}$-$CH_3$) - 1H à 4,00 ppm et 1H à 3,74 ppm (D, J = 13 Hz, $C\underline{H}_2SCO$) - 1H à 3,60 ppm et 1H à 3,22 ppm (D, J = 17 Hz, $C\underline{H}_2S$) - 2H à 2,60 ppm (M, $C\underline{H}_2N^+H_3$) - 1H à 2,50 ppm

(M, SCO-C$\underline{H}$) - 4H à 1,80 ppm, 1H à 1,44 ppm, 2H à 1,30 ppm et 2H à 1,00 ppm (M, $\underline{H}$ cyclohexane) - 3H à 1,36 ppm (D, J = 7 Hz, N-O-CH-C$\underline{H}_3$).

° Signaux dédoublés à cause de la diastéréoisomérie de l'oxime.

RMN n° 91

1H à 9,50 ppm (D, J = 9 Hz, CO N$\underline{H}$) - 3H à 7,82 ppm (S.e., $CH_2N^+\underline{H}_3$) - 2H à 7,30 ppm (S.e., $N\underline{H}_2$ thiazole) - 1H

à 6,76 ppm (S. H thiazole) - 1H à 5,74 ppm (D de D, $J_1$ = 9 Hz, $J_2$ = 4 Hz, $H_7$) - 1H à 5,08 ppm (D, J = 4 Hz, $H_6$) - 2H à 4,52 ppm (S, N O $CH_2$-COOH) - 1H à 4,00 ppm et 1H à 3,71 ppm (D, J = 13 Hz, $CH_2SCO$) - 1H à 3,56 ppm et 1H à 3,21 ppm (D, J = 17 Hz, $CH_2S$) - 2H à 2,58 ppm (M, $CH_2$ $N^+H_3$) - 1H à 2,51 ppm

(M, SCOCH) - 4H à 1,90 ppm, 1H à 1,45 ppm, 2H à 1,30 ppm et 2H à 0,95 ppm (M, H cyclohexane).

RMN n° 92

1H à 9,55 ppm (D, J = 9 Hz, CONH) - 3H à 7,80 ppm (S.e., $CH_2NH_3^+$) - 2H à 7,23 ppm (S.e., $NH_2$ thiazole) - 1H à 6,68 ppm (S, H thiazole) - 1H à 5,73 ppm (D de D, $J_1$ = 9 Hz, $J_2$ = 4 Hz, $H_7$) - 1H à 5,06 ppm (D, J = 4 Hz, $H_6$) - 1H à 4,00 ppm et 1H à 3,70 ppm (D, J = 13 Hz, $CH_2SCO$) - 2H à 3,90 ppm (D, J = 7 Hz, N-O-$CH_2$-) - 1H à 3,60 ppm et 1H à 3,23 ppm (D, J = 17 Hz, $CH_2S$) - 2H à 2,58 ppm (M, $CH_2N^+H_3$) - 1H à 2,51 ppm

(M, SCO-CH) - 4H à 1,85 ppm, 1H à 1,44 ppm, 2H à 1,30 ppm et 2H à 0,95 ppm (M, H cyclohexane) - 1H à 1,08 ppm, 2H à 0,45 ppm et 2H à 0,23 ppm (H cyclopropane).

RMN n° 93

1H à 9,55 ppm (D, J = 9 Hz, CONH) - 3H à 7,80 ppm (S.e., $CH_2N^+H_3$) - 2H à 7,20 ppm (S.e., $NH_2$ thiazole) - 1H à 6,69 ppm (S, H thiazole) - 1H à 5,68 ppm (D de D, $J_1$ = 9 Hz, $J_2$ = 4 Hz, $H_7$) - 1H à 5,05 ppm (D, J = 4 Hz, $H_6$) - 1H à 4,00 ppm et 1H à 3,70 ppm (D, J = 13 Hz, $CH_2SCO$) - 3H à 3,75 ppm (S, $CH_3ON$) - 1H à 3,60 ppm et 1H à 3,25 ppm (D, J = 17 Hz, $CH_2S$) - 2H à 2,57 ppm (M, $CH_2NH_3^+$) - 1H à 2,50 ppm

(M, SCO-CH) - 4H à 1,90 ppm, 1H à 1,47 ppm, 2H à 1,30 ppm et 2H à 0,97 ppm (M, H cyclohexane).

RMN n° 94

1H à 10,80 ppm (S, ArNHCO) - 1H à 9,55 ppm (D, J = 9 Hz, NHCO) - 3H à 8,16 ppm (S.e., $CH_2N^+H_3$) - 2H à 7,92 ppm (D, J = 8 Hz, H aromatiques ortho SCO) - 2H à 7,75 ppm (D, J = 8 Hz, H aromatiques méta SCO) - 2H à 7,35 ppm (S.e., $NH_2$ thiazole) - 1H à 6,67 ppm (S, H thiazole) - 1H à 5,80 ppm (D de D, $J_1$ = 9 Hz, $J_2$ = 4 Hz, $H_7$) - 1H à 5,08 ppm (D, J = 4 Hz, $H_6$) - 1H à 4,26 ppm (D, J = 13 Hz, $CH_2SCO$) - 1H à 3,91 ppm (D, J = 13 Hz, $CH_2SCO$) - 3H à 3,80 ppm (S, $CH_3ON$) - 1H à 3,67 ppm (D, J = 17 Hz, $CH_2S$) - 1H à 3,32 ppm (D, J = 17 Hz, $CH_2S$).

RMN n° 95

1H à 10,84 ppm (S, ArNHCO) - 1H à 9,57 ppm (D, J = 9 Hz, NHCO) - 3H à 8,15 ppm (S.e., $CH_2N^+H_3$) - 2H à 7,95 ppm (D, J = 8 Hz, H aromatiques ortho SCO) - 2H à 7,74 ppm (D, J = 8 Hz, H aromatiques méta SCO) - 2H à 7,55 ppm (S.e., $NH_2$ thiazole) - 1H à 6,66 ppm (S, H thiazole) - 1H à 5,80 ppm (D de D, $J_1$ = 9 Hz, $J_2$ = 4 Hz, $H_7$) - 1H à 5,10 ppm (D, J = 4 Hz, $H_6$) - 1H à 4,25 ppm (D, J = 13 Hz, $CH_2SCO$) - 1H à 3,95 ppm (D, J = 13 Hz, $CH_2SCO$) - 2H à 3,84 ppm (D, J = 7 Hz, N-O-$CH_2$-) - 2H à 3,82 ppm (M, $CH_2N^+H_3$) - 1H à 3,66 ppm (D, J = 17 Hz, $CH_2S$) - 1H à 3,34 ppm (D, J = 17 Hz, $CH_2S$) - 1H à 1,10 ppm

(M, N-O-$CH_2$-CH) - 2H à 0,55 ppm et 2H à 0,23 ppm ($CH_2$ cyclopropane).

RMN n° 96

1H à 10,84 ppm (S, ArNHCO) - 1H à 9,45 ppm (D, J = 9 Hz, CONH) - 3H à 8,15 ppm (S.e., $CH_2N^+H_3$) - 2H à 7,92 ppm (D, J = 8 Hz, H aromatiques ortho SCO) - 2H à 7,75 ppm (D, J = 8 Hz, H aromatiques méta SCO) - 2H à 7,50 ppm (S.e., $NH_2$ thiazole) - 1H à 6,68 ppm (S, H thiazole) - 1H à 5,79 ppm (D de D, $J_1$ = 9 Hz, $J_2$ = 4 Hz, $H_7$) - 1H à 5,12 ppm (D, J = 4 Hz, $H_6$) - 1H à 4,30 ppm (D, J = 13 Hz, $CH_2SCO$) - 1H à 3,92 ppm (D, J = 13 Hz, $CH_2SCO$) - 2H à 3,79 ppm (M, $CH_2N^+H_3$) - 1H à 3,67 ppm (D, J = 17 Hz, $CH_2S$) - 1H à 3,34 ppm (D, J = 17 Hz, $CH_2S$) - 6H à 1,41 ppm

(2S, -C($CH_3$)($CH_3$)-).

RMN n° 97

1H à 10,82 ppm (S, ArNHCO) - 1H à 9,47 ppm (D, J = 9 Hz, NHCO) - 3H à 8,10 ppm (S.e., $CH_2N^+H_3$) - 2H à 7,95 ppm (D, J = 8 Hz, H aromatiques ortho SCO) - 2H à 7,72 ppm (D, J = 8 Hz, H aromatiques méta SCO) - 2H à 7,20 ppm (S.e., $NH_2$ thiazole) - 1H à 6,74 ppm (S, H thiazole) - 1H à 5,76 ppm (D de D, $J_1$ = 9 Hz, $J_2$ = 4 Hz, $H_7$) - 1H à 5,08 ppm (D, J = 4 Hz, $H_6$) - 2H à 4,55 ppm (S, $CH_2ON$) - 1H à 4,27 ppm (D, J = 13 Hz, $CH_2SCO$) - 1H à 3,92 ppm (D, J = 13 Hz, $CH_2SCO$) - 2H à 3,81 ppm (S.e., $CH_2N^+H_3$) - 1H à 3,71 ppm (D, J = 17 Hz, $CH_2S$) - 1H à 3,40 ppm (D, J = 17 Hz, $CH_2S$).

Les produits selon l'invention ont été étudiés en ce qui concerne leurs propriétés pharmacologiques et plus particulièrement l'action bactériostatique. Celle-ci a été déterminée in vitro par la méthode des dilutions.

L'étude a porté à la fois sur des souches à Gram positif et des souches à Gram négatif.

Les résultats, exprimés en concentrations minimales inhibitrices (CMI - µg/ml), sont rassemblés dans le tableau V.

**TABLEAU V**

| Produits | SOUCHES | | | | | | | |
|---|---|---|---|---|---|---|---|---|
| n° SR | Staphylocoque Smith | Escherichia Coli Col $E_1$ AMP | Escherichia Coli Sol RL 90 | Klebsiella R 30 | Enterobacter R 046 | Proteus morganii 1510 | Providencia 155 | Pseudomonas aeruginosa NCTC 8203 |
| 42317 | 0,25 | - | 1 | 8 | 1 | 8 | 1 | 16 |
| 42319 | 4 | - | 4 | 1 | 16 | 16 | 2 | 2 |
| 42800 | 8 | 0,0625 | 0,5 | 0,25 | 64 | 8 | 0,5 | 2 |
| 42801 | 4 | ⩽0,0312 | 0,5 | 0,25 | 16 | 2 | 1 | 2 |
| 42802 | 4 | 0,0625 | 0,5 | 0,5 | - | 32 | 4 | 2 |
| 42803 | 4 | 0,0625 | 1 | 0,25 | 64 | 16 | 1 | 2 |
| 42804 | 8 | 0,0625 | 1 | 0,5 | 64 | 16 | 1 | 2 |
| 42808 | 2 | 0,25 | 1 | 0,5 | 64 | 16 | 1 | 1 |
| 42809 | 2 | 0,0625 | 1 | 0,5 | 64 | 16 | 1 | 1 |
| 42883 | 0,125 | 0,0625 | 1 | 2 | 16 | 2 | 1 | 8 |
| 42885 | 0,25 | ⩽0,0312 | 0,25 | 2 | 32 | 2 | 0,5 | 2 |
| 42889 | 0,125 | ⩽0,0312 | 1 | 2 | 16 | 8 | 2 | 32 |
| 42893 | 0,125 | 0,125 | 0,125 | 2 | 8 | 2 | 1 | 8 |
| 42895 | 0,5 | ⩽0,0312 | 0,125 | 2 | 8 | 2 | 2 | 8 |
| 42897 | 0,5 | ⩽0,0312 | 0,125 | 1 | 8 | 2 | 1 | 8 |
| 42880 | 8 | 0,0625 | 1 | 2 | 64 | 16 | 1 | 4 |
| 42882 | 2 | 0,0625 | 1 | 0,5 | 64 | 32 | 1 | 8 |
| 42943 | 2 | 0,0625 | 1 | 1 | 64 | 32 | 2 | 8 |
| 42946 | 1 | ⩽0,0312 | 0,25 | 2 | 16 | 2 | 1 | 8 |
| 42965 | 1 | ⩽0,0312 | 0,125 | 0,5 | 4 | 1 | 0,125 | 2 |
| 42967 | 0,125 | 0,0625 | 1 | 16 | 32 | 8 | 2 | 16 |
| 43015 | 4 | 0,0625 | 2 | 1 | - | 16 | 1 | 4 |
| 43016 | 2 | 0,0625 | 0,25 | 0,5 | 32 | 8 | 0,5 | 2 |
| 43019 | 4 | ⩽0,0312 | 1 | 1 | 64 | 32 | 1 | 2 |
| 43027 | 0,125 | ⩽0,0312 | 1 | - | 32 | 8 | 1 | 2 |
| 43029 | 0,5 | 0,0625 | 0,5 | 2 | 16 | 2 | 1 | 16 |
| 43147 | 1 | ⩽0,0312 | 0,25 | 1 | 16 | 2 | 0,5 | 8 |
| 43179 | 0,5 | 0,0625 | 1 | 1 | 8 | 1 | 1 | 16 |
| 43183 | 1 | ⩽0,0312 | 0,125 | 1 | 16 | 1 | 0,5 | 16 |
| 43185 | 1 | ⩽0,0312 | 0,25 | 2 | 16 | 2 | 1 | 4 |
| 43187 | 0,125 | ⩽0,0312 | 0,25 | 2 | 16 | 2 | 1 | 4 |
| 43189 | 0,125 | ⩽0,0312 | 0,125 | 4 | 16 | 2 | 0,5 | 1 |
| 43191 | 0,125 | ⩽0,0312 | 1 | 16 | 32 | 8 | 1 | 4 |
| 43226 | 0,25 | ⩽0,0312 | 0,125 | 1 | 8 | 1 | 0,25 | 2 |
| 43323 | 1 | ⩽0,0312 | 0,5 | 1 | 16 | 2 | 1 | 8 |
| 43336 | 1 | ⩽0,0312 | 0,5 | 2 | 32 | 2 | 0,5 | 16 |
| 43417 | 0,5 | ⩽0,0312 | 0,25 | 1 | 8 | 1 | 0,5 | 4 |
| 43419 | 0,5 | ⩽0,0312 | 0,5 | 2 | 8 | 2 | 1 | 8 |
| 43464 | 1 | ⩽0,0312 | 0,25 | 2 | 16 | 2 | 1 | 16 |
| 43520 | 0,125 | 0,0625 | 0,5 | 8 | 32 | 8 | 1 | 2 |
| 43559 | 0,5 | ⩽0,0312 | 0,125 | 1 | 8 | 1 | 0,5 | 2 |
| 43666 | 0,5 | ⩽0,0312 | 0,5 | 4 | 32 | 4 | 1 | 4 |

Les résultats figurant dans le tableau V montrent que les produits selon l'invention présentent un large spectre d'activité, tant sur les bactéries Gram positif que sur les bactéries Gram négatif.

Par ailleurs, d'après les essais effectués sur les animaux, la toxicité des produits selon l'invention a paru suffisamment faible pour permettre leur utilisation en thérapeutique.

Les produits de l'invention peuvent donc être employés comme antibiotiques en médecine humaine ou vétérinaire. Ils présentent un large spectre et peuvent être utilisés dans toutes les infections bactériennes à germes sensibles.

Les produits peuvent être administrés par voie générale (parentérale, orale) ou par voie topique.

Les compositions pharmaceutiques sont réalisées à partir des composés (I) sous une forme soluble obtenue par salification d'une des fonctions acides de la molécule ou d'une des fonctions amine de la chaîne B.

Les compositions pharmaceutiques contenant à titre d'ingrédient actif l'antibiotique selon l'invention en combinaison avec un véhicule pharmaceutiquement acceptable peuvent être solides ou liquides et se présenter, par exemple, sous forme de préparations injectables, de comprimés, gélules, granulés, pommades, crèmes, gels ou suppositoires. La posologie peut varier dans de larges proportions, en particulier suivant le type et la gravité de l'infection à traiter et suivant le mode d'administration. Le plus souvent, chez l'adulte, par voie injectable, elle est comprise entre 0,250 g et 4 g par jour.

A titre d'exemple de préparation galénique, on peut préparer une solution injectable contenant, pour chaque ampoule :

SR 43189: 1 g

Eau pour préparation injectable: 5 ml

Carbonate de sodium q.s. pour pH = 6,3

**Revendications** pour les états contractants: BE, CH, DE, FR, GB, IT, LI, LU, NL, SE

1. Dérivés des céphalosporines répondant à la formule générale:

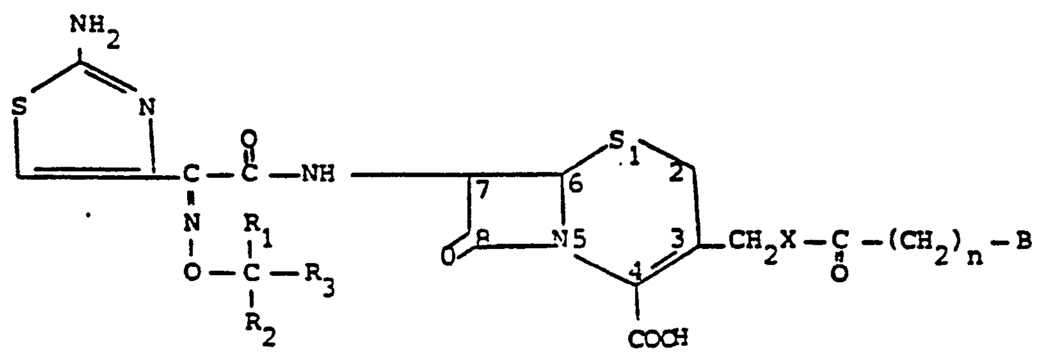

(I)

dans laquelle:

- X désigne un atome d'oxygène ou un atome de soufre.
- n est zéro, 1 ou 2.
- $R_1$, $R_2$ et $R_3$ désignent chacun un atome d'hydrogène, ou encore $R_1$ et $R_2$ désignent un atome d'hydrogène ou un groupe méthyle et $R_3$ désigne un groupe carboxyle ou cyclopropyle, ou encore
- $R_1$ et $R_2$ pris ensemble avec l'atome de carbone auquel ils sont liés forment un noyau cyclobutyle ou cyclopentyle et $R_3$ désigne un groupe carboxyle.
- B est le reste d'une amine primaire ou secondaire choisie par les groupements suivants:

- Z-$NH_2$ où Z est un groupe alkylène à chaîne droite ou ramifiée ayant de 2 à 7 atomes de carbone, éventuellement interrompue par un atome de soufre, ou encore Z représente un groupe 1,3 cyclohexylène ou 1,4 cyclohexylène.

- Z'-Alk-NH-R où Z' représente un groupe 1,2-phénylène ou 1,3-phénylène ou 1,4-phénylène éventuellement substitué par 1, 2 ou 3 groupes méthyle ou bien Z' représente un groupe 1,2-cyclohexylène, 1,3-cyclohexylène ou 1,4-cyclohexylène.

Alk représente un groupe alkylène droit ou ramifié ayant de 1 à 3 atomes de carbone, éventuellement interrompu par un atome de soufre.

R représente un atome d'hydrogène ou un groupe alkyle ayant de 1 à 3 atomes de carbone.

- Z"-N-CO-Y-NH-R où Z" représente un groupe 1,3-phénylène ou 1,4-phénylène,
  |
  R'

Y désigne un groupe alkylène $(CH_2)_m$ dans lequel m = 1, 2 ou 3,

R' est hydrogène ou méthyle et

R est tel que désigné ci-dessus.

- Z''-CO-NH-Y-$NH_2$ ou Z'' et Y sont tels que définis ci-dessus.

- Z''-Y'-NH-CO-Y-$NH_2$ où Z'' et Y sont tels que définis ci-dessus et Y' représente un groupe alkylène droit ou ramifié ayant 1 ou 2 atomes de carbone

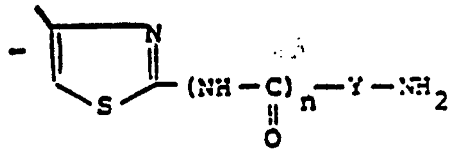

où n = 0 ou 1 et Y est tel que défini précédemment.

- un groupe 2-pipéridyl, 3-pipéridyl ou 4-pipéridyl éventuellement substitué sur l'atome d'azote par un groupe -CO-Y-$NH_2$ ou Y est tel que défini précédemment.

- un groupe 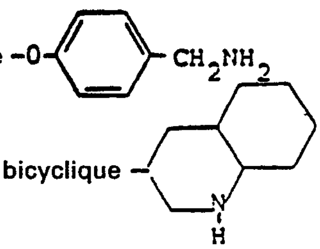

- le groupe bicyclique

ainsi que les sels et les esters pharmaceutiquement acceptables desdits dérivés.

2. Procédé pour l'obtention des dérivés des céphalosporines selon la revendication 1, caractérisé en ce qu'il consiste :

1) à faire réagir sur le composé de formule :

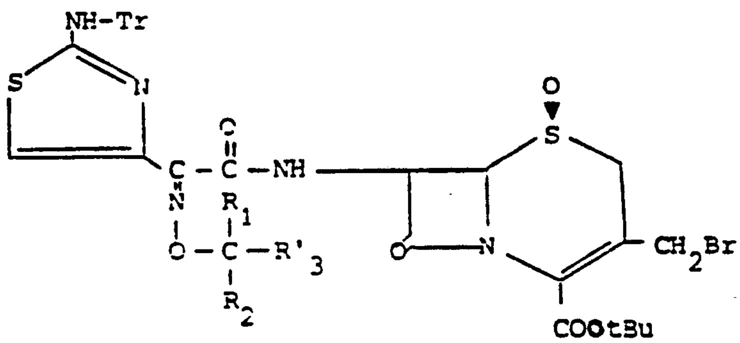

dans laquelle

$R_1$ et $R_2$ sont tels que définis à la revendication 1,

$R'_3$ désigne l'hydrogène, un groupe cyclopropyle ou un groupe ester, facilement labile, et dont la fonction amine a été préalablement protégée, l'acide ou le thioacide de formule B'-$(CH_2)_n$-COXH, dans laquelle X et n sont tels que définis à la révendication 1; B' représente le groupe B tel que défini à la revendication 1 dans lequel la fonction amine est protégée,

2) à soumettre le composé obtenu à l'action du tribromure de phosphore à basse température et au sein d'un solvant, pour éliminer la fonction S-oxyde,

3) à éliminer les groupes protecteurs présents sur les fonctions amines et carboxyliques, par hydrolyse en milieu acide,

4) à isoler le composé de formule (I) ainsi obtenu sous la forme d'un sel du groupement aminé B,

5) à transformer éventuellement ledit sel en un autre sel du groupement aminé B,

6) à isoler, éventuellement, à partir d'un sel du groupement aminé B, par action d'une base, le composé de formule (I) attendu et à le transformer éventuellement, par des procédés connus, en un sel ou un ester, dérivant d'un ou deux groupes carboxyliques présents dans la molécule.

3. Procédé pour l'obtention des dérivés des céphalosporines de formule I selon la revendication 1, dans laquelle X est le soufre, caractérisé en ce qu'il consiste

1) à faire réagir le composé de formule:

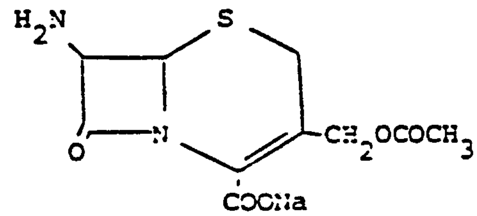

en milieu aqueux tamponné à pH: 6,3 - 6,4, avec le thioacide de formule B'-$(CH_2)_n$-COSNa, dans laquelle n est tel que défini à la revendication 1 et B' représente le groupe B tel que défini à la revendication 1 dans lequel la fonction amine est protégée; pour obtenir le composé de formule:

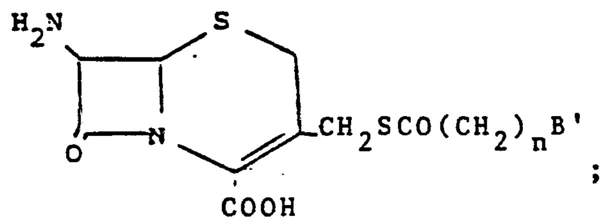

;

2) à acyler le composé ainsi obtenu, dont on a bloqué temporairement la fonction carboxylique, par le chlorure de l'acide de formule:

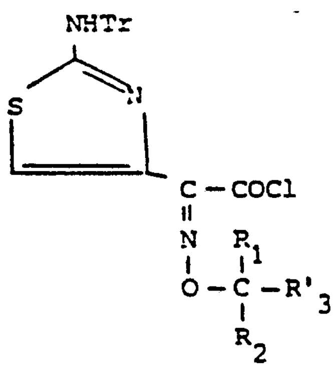

dans laquelle $R_1$ et $R_2$ sont tels que définis à la revendication 1 et $R'_3$ désigne l'hydrogène, un groupe cyclopropyle ou un groupe ester, facilement labile; pour obtenir le composé de formule (I) attendu, protégé;

3) à éliminer les groupes protecteurs présents sur les fonctions amines et carboxyliques, par hydrolyse en milieu acide;

4) à isoler le composé de formule (I) ainsi obtenu sous la forme d'un sel du groupement aminé B;

5) à transformer éventuellement ledit sel en un autre sel du groupement aminé B;

6) à isoler, éventuellement, à partir d'un sel du groupement aminé B, par action d'une base, le composé de formule (I) attendu et à le transformer éventuellement, par des procédés connus, en un sel ou un ester, dérivant d'un ou deux groupes carboxyliques présents dans la molécule.

4. Composition pharmaceutique caractérisée en ce qu'elle contient un dérivé de céphalosporines selon la revendication 1 en association avec un véhicule pharmaceutique acceptable.

5. Composition antibiotique, caractérisée en ce qu'elle contient un dérivé de céphalosporines selon la revendication 1 en association avec un véhicule pharmaceutique acceptable.

**Revendications** pour l'état contractant: AT

1. Procédé pour l'obtention de dérivés des céphalosporines répondant à la formule générale:

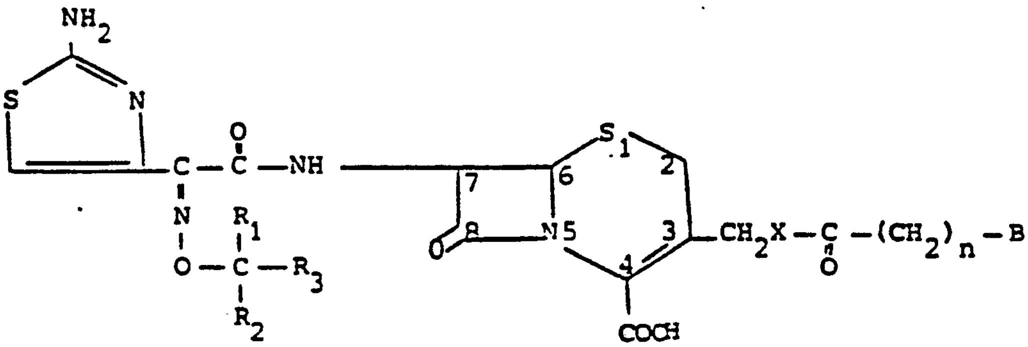 (I)

dans laquelle:

- X désigne un atome d'oxygène ou un atome de soufre.
- n est zéro, 1 ou 2.
- $R_1$, $R_2$ et $R_3$ désignent chacun un atome d'hydrogène, ou encore $R_1$ et $R_2$ désignent un atome d'hydrogène ou un groupe méthyle et $R_3$ désigne un groupe carboxyle ou cyclopropyle, ou encore
- $R_1$ et $R_2$ pris ensemble avec l'atome de carbone auquel ils sont liés forment un noyau cyclobutyle ou cyclopentyle et $R_3$ désigne un groupe carboxyle.
- B est le reste d'une amine primaire ou secondaire choisie par les groupements suivants:

- Z-$NH_2$ ou Z est un groupe alkylène à chaîne droite ou ramifiée ayant de 2 à 7 atomes de carbone, éventuellement interrompue par un atome de soufre, ou encore Z représente un groupe 1,3 cyclohexylène ou 1,4 cyclohexylène.

- Z'-Alk-NH-R ou Z' représente un groupe 1,2-phénylène ou 1,3-phénylène ou 1,4-phénylène éventuellement substitué par 1, 2 ou 3 groupes méthyle ou bien Z' représente un groupe 1,2-cyclohexylène, 1,3-cyclohexylène ou 1,4-cyclohexylène.

Alk représente un groupe alkylène droit ou ramifié ayant de 1 à 3 atomes de carbone, éventuellement interrompu par un atome de soufre.

R représente un atome d'hydrogène ou un groupe alkyle ayant de 1 à 3 atomes de carbone.

Z"-N-CO-Y-NH-R où Z" représente un groupe 1,3-phénylène ou 1,4-phénylène,
 |
 R'

Y désigne un groupe alkylène $(CH_2)_m$ dans lequel m = 1, 2 ou 3,
R' est hydrogène ou méthyle et
R est tel que désigné ci-dessus.

- Z"-CO-NH-Y-$NH_2$ ou Z" et Y sont tels que définis ci-dessus.
- Z"-Y'-NH-CO-Y-$NH_2$ où Z" et Y sont tels que définis ci-dessus et Y' représente un groupe alkylène droit ou ramifié ayant 1 ou 2 atomes de carbone

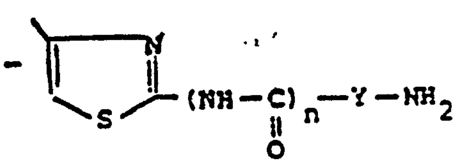

- un groupe 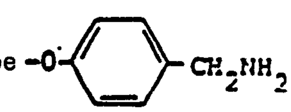

- le groupe bicyclique 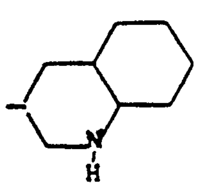

ainsi que de leurs sels et esters pharmaceutiquement acceptables;

caractérisé en ce qu'il consiste:

1) à faire réagir sur le composé de formule:

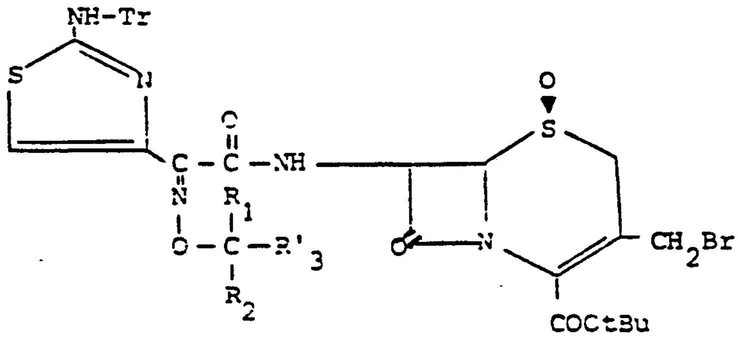

dans laquelle

$R_1$ et $R_2$ sont tels que définis ci-dessus,

$R'_3$ désigne l'hydrogène, un groupe cyclopropyle ou un groupe ester, facilement labile, et dont la fonction amine a été préalablement protégée, l'acide ou le thioacide de formule $B'-(CH_2)_n-COXH$, dans laquelle X et n sont tels que définis ci-dessus; B' représente le groupe B tel que défini ci-dessus dans lequel la fonction amine est protégée,

2) à soumettre le composé obtenu à l'action du tribromure de phosphore à basse température et au sein d'un solvant, pour éliminer la fonction S-oxyde,

3) à éliminer les groupes protecteurs présents sur les fonctions amines et carboxyliques, par hydrolyse en milieu acide,

4) à isoler le composé de formule (I) ainsi obtenu sous la forme d'un sel du groupement aminé B,

5) à transformer éventuellement ledit sel en un autre sel du groupement aminé B,

6) à isoler, éventuellement, à partir d'un sel du groupement aminé B, par action d'une base, le composé de formule (I) attendu et à le transformer éventuellement, par des procédés connus, en un sel ou un ester, dérivant d'un ou deux groupes carboxyliques présents dans la molécule.

2. Procédé pour l'obtention de dérivés des céphalosporines de formule I selon la revendication 1 dans laquelle X est le soufre, caractérisé en ce qu'il consiste

1) à faire réagir le composé de formule:

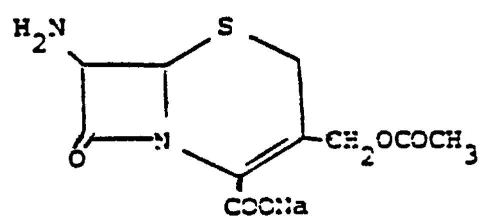

en milieu aqueux tamponné à pH: 6,3 - 6,4, avec le thioacide de formule $B'-(CH_2)_n-COSNa$, dans laquelle n est tel que défini à la revendication 1 et B' représente le groupe B tel que défini à la revendication 1 dans lequel la fonction amine est protégée; pour obtenir le composé de formule:

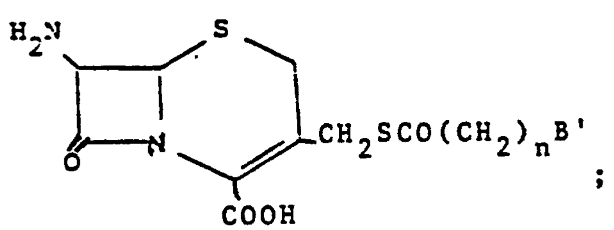

2) à acyler le composé ainsi obtenu, dont on a bloqué temporairement la fonction carboxylique, par le chlorure de l'acide de formule:

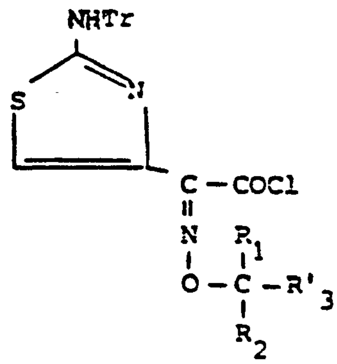

dans laquelle $R_1$ et $R_2$ sont tels que définis à la revendication 1 et $R'_3$ désigne l'hydrogène, un groupe cyclopropyle ou un groupe ester, facilement labile; pour obtenir le composé de formule (1) attendu, protégé,

3) à éliminer les groupes protecteurs présents sur les fonctions amines et carboxyliques, par hydrolyse en milieu acide,

4) à isoler le composé de formule (I) ainsi obtenu sous la forme d'un sel du groupement aminé B,

5) à transformer éventuellement ledit sel en un autre sel du groupement aminé B,

6) à isoler, éventuellement, à partir d'un sel du groupement aminé B, par action d'une base, le composé de formule (I) attendu et à le transformer éventuellement, par des procédés connus, en un sel ou un ester, dérivant d'un ou deux groupes carboxyliques présents dans la molécule.

3. Utilisation des dérivés des céphalosporines de formule (I) selon la revendication 1, pour la préparation de compositions pharmaceutiques.

4. Utilisation selon la revendication 3 pour la préparation de compositions antibiotiques.

**Patentansprüche** für die Vertragsstaaten: BE, CH, DE, FR, GB, IT, LI, LU, NL, SE

1. Cephalosporinderivate der allgemeinen Formel:

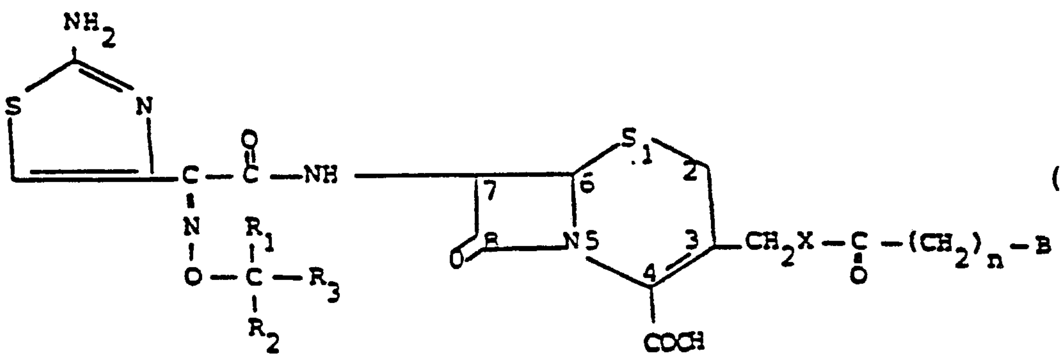

(I)

worin:

- X ein Sauerstoffatom oder ein Schwefelatom bezeichnet;
- n für Null, 1 oder 2 steht;
- $R_1$, $R_2$ und $R_3$ jeweils ein Wasserstoffatom bezeichnen, oder aber $R_1$ und $R_2$ ein Wasserstoffatom oder ein Methylgruppe bezeichnen und $R_3$ für ein Carboxyl- oder Cyclopropylgruppe steht; oder aber
- $R_1$ und $R_2$, zusammen mit dem Kohlenstoffatom, an die sie gebunden sind, einen Cyclobutylkern oder Cyclopentylkern bilden und $R_3$ eine Carboxylgruppe bezeichnet;
- B der Rest eines primären oder sekundären Amins ist, ausgewählt aus den folgenden Gruppen:
  - Z-$NH_2$, worin Z eine Alkylengruppe mit gerader oder verzweigter Kette mit 2 bis 7 Kohlenstoffatomen, gegebenenfalls unterbrochen durch ein Schwefelatom darstellt, oder aber Z für eine 1,3-Cyclohexylen- oder 1,4-Cyclohexylengruppe steht;
  - Z'-Alk-NH-R, worin Z' eine 1,2-Phenylen- oder 1,3-Phenylen- oder 1,4-Phenylengruppe, gegebenfalls substituiert durch 1,2-Cyclohexylen-, 1,3-Cyclohexylen- oder 1,4-Cyclohexylengruppe darstellt,
  Alk eine gerade oder verzweigte Alkylengruppe mit 1 bis 3 Kohlenstoffatomen, gegebenfalls unterbrochen von einem Schwefelatom, darstellt,
  R ein Wasserstoffatom oder eine Alkylgruppe mit 1 bis 3 Kohlenstoffatomen bedeutet;
  - Z"-N-CO-Y-NH-R,
  |
  R'

worin Z" eine 1,3-Phenylen- oder 1,4-Phenylengruppe darstellt, Y eine Alkylengruppe $(CH_2)_m$ bezeichnet, worin m = 1, 2 oder 3, R' für Wasserstoff oder Methyl steht und R die obige Bedeutung hat;
- Z"-CO-NH-Y-$NH_2$ worin Z" und Y wie oben definiert sind;
- Z"-Y'-NH-CO-Y-$NH_2$, worin Z" und Y wie oben definiert sind und Y' eine gerade oder verzweigte Alkylengruppe mit 1 oder 2 Kohlenstoffatomen darstellt;

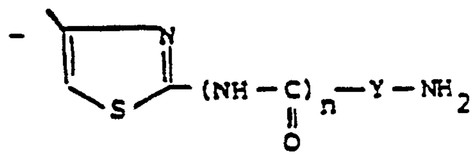

worin n = 0 oder 1 und Y wie zuvor definiert ist;

- einer Gruppe 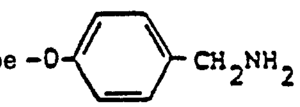

- der bicyclischen Gruppe 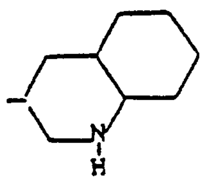

sowie die pharmazeutisch akzeptablen Salze und Ester dieser Derivate.

2. Verfahren zur Darstellung der Cephalosporinderivate nach Anspruch 1, dadurch gekennzeichnet, daß es darin besteht, daß:

1) die Verbindung der Formel:

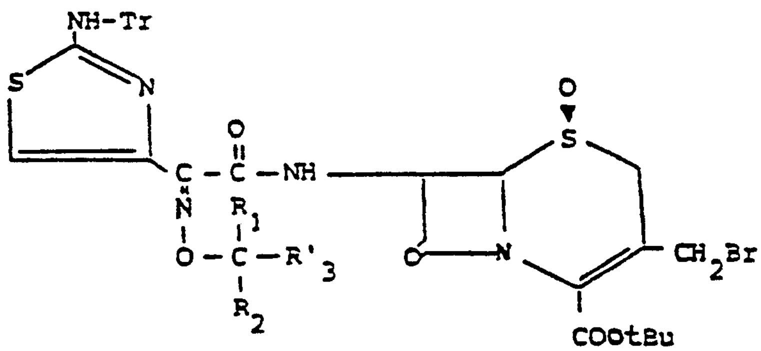

worin $R_1$ und $R_2$ wie im Anspruch 1 definiert sind, $R'_3$ Wasserstoff, eine Cyclopropylgruppe oder eine labile Estergruppe, deren Aminfunktion zuvor geschützt wurde, bezeichnet, mit der Säure oder Thiosäure der Formel B'-$(CH_2)_n$-COXH, worin X und n wie im Anspruch 1 definiert sind, B' die Gruppe B wie im Anspruch 1 definiert, in der die Aminfunktion geschützt ist, darstellt, reagieren gelassen wird;

2) die erhaltene Verbindung mit Phosphortribromid bei niedriger Temperatur und in einem Lösungsmittel zwecks Entfernens der S-Oxidfunktion behandelt wird;

3) die an den Amin- und Carboxylfunktionen vorhandenen Schutzgruppen durch Hydrolyse in saurem Milieu entfernt werden;

4) die so erhaltene Verbindung der Formel (I) in Form eines Salzes der Aminogruppe B isoliert wird;

5) gegebenenfalls das Salz in ein anderes Salz der Aminogruppe B übergeführt wird;

6) gegebenenfalls, ausgehend von einem Salz der Aminogruppe B, durch Einwirken einer Base die erwartete Verbindung der Formel (I) isoliert und gegebenenfalls mittels bekannter Verfahren in ein Salz oder einen Ester, abgeleitet von einer oder zwei im Molekül vorhandenen Carboxylgruppen, übergeführt wird.

3. Verfahren zur Darstellung der Cephalosporinderivate der Formel I nach Anspruch 1, worin X für Schwefel steht, dadurch gekennzeichnet, daß es darin besteht, daß:

1) die Verbindung der Formel:

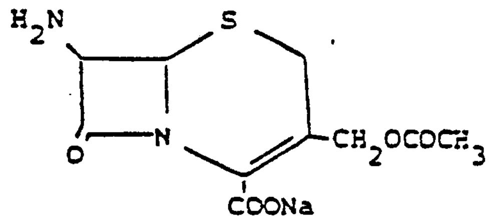

in wässerigem, auf einen pH von 6,3 - 6,4 gepuffertem Milieu mit der Thiosäure der Formel $B'-(CH_2)_n-COSNa$, worin n wie im Anspruch 1 definiert ist und B' die Gruppe B wie im Anspruch 1 definiert, in der die Aminfunktion geschützt ist, darstellt;
zur Herstellung der Verbindung der Formel:

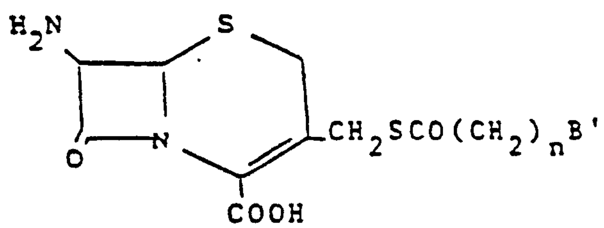

reagieren gelassen wird;
2) die so erhaltene Verbindung, deren Carboxylfunktion vorübergehend blockiert wurde, mit dem Säurechlorid der Formel:

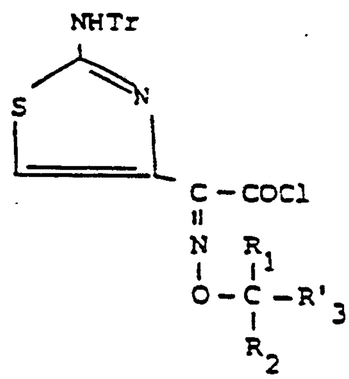

worin $R_1$ und $R_2$ wie im Anspruch 1 definiert sind und $R'_3$ Wasserstoff, eine Cyclpropylgruppe oder eine labile Estergruppe darstellt, zur Herstellung der erwarteten Verbindung der Formel (I), geschützt, acyliert wird;
3) die an den Amin- und Carboxylfunktionen vorhandenen Schutzgruppen durch Hydrolyse in saurem Milieu entfernt werden;
4) die so erhaltene Verbindung der Formel (I) in Form eines Salzes der Aminogruppe B isoliert wird;
5) gegebenenfalls das Salz in ein anderes Salz der Aminogruppe B übergeführt wird;
6) gegebenenfalls, ausgehend von einem Salz der Aminogruppe B, durch Einwirken einer Base, die erwartete Verbindung der Formel (I) isoliert und gegebenenfalls mittels bekannter Verfahren in ein Salz oder einen Ester, abgeleitet von einer oder zwei im Molekül vorhandenen Carboxylgruppe(n), übergeführt wird.

4. Pharmazeutische Zusammensetzung, dadurch gekennzeichnet, daß sie ein Cephalosporinderivat nach Anspruch 1 in Verbindung mit einem pharmazeutisch akzeptablen Vehikel enthält.

5. Antibiotische Zusammensetzung, dadurch gekennzeichnet, daß sie ein Cephalosporinderivat nach Anspruch 1 in Verbindung mit einem pharmazeutisch akzeptablen Vehikel enthält.

**Patentansprüche** für den Vertragsstaat: AT

1. Verfahren zur Darstellung von Cephalosporinderivaten der allgemeinen Formel:

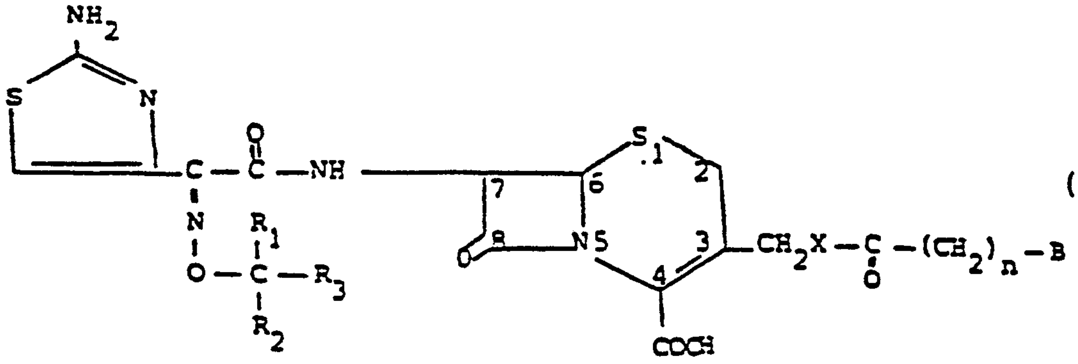

(I)

worin:

- X ein Sauerstoffatom oder ein Schwefelatom bezeichnet;
- n für Null, 1 oder 2 steht;
- $R_1$, $R_2$ und $R_3$ jeweils ein Wasserstoffatom bezeichnen, oder aber $R_1$ und $R_2$ ein Wasserstoffatom oder eine Methylgruppe bezeichnen und $R_3$ für eine Carboxyl- oder Cyclopropylgruppe steht; oder aber
- $R_1$ und $R_2$, zusammen mit dem Kohlenstoffatom, an das sie gebunden sind, einen Cyclobutylkern oder Cyclopentylkern bilden und $R_3$ eine Carboxylgruppe bezeichnet;
- B der Rest eines primären oder sekundären Amins ist, ausgewählt aus den folgenden Gruppen:
  - $Z-NH_2$, worin Z eine Alkylengruppe mit gerader oder verzweigter Kette mit 2 bis 7 Kohlenstoffatomen, gegebenenfalls unterbrochen durch ein Schwefelatom darstellt, oder aber Z für eine 1,3-Cyclohexylen- oder 1,4-Cyclohexylengruppe steht;
  - Z'-Alk-NH-R, worin Z' eine 1,2-Phenylen- oder 1,3-Phenylen- oder 1,4-Phenylengruppe, gegebenenfalls substituiert durch 1, 2 oder 3 Methylgruppen, ist, oder aber Z' eine 1,2-Cyclohexylen-, 1,3-Cyclohexylen- oder 1,4-Cyclohexylengruppe darstellt,
    Alk eine gerade oder verzweigte Alkylengruppe mit 1 bis 3 Kohlenstoffatomen, gegebenenfalls unterbrochen von einem Schwefelatom, darstellt,
    R ein Wasserstoffatom oder eine Alkylgruppe mit 1 bis 3 Kohlenstoffatomen bedeutet;
  - Z"-N-CO-Y-NH-R,
    |
    R'
    worin Z" eine 1,3-Phenylen- oder 1,4-Phenylengruppe darstellt, Y eine Alkylengruppe $(CH_2)_m$ bezeichnet, worin m = 1, 2 oder 3, R' für Wasserstoff oder Methyl steht und R die obige Bedeutung hat;
  - $Z''-CO-NH-Y-NH_2$ worin Z" und Y wie oben definiert sind;
  - $Z''-Y'-NH-CO-Y-NH_2$, worin Z" und Y wie oben definiert sind und Y' eine gerade oder verzweigte Alkylengruppe mit 1 oder 2 Kohlenstoffatomen darstellt;

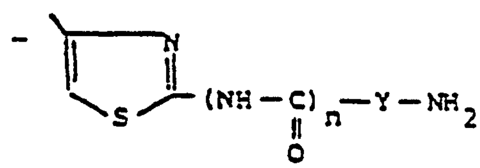

worin n = 0 oder 1 und Y wie zuvor definiert ist;
  - einer 2-Piperidyl-, 3-Piperidyl- oder 4-Piperidylgruppe, die gegebenenfalls am Stickstoffatom durch eine Gruppe $-CO-Y-NH_2$, worin Y wie zuvor definiert ist, substituiert ist;
  - einer Gruppe 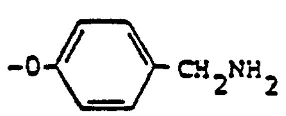
  - der bicyclischen Gruppe 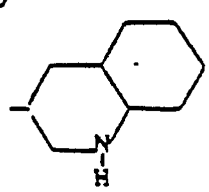

sowie deren pharmazeutisch akzeptabler Salze und Ester, dadurch gekennzeichnet, daß es darin besteht, daß:

1) die Verbindung der Formel:

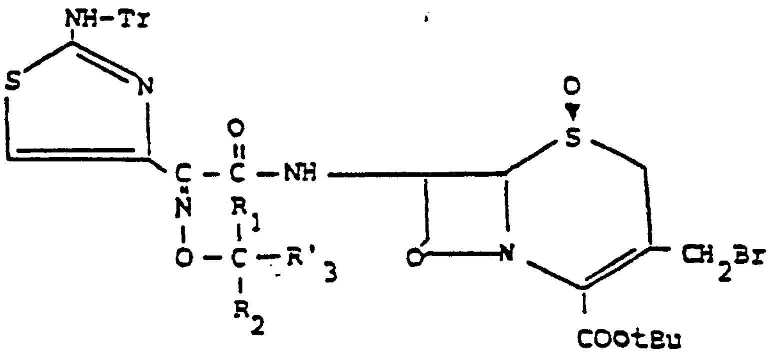

worin $R_1$ und $R_2$ wie oben definiert sind, $R'_3$ Wasserstoff, eine Cyclopropylgruppe oder eine labile Estergruppe, deren Aminfunktion zuvor geschützt wurde, bezeichnet, mit der Säure oder Thiosäure der Formel $B'-(CH_2)_n-COXH$, worin X und n wie oben definiert sind, B' die Gruppe B wie oben definiert, in der die Aminfunktion geschützt ist, darstellt, reagieren gelassen wird;

2) die erhaltene Verbindung mit Phosphortribromid bei niedriger Temperatur und in einem Lösungsmittel zwecks Entfernens der S-Oxidfunktion behandelt wird;

3) die an den Amin- und Carboxylfunktionen vorhandenen Schutzgruppen durch Hydrolyse in saurem Milieu entfernt werden;

4) die so erhaltene Verbindung der Formel (I) in Form eines Salzes der Aminogruppe B isoliert wird;

5) gegebenenfalls das Salz in ein anderes Salz der Aminogruppe B übergeführt wird;

6) gegebenenfalls, ausgehend von einem Salz der Aminogruppe B, durch Einwirken einer Base die erwartete Verbindung der Formel (I) isoliert und gegebenenfalls mittels bekannter Verfahren in ein Salz oder einen Ester, abgeleitet von einer oder zwei im Molekül vorhandenen Carboxylgruppen, übergeführt wird.

2. Verfahren zur Darstellung von Cephalosporinderivaten der Formel I nach Anspruch 1, worin X für Schwefel steht, dadurch gekennzeichnet, daß es darin besteht, daß:

1) die Verbindung der Formel:

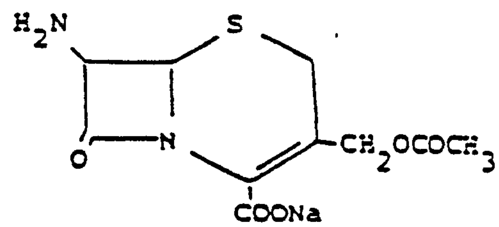

in wässerigem, auf einen pH von 6,3 - 6,4 gepuffertem Milieu mit der Thiosäure der Formel $B'-(CH_2)_n-COSNa$, worin n wie im Anspruch 1 definiert ist und B' die Gruppe B wie im Anspruch 1 definiert, in der die Aminfunktion geschützt ist, darstellt; zur Herstellung der Verbindung der Formel:

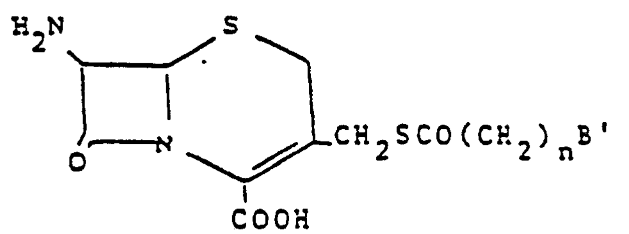

reagieren gelassen wird;

2) die so enthaltene Verbindung, deren Carboxylfunktion vorübergehend blockiert wurde, mit dem Säurechlorid der Formel:

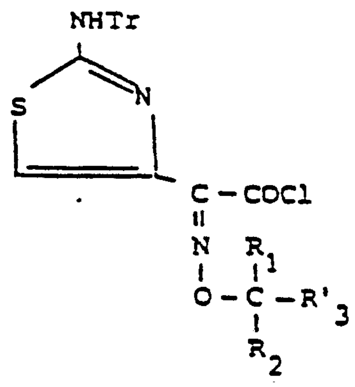

worin $R_1$ und $R_2$ wie im Anspruch 1 definiert sind und $R'_3$ Wasserstoff, eine Cyclopropylgruppe oder eine labile Estergruppe darstellt, zur Herstellung der erwarteten Verbindung der Formel (I), geschützt, acyliert wird;

3) die an den Amin- und Carboxylfunktionen vorhandenen Schutzgruppen durch Hydrolyse in saurem Milieu entfernt werden;

4) die so erhaltene Verbindung der Formel (I) in Form eines Salzes der Aminogruppe B isoliert wird;

5) gegebenenfalls das Salz in ein anderes Salz der Aminogruppe B übergeführt wird;

6) gegebenfalls, ausgehend von einem Salz der Aminogruppe B, durch Einwirken einer Base, die erwartete Verbindung der Formel (I) isoliert und gegebenfalls mittels bekannter Verfahren in ein Salz oder einen Ester, abgeleitet von einer oder zwei im Molekül vorhandenden Carboxylgruppe(n), übergeführt wird.

3. Verwendung der Cephalosporinderivate der Formel (I) nach Anspruch 1 zur Herstellung von pharmazeutischen Zusammensetzungen.

4. Verwendung nach Anspruch 3 zur Herstellung von antibiotischen Zusammensetzungen.

**Claims** for the contracting states: BE, CH, DE, FR, GB, IT, LI, LU, NL, SE

1. Cephalosporin derivatives of general formula:

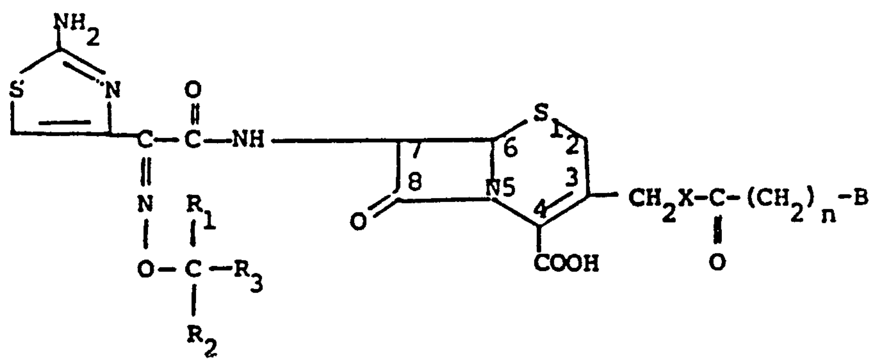 (I)

in which:

- X is an oxygen atom or a sulfur atom
- n is zero, 1 or 2,
- $R_1$, $R_2$ and $R_3$ each designate, a hydrogen atom or else
- $R_1$ and $R_2$ designate a hydrogen atom or a methyl group, and $R_3$ designates a carboxyl or cyclopropyl group or else
- $R_1$ and $R_2$ taken together with the carbon atom to which they are linked form a cyclobutyl or cyclopentyl group and $R_3$ is a carboxyl group,
- B is the residue of a primary or secondary amine selected from the following groups:
  - Z-$NH_2$ where Z is an alkylene group with a straight or branched chain having from 2 to 7 carbon atoms, possibly interrupted by a sulfur atom, or else Z is a 1,3-cyclohexylene or 1,4-cyclohexylene group,
  - Z'-Alk-NH-R where Z' is a 1,2-phenylene or 1,3-phenylene or 1,4-phenylene group possibly substituted by 1,2 or 3 methyl groups or else Z' is a 1,2-cyclohexylene, 1,3-cyclohexylene or 1,4-cyclohexylene group.

    Alk is a straight or branched alkylene group having from 1 to 3 carbon atoms, possibly interrupted by a sulfur atom,

    R is a hydrogen atom or an alkyl group having from 1 to 3 carbon atoms,
  - Z"-N-CO-Y-NH-R,<br>      |<br>      R'

where Z" is a 1,3-phenylene or 1,4-phenylene group,

Y is an alkylene group $(CH_2)_m$ in which m = 1, 2 or 3, R' is hydrogen or methyl and R is as defined above,

- Z"-CO-NH-Y-$NH_2$ where Z" and Y are as defined above,
- Z"-Y'-NH-CO-Y-$NH_2$ where Z" and Y are as defined above and Y' is a straight or branched alkylene group with 1 or 2 carbon atoms,
- 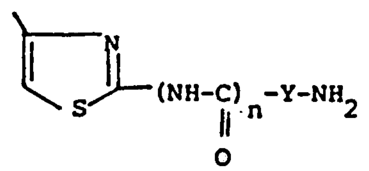

where n = 0 or 1 and Y is as defined above,

- a 2-piperidyl, 3-piperidyl or 4-piperidyl group possibly substituted on the nitrogen atom by a -CO-Y-$NH_2$ group where Y is as defined above,
- a group 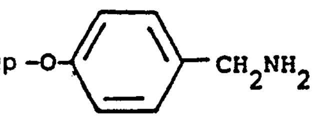 ,
- the bicyclic group 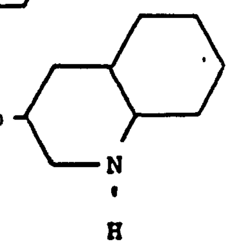 ,

as well as the pharmaceutically acceptable salts and esters thereof.

2. Process for the preparation of the cephalosporin derivatives according to claim 1, characterized in that said process consists:

1) in reacting on the compound of formula:

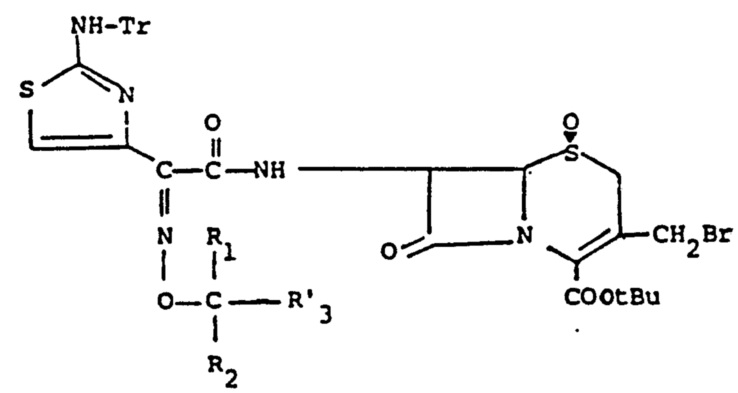

in which $R_1$ and $R_2$ are as defined in claim 1, $R'_3$ is hydrogen, a cyclopropyl group or a readily labile ester group, of which the amine function has been protected beforehand, the acid or thioacid of formula B'-$(CH_2)_n$-COXH, in which X and n are as defined in claim 1, B' is the B group such as defined in claim 1, in which the amine function is protected,

2) in subjecting the resulting compound to the action of phosphorous tribromide at low temperature and within a solvent, in order to eliminate the S-oxide function,

3) in eliminating the protecting groups present on the amine and carboxylic functions, by hydrolysis in acid medium,

4) in isolating the resulting compound of formula (I) in the form of a salt of amine group B,

5) in optionally converting said salt into another salt of amine group B,

6) in optionally isolating the expected compound of formula I, from a salt of amine group B, by the action of a base, and in optionally converting said compound, by known methods, into a salt or an ester deriving from one or two carboxylic groups present in the molecule.

3. Process for the preparation of the cephalosporin derivatives of formula I, according to claim 1 in which X is sulfur, characterized in that said process consists:

1) in reacting the compound of formula:

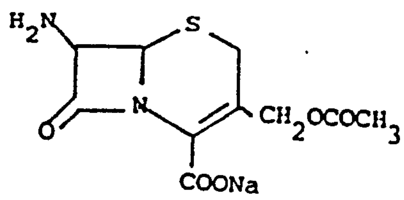

in buffered aqueous medium at pH: 6.3 - 6.4, with the thioacid of formula $B'-(CH_2)_n$ COSNa, in which n is as defined in claim 1, and B' is the group B defined in claim 1, in which the amine function is protected, in order to obtain the compound of formula:

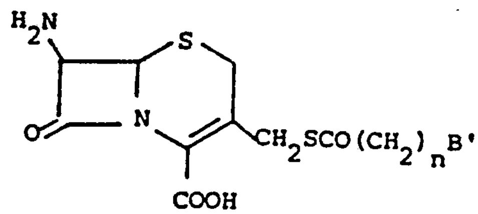

2) in acylating the resulting compound of which the carboxylic function has been temporarily blocked, with the chloride of the acid of formula:

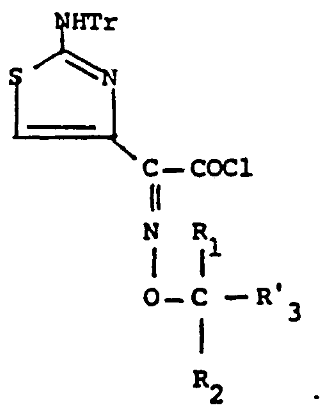

in which $R_1$ and $R_2$ are as defined in claim 1 and $R'_3$ is hydrogen, a cyclopropyl group or a readily labile ester group, in order to obtain the expected protected compound of formula (I),

3) in eliminating the protecting groups present on the amine and carboxylic functions, by hydrolysis in acid medium,

4) in isolating the resulting compound of formula (I) as a salt of the amine group B,

5) in optionally converting said salt into another salt of the amine group B,

6) in optionally isolating the expected compound of formula I from a salt of amine group B, by the action of a base, and in optionally converting said compound by known methods, into a salt or an ester, deriving from one or two carboxylic groups present in the molecule.

4. Pharmaceutical composition, characterized in that it contains a cephalosporin derivative according to claim 1, in combination with a pharmaceutically acceptable vehicle.

5. Antibiotic composition, characterized in that it contains a cephalosporin derivative according to claim 1, in combination with a pharmaceutically acceptable vehicle.

**Claims** for the contracting state: AT

1. Process for the preparation of the cephalosporin derivatives of general formula:

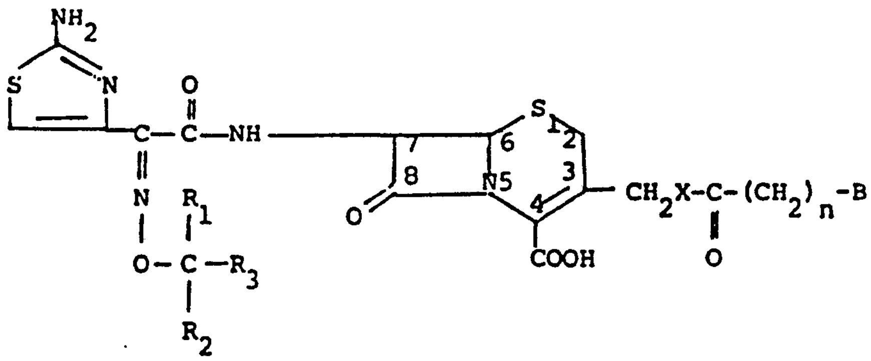 (I)

in which:

- X is an oxygen atom or a sulfur atom
- n is zero, 1 or 2,
- $R_1$, $R_2$ and $R_3$ each designate, a hydrogen atom or else $R_1$ and $R_2$ designate a hydrogen atom or a methyl group, and $R_3$ designates a carboxyl or cyclopropyl group or else
- $R_1$ and $R_2$ taken together with the carbon atom to which they are linked from a cyclobutyl or cyclopentyl group and $R_3$ is a carboxyl group,
- B is the residue of a primary or secondary amine selected by the following groups:
- Z-$NH_2$ where Z is an alkylene group with a straight or branched chain having from 2 to 7 carbon atoms, possibly interrupted by a sulfur atom, or else Z is a 1,3-cyclohexylene or 1,4-cyclohexylene group,
- Z'-Alk-NH-R where Z' is a 1,2-phenylene or 1,3-phenylene or 1,4-phenylene group possibly substituted by 1, 2 or 3 methyl groups or else Z' is a 1,2-cyclohexylene, 1,3-cyclohexylene or 1,4-cyclohexylene group,
  Alk is a straight or branched alkylene group having from 1 to 3 carbon atoms, possibly interrupted by a sulfur atom,
  R is a hydrogen atom or an alkyl group having from 1 to 3 carbon atoms,
  - Z"-N(R')-CO-Y-NH-R,
  where Z" is a 1,3-phenylene or 1,4-phenylene group,
  Y is an alkylene group $(CH_2)_m$ in which m = 1, 2 or 3, R' is hydrogen or methyl and R is as defined above,
- Z"-CO-NH-Y-$NH_2$ where Z" and Y are as defined above,
- Z"-Y'-NH-CO-Y-$NH_2$ where Z" and Y are as defined above and Y' is a straight or branched alkylene group with 1 or 2 carbon atoms,
- 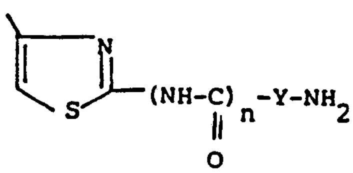
  where n = 0 or 1 and Y is as defined above,
- a 2-piperidyl, 3-piperidyl or 4-piperidyl group possibly substituted on the nitrogen atom by a -CO-Y-$NH_2$ group where Y is as defined above,
- a group 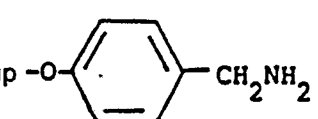 ,
- the bicyclic group 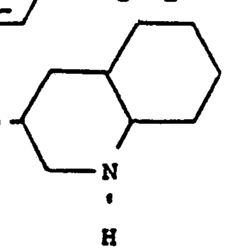 ,

as well as the pharmaceutically acceptable salts and esters thereof, characterized in that said process consists:

1) in reacting on the compound of formula:

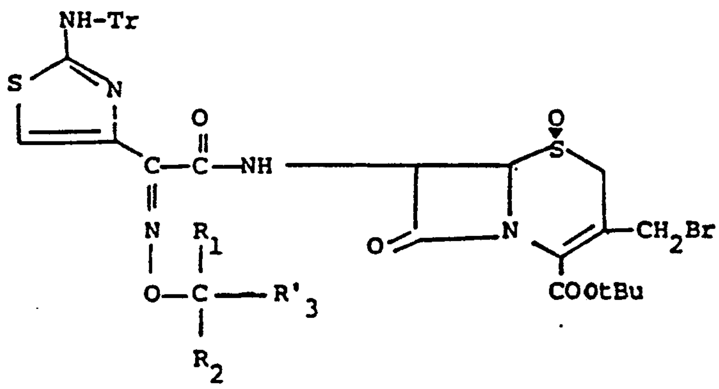

in which $R_1$ and $R_2$ are as defined above, $R'_3$ is hydrogen, a cyclopropyl group or a readily labile ester group, of which the amine function has been protected beforehand, the acid or thioacid of formula $B'-(CH_2)_n-COXH$, in which X and n are as defined above, B' is the B group such as defined above, in which the amine function is protected,

2) in subjecting the resulting compound to the action of phosphorous tribromide at low temperature and within a solvent, in order to eliminate the S-oxide function,

3) in eliminating the protecting groups present on the amine and carboxylic functions, by hydrolysis in acid medium,

4) in isolating the resulting compound of formula (I) in the form of a salt of amine group B,

5) in optionally converting said salt into another salt of amine group B,

6) in optionally isolating the expected compound of formula 1, from a salt of amine group B, by the action of a base, and in optionally converting said compound, by known methods, into a salt or an ester deriving from one or two carboxylic groups present in the molecule.

2. Process for the preparation of the cephalosporin derivatives of formula I, according to claim 1 in which X is sulfur, characterized in that consists:

1) in reacting the compound of formula:

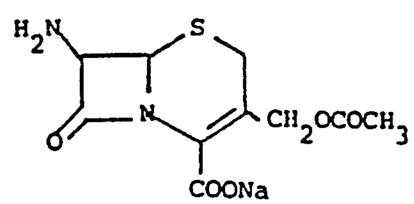

in buffered aqueous medium at pH: 6.3 - 6.4, with the thioacid of formula $B'-(CH_2)$ COSNa, in which n is as defined in claim 1, and B' is the group B defined in claim 1, in which the amine function is protected, in order to obtain the compound of formula:

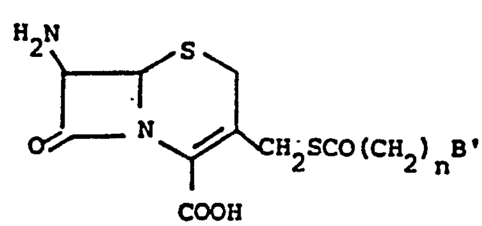

2) acylating the resulting compound of which the carboxylic function has been temporarily blocked, with the chloride from the acid of formula:

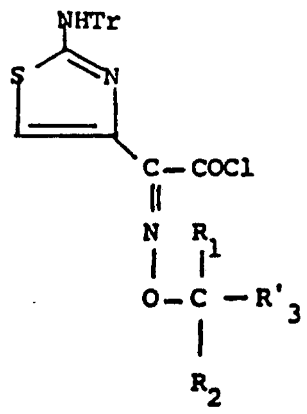

in which $R_1$ and $R_2$ are as defined in claim 1, and $R'_3$ is hydrogen, a cyclopropyl group or a readily labile ester group, in order to obtain the expected protected compound of formula (I),

3) in eliminating the protecting groups present on the amine and carboxylic functions, by hydrolysis in acid medium,

4) in isolating the resulting compound of formula (I) as a salt of the amine group B,

5) in optionally converting said salt into another salt of the amine group B,

6) in optionally isolating the expected compound of formula I from a salt of amine group B, by the action of a base, and in optionally converting said compound by known methods, into a salt or an ester, deriving from one or two carboxylic groups present in the molecule.

3. Use of the cephalosporin derivatives of formula I according to claim 1, for the preparation of pharmaceutical compositions.

4. Use according to claim 3, for the preparation of antibiotic compositions.